# EUROPEAN PATENT SPECIFICATION

(11) **EP 2 968 546 B1**
(45) Date of publication and mention of the grant of the patent: **20.09.2023**
(21) Application number: 14764165.8
(22) Date of filing: 14.03.2014
(51) Int. Cl.: A61K 39/395, C07K 16/40, C07K 14/81

(54) **METHODS OF GENERATING BIOACTIVE PEPTIDE-BEARING ANTIBODIES AND COMPOSITIONS COMPRISING THE SAME**
VERFAHREN ZUR ERZEUGUNG VON BIOAKTIVEM PEPTID TRAGENDEN ANTIKÖRPERN UND ZUSAMMENSETZUNGEN DAMIT
MÉTHODES DE GÉNÉRATION D'ANTICORPS PORTEURS DE PEPTIDE BIOACTIF ET COMPOSITIONS LES COMPRENANT

(30) Priority: 15.03.2013 US 201361962289 P; 16.05.2013 US 201361823964 P
(43) Date of publication of application: 20.01.2016
(73) Proprietor: Omeros Corporation, Seattle, WA 98119 (US)
(72) Inventor: CUMMINGS, W., Jason, Bellevue, WA 98004 (US); GRAY, Patrick, Seattle, WA 98122 (US); TJOELKER, Larry, W., Kirkland, WA 98034 (US); YABUKI, Munehisa, Seattle, WA 98103 (US)
(74) Representative: Avidity IP
(86) International application number: PCT/US2014/028999
(87) International publication number: WO 2014/144542

(56) References cited:
- WO-A1-2004/106384
- WO-A1-2012/007777
- WO-A1-2012/007777
- WO-A2-2007/048022
- US-A1- 2003 104 497
- US-A1- 2011 091 450
- US-A1- 2012 135 012
- US-A1- 2012 258 095
- D. HEJA ET AL: "Monospecific Inhibitors Show That Both Mannan-binding Lectin-associated Serine Protease-1 (MASP-1) and -2 Are Essential for Lectin Pathway Activation and Reveal Structural Plasticity of MASP-2", JOURNAL OF BIOLOGICAL CHEMISTRY, vol. 287, no. 24, 8 June 2012 (2012-06-08) , pages 20290-20300, XP055285722, US ISSN: 0021-9258, DOI: 10.1074/jbc.M112.354332
- LI LINGNA ET AL: "Antitumor Efficacy of a Thrombospondin 1 Mimetic CovX-Body", TRANSLATIONAL ONCOLOGY, NEOPLASIA PRESS, UNITED STATES, vol. 4, no. 4, 1 August 2011 (2011-08-01), pages 249-257, XP009156741, ISSN: 1936-5233, DOI: 10.1593/TLO.11136
- J CORONELLA ET AL: "Selective Activity Against Proliferating Tumor Endothelial Cells by CVX-22, A Thrombospondin-1 Mimetic CovX-Body", ANTICANCER RESEARCH, vol. 29, 1 June 2009 (2009-06-01), pages 2243-2252, XP055304693,
- HANHUA HUANG ET AL: "Specifically targeting angiopoietin-2 inhibits angiogenesis, Tie2-expressing monocyte infiltration, and tumor growth", CLINICAL CANCER RESEARCH, THE AMERICAN ASSOCIATION FOR CANCER RESEARCH, US, vol. 17, no. 5, 1 March 2011 (2011-03-01), pages 1001-1011, XP002663787, ISSN: 1078-0432, DOI: 10.1158/1078-0432.CCR-10-2317 [retrieved on 2011-01-13]

## Description

### FIELD OF THE INVENTION

The present invention relates to methods for generating bioactive peptide-bearing antibodies and fragments thereof, such as antibodies comprising bioactive peptides (e.g., inhibitory peptide-bearing MASP-2 antibodies) and compositions comprising such bioactive peptide-bearing antibodies for use in inhibiting complement activation.

### STATEMENT REGARDING SEQUENCE LISTING

The sequence listing associated with this application is provided in text format in lieu of a paper copy. The name of the text file containing the sequence listing is MP_1_0207_PCT_Sequence_Listing_20140311_ST25. The text file is297 KB, was created on March 12, 2014; and is being submitted via EFS-Web with the filing of the specification.

### BACKGROUND

The complement system provides an early acting mechanism to initiate, amplify and orchestrate the immune response to microbial infection and other acute insults (M.K. Liszewski and J.P. Atkinson, 1993, in Fundamental Immunology, Third Edition, edited by W.E. Paul, Raven Press, Ltd., New York) in humans and other vertebrates. While complement activation provides a valuable first-line defense against potential pathogens, the activities of complement that promote a protective immune response can also represent a potential threat to the host (K.R. Kalli, et al., Springer Semin. Immunopathol. 15:417-431, 1994; B.P. Morgan, Eur. J. Clinical Investig. 24:219-228, 1994). For example, the C3 and C5 proteolytic products recruit and activate neutrophils. While indispensable for host defense, activated neutrophils are indiscriminate in their release of destructive enzymes and may cause organ damage. In addition, complement activation may cause the deposition of lytic complement components on nearby host cells as well as on microbial targets, resulting in host cell lysis.

The complement system has also been implicated in the pathogenesis of numerous acute and chronic disease states, including: myocardial infarction, stroke, acute respiratory distress syndrome, reperfusion injury, septic shock, capillary leakage following thermal burns, post cardiopulmonary bypass inflammation, transplant rejection, rheumatoid arthritis, multiple sclerosis, myasthenia gravis, age-related macular degeneration, paroxysmal nocturnal hemoglobinuria, and Alzheimer's disease. In almost all of these conditions, complement is not the cause but is one of several factors involved in pathogenesis. Nevertheless, complement activation may be a major pathological mechanism and represents an effective point for clinical control in many of these disease states.

The growing recognition of the importance of complement-mediated tissue injury in a variety of disease states underscores the need for effective complement inhibitory drugs. To date, Eculizumab (Soliris^{®}), an antibody against C5, is the only complement-targeting drug that has been approved for human use. Yet, C5 is one of several effector molecules located "downstream" in the complement system, and blockade of C5 does not inhibit activation of the complement system. Therefore, an inhibitor of the initiation steps of complement activation would have many significant advantages over a "downstream" complement inhibitor.

Three distinct pathways of complement activation have been defined. The classical pathway is activated upon binding of particular antibody isotypes to a pathogen or host antigen. The lectin pathway is activated upon binding of pattern recognition lectins, such as mannan-binding lectin (MBL), CL-11, or ficolins L, M, or H to complex microbial or host macromolecules such as polysaccharides. Finally, the alternative pathway serves to amplify the signals generated by the classical and lectin pathways. A family of serine proteases is integral to the initial activation steps of all three pathways. C1r and C1s form the enzymatic components of the C1 complex that is assembled by complement-activating antibodies. In addition, there are three MBL-associated serine proteases (MASPs) that initiate and/or propagate the protease cascades of the lectin and alternative pathways (Yongqing et al., Biochim. Biophys. Acta 1824:253, 2012).

MASP-1, MASP-2 and MASP-3 share identical domain organizations with those of C1r and C1s, the enzymatic components of the C1 complex (Sim, R.B., et al., Biochem. Soc. Trans. 28:545, 2000). These domains include an N-terminal C1r/C1s/sea urchin VEGF/bone morphogenic protein (CUB) domain, an epidermal growth factor-like domain, a second CUB domain, a tandem of complement control protein domains, and a serine protease domain. As in the C1 proteases, activation of the MASP proteases occurs through cleavage of an Arg-Ile bond adjacent to the serine protease domain, which splits the enzyme into disulfide-linked A and B chains, the latter consisting of the serine protease domain.

The generation of specific peptide inhibitors of MASP-1 and MASP-2, termed SGMI-1 and SGMI-2, respectively, is described in Heja et al., J Biol Chem 287:20290 (2012) and Heja et al., PNAS 109:10498 (2012), each of which is hereby incorporated herein by reference. SGMI-1 and SGMI-2 are each 36 amino acid peptides which were selected from a phage library of variants of the *Schistocerca gregaria* protease inhibitor 2 in which six of the eight positions of the protease binding loop were fully randomized. Mechanistically, both SGMI-1 and SGMI-2 block the lectin pathway of complement activation without affecting the classical pathway (Heja et al., 2012. Proc. Natl. Acad. Sci. 109:10498). However, peptides such as SGMI-1 and SGMI-2 have limited potential for use in therapeutic applications because of the short half-life of peptides in serum.

### SUMMARY

This summary is provided to introduce a selection of concepts in a simplified form that are further described below in the Detailed Description. The features of the invention are set out in accordance with the claims appended hereto. Other disclosures herein not falling within the claims are for the assistance of the skilled person in understanding and practicing the disclosed invention. For the avoidance of doubt, it is noted that the present invention does not extend to methods of treatment of the human or animal body Any reference in the description to methods of treatment or in vivo diagnosis refer to the compounds, pharmaceutical compositions and medicaments of the present invention for use in method of treatment of the human or animal body by therapy or for in vivo diagnosis.

Described herein is an isolated antibody, or antigen-binding fragment thereof, comprising a bioactive peptide amino acid sequence, wherein the bioactive peptide amino acid sequence is an inhibitor of the complement system and is fused to at least one of: (i) the amino terminal region of at least one of: a light chain variable region and/or a heavy chain variable region; or (ii) the carboxy terminal region of at least one of: a light chain constant region and/or a heavy chain constant region, wherein the antibody inhibits complement activation.

The present invention provides an isolated bioactive peptide-bearing antibody, or antigen-binding fragment thereof, comprising:
(i) a heavy chain variable region and a light chain variable region each comprising three CDRs that specifically bind to human MASP-2, set forth as SEQ ID NO:4, wherein said heavy chain variable region comprises SEQ ID NO: 111 and said light chain variable region comprises SEQ ID NO: 115; and
(ii) an SGMI peptide sequence set forth as SEQ ID NO:6 or SEQ ID NO:9; wherein the antibody is selected from the group consisting of:
   (a) wherein the SGMI peptide sequence comprising SGMI-1 set forth as SEQ ID NO:6 is fused to the amino terminal region of the heavy chain variable region of the antibody;
   (b) wherein the SGMI peptide sequence comprising SGMI-1 set forth as SEQ ID NO:6 is fused to the amino terminal region of the light chain variable region of the antibody;
   (c) wherein the SGMI peptide sequence comprising SGMI-1 set forth as SEQ ID NO:6 is fused to the carboxy terminal region of the light chain constant region of the antibody;
   (d) wherein the SGMI peptide sequence comprising SGMI-2 set forth as SEQ ID NO:9 is fused to the carboxy terminal region of the heavy chain constant region of the antibody;
   (e) wherein the SGMI peptide sequence comprising SGMI-2 set forth as SEQ ID NO:9 is fused to the amino terminal region of the light chain variable region of the antibody; and
   (f) wherein the SGMI peptide sequence comprising SGMI-2 set forth as SEQ ID NO:9 is fused to the carboxy terminal region of the light chain constant region of the antibody, wherein the antibody or antigen binding fragment thereof inhibits MASP-2-dependent lectin pathway complement activation.

Also described herein is an isolated antibody, or antigen binding fragment thereof, comprising:(i) a heavy chain variable region and/or a light chain variable region comprising one or more CDRs that specifically bind to MASP-2; and (ii) at least one SGMI core peptide sequence comprising an amino acid sequence according to: X₁CTX₂X₃X₄CX₅Q (SEQ ID NO:5), wherein: X₁ is F or V, X₂ is R or K, X₃ is K or L, X4 is L or W, and X₅ is Y or N; and wherein the SGMI core peptide sequence is fused to at least one of: (a) the amino terminal region of at least one of: a light chain variable region and/or a heavy chain variable region; or (b) the carboxy terminal region of at least one of: a light chain variable region and/or a heavy chain variable region; or (c) the carboxy terminal region of at least one of: a light chain human constant region and/or a heavy chain human constant region; wherein the antibody or antigen binding fragment thereof, inhibits MASP-2-dependent lectin pathway complement activation.

Also described herein is a method of generating an antibody comprising a variable region comprising one or more complementary determining regions (CDRs) that specifically bind MASP-2 and at least one SGMI core peptide sequence, the method comprising culturing a cell comprising a nucleic acid molecule encoding the amino acid sequence of said peptide-bearing antibody under conditions allowing for expression of the nucleic acid molecules encoding the antibody and isolating said peptide-bearing antibody.

Also described herein is an isolated monoclonal antibody or antigen-binding fragment thereof that binds to human MASP-2 and which further comprises an amino acid sequence corresponding to at least one of SGMI-1 (set forth as SEQ ID NO:6) or SGMI-2 (set forth as SEQ ID NO:9), wherein said antibody or antigen binding fragment thereof inhibits C4 activation on a mannan-coated substrate with an IC₅₀ of 10nM or less in 1% human serum. In one case, said antibody or antigen binding fragment thereof specifically recognizes at least part of an epitope recognized by (i) a reference antibody comprising a heavy chain variable region as set forth as SEQ ID NO: 111 and a light chain variable region set forth as SEQ ID NO:115, or (ii) a reference antibody produced by the hybridoma cell line deposited in the European Collection of Cell Cultures (ECACC), Salisbury Wiltshire, United Kingdom, under the accession number 03050904.

Also described herein is a method of making a bioactive peptide-bearing antibody, the method comprising: (a) engrafting the amino acid sequence of at least one bioactive peptide of interest into: (i) at least one of CDR-H1, CDR-H2 or CDR-H3 of a heavy chain variable region comprising one or more chicken framework regions and/or (ii) at least one of CDR-L1, CDR-L2 or CDR-L3 of the light chain variable region comprising one or more chicken framework regions, and (b) determining whether the peptide-bearing antibody has at least substantially the same or increased biological activity as the isolated bioactive peptide.

Also described herein is an isolated antibody, or antigen-binding fragment thereof, comprising one or more bioactive peptide amino acid sequence(s), wherein at least one bioactive peptide amino acid sequence is engrafted into at least one of: (i) a light chain variable region comprising one or more chicken framework regions and/or (ii) a heavy chain variable region comprising one or more chicken framework regions. In some cases, a bioactive peptide is engrafted into at least one of CDR-H1, CDR-H2 or CDR-H3 of a heavy chain variable region comprising one or more chicken framework regions. In some cases, a bioactive peptide is engrafted into at least one of CDR-L1, CDR-L2 or CDR-L3 of a light chain variable region comprising one or more chicken framework regions.

Also described herein is a method of making a bioactive peptide-bearing antibody, the method comprising: (a) fusing the amino acid sequence of at least one bioactive peptide of interest onto: (i) an amino terminal region of at least one of: a light chain variable region comprising one or more framework regions and/or a heavy chain variable region comprising one or more framework regions, and/or (ii) a carboxy terminal region of at least one of: a light chain constant region and/or a heavy chain constant region; and (b) determining whether the peptide-bearing antibody has at least substantially the same or increased biological activity as compared to the isolated bioactive peptide.

Also described herein is an isolated antibody, or antigen-binding fragment thereof, comprising a bioactive peptide amino acid sequence, wherein the bioactive peptide amino acid sequence is fused to at least one of: (i) the amino terminal region of at least one of: a light chain variable region comprising one or more framework regions and/or a heavy chain variable region comprising one or more framework regions; or (ii) the carboxy terminal region of at least one of: a light chain constant region and/or a heavy chain constant region, wherein the peptide-bearing antibody has at least substantially the same or increased biological activity as the isolated bioactive peptide.

Also described herein is an isolated polypeptide comprising: (i) a region comprising an SGMI core sequence, the SGMI core sequence comprising an amino acid sequence according to: X₁CTX₂X₃X₄CX₅Q (SEQ ID NO:5) wherein:X₁ is F or V, X₂ is R or K, X₃ is K or L, X₄ is L or W, and X₅ is Y or N; and (ii) a region comprising human IgG1 Fc, wherein the polypeptide inhibits the activity of at least one of MASP-1 or MASP-2.

Also described herein are pharmaceutical compositions comprising the bioactive-peptide bearing antibodies, and antigen-binding fragments thereof, as disclosed herein.

Also described herein is a method of inhibiting lectin pathway complement activation in a mammalian subject in need thereof, comprising administering to the subject a composition comprising an SGMI-peptide bearing antibody, or antigen-binding fragment thereof, in an amount sufficient to inhibit lectin pathway activation.

Also described herein is a method of manufacturing a medicament for use in inhibiting MASP-2-dependent lectin complement activation and/or MASP-1-dependent lectin complement activation for treating, preventing, or reducing the severity of a lectin complement-mediated vascular condition, an ischemia reperfusion injury, atherosclerosis, inflammatory gastrointestinal disorder, a pulmonary condition, an extracorporeal reperfusion procedure, a musculoskeletal condition, a renal condition, a skin condition, organ or tissue transplant, nervous system disorder or injury, a blood disorder, a urogenital condition, diabetes, chemotherapy or radiation therapy, malignancy, an endocrine disorder, a coagulation disorder, a thrombotic microangiopathy, or an ophthalmologic condition, comprising combining a therapeutically effective amount of a bioactive peptide-bearing antibody (e.g., an SGMI-2 bearing antibody, such as an SGMI-2- MASP-2 antibody and/or an SGMI-1 bearing antibody, such as an SGMI-1 MASP-2 antibody, or an SGMI-1 and SGMI-2 bearing antibody) or antigen-binding fragment thereof, capable of inhibiting MASP-2-dependent lectin pathway activation and/or MASP-1-dependent lectin pathway activation in a pharmaceutical carrier.

Also described herein is a method of manufacturing a medicament for use in inhibiting lectin pathway activation and MASP-1-dependent alternative pathway activation for treating, preventing, or reducing the severity of a disease or disorder selected from the group consisting of: Paroxysmal nocturnal hemoglobinuria, age-related macular degeneration, ischemia-reperfusion injury, arthritis, disseminated intravascular coagulation, thrombotic microangiopathy (including hemolytic uremic syndrome (HUS), atypical HUS (aHUS) and thrombotic thrombocytopenic purpura (TTP)), asthma, dense deposit disease, pauci-immune necrotizing crescentic glomerulonephritis, traumatic brain injury, aspiration pneumonia, endophthalmitis, neuromyelitis optica and Behcet's disease comprising combining a therapeutically effective amount of at least one of (i) a bioactive peptide bearing antibody capable of inhibiting MASP-1 activity (e.g., an antibody comprising SGMI-1), (ii) an SGMI-1-MASP-2 antibody, (iii) a SGMI-1 and SGMI-2 bearing antibody, or (iv) a first SGMI-1-bearing antibody and a second SGMI-2-bearing antibody in a pharmaceutical carrier.

As described herein, the various cases of the bioactive-bearing antibodies and fragments thereof can be used in the pharmaceutical compositions of the invention.

As described herein, the pharmaceutical compositions of the invention can be used in accordance with the methods of the disclosure.

### DESCRIPTION OF THE DRAWINGS

The foregoing disclosure and many its attendant advantages will become more readily appreciated as the same become better understood by reference to the following detailed description, when taken in conjunction with the accompanying drawings, wherein:
FIGURE 1 is a bar graph showing the percent C5b-C9 formation in the presence of positive serum, negative serum, isotype control, SGMI-1Fc or SGMI-2Fc, demonstrating that both SGMI-1Fc and SGMI-2Fc inhibit the activation of the lectin pathway, as described in Example 2;
FIGURE 2 graphically illustrates the level of C3b deposition for 1% normal serum plus isotype control, SGMI-1Fc or SGMI-2Fc over a concentration range of 0.15 nM to 1000 nM, demonstrating that both SGMI-1Fc and SGMI-2Fc inhibited C3b deposition from normal serum in mannan-coated ELISA wells, as described in Example 2;
FIGURE 3 illustrates an exemplary parental (DTLacO) variable heavy chain polypeptide sequence compared to a variable heavy chain polypeptide sequence comprising a bioactive peptide amino acid sequence engrafted within complementarity determining region-3 (CDR-3);
FIGURE 4 shows an alignment of the amino acid sequences of exemplary variable heavy chain polypeptides comprising the bioactive peptide SGMI-1, and variants thereof, engrafted within CDR-3, including optional linkers at the C-terminus and/or N-terminus of the bioactive peptide;
FIGURE 5 illustrates an exemplary parental (DTLacO) variable light chain polypeptide sequence compared to a variable light chain polypeptide sequence comprising a bioactive peptide engrafted within CDR-1;
FIGURE 6 shows an alignment of the amino acid sequences of exemplary variable light chain polypeptides comprising the bioactive peptide SGMI-1 or SGMI-2, and variants thereof, engrafted within CDR-1, including optional linkers at the C-terminus and/or N-terminus of the bioactive peptide.
FIGURE 7A graphically illustrates the inhibitory activity of various representative chimeric chicken/human mAbs containing SGMI-1 engrafted into CDR-H3 on C5b-C9 deposition;
FIGURE 7B graphically illustrates the inhibitory activity of additional various representative chimeric chicken/human mAbs containing SGMI-1 engrafted into CDR-3 on C5b-C9 deposition;
FIGURE 8A graphically illustrates the inhibitory activity of various representative chimeric chicken/human mAbs containing SGMI-1 engrafted into CDR-H3 on complement C3b deposition activity in a dose-response manner, as described in Example 3;
FIGURE 8B graphically illustrates the inhibitory activity of additional various representative chimeric chicken/human mAbs containing SGMI-1 engrafted into CDR-H3 on complement C3b deposition activity in a dose-response manner, as described in Example 3;
FIGURE 8C graphically illustrates the inhibitory activity of various representative chimeric chicken/human monoclonal antibodies (mAbs) containing SGMI-1 engrafted into CDR-L1 on complement C3b deposition activity in a dose-response manner, as described in Example 3;
FIGURE 8D graphically illustrates the inhibitory activity of additional various representative chimeric chicken/human mAbs containing SGMI-1 engrafted into CDR-L1 on complement C3b deposition activity in a dose-response manner, as described in Example 3;
FIGURE 9A graphically illustrates the inhibitory activity of a chimeric chicken/human mAb comprising SGMI-2 engrafted within CDR-L1 (Ab-SGMI-2L-Igk) and a combination of SGMI-1 engrafted within CDR-H3 and SGMI-2 engrafted within CDR-L1 (Ab-SGMI-1-L1-IgG1/SGMI-2L-Igλ), demonstrating that the chimeric combination SGMI-1 - SGMI-2 mAb (Ab-SBMI-1-L1-IgG1/SGMI-2L-Igλ) inhibits C5b-C9 deposition, as described in Example 3;
FIGURE 9B graphically illustrates the inhibitory activity of a chimeric chicken/human mAb comprising a combination of SGMI-1 engrafted within CDR-H3 and SGMI-2 engrafted within CDR-L1 (Ab-SGMI-1-L1-IgG1/SGMI-2L-Igλ), demonstrating that the chimeric combination SGMI-1 - SGMI-2 mAb (Ab-SGMI-1-L1-IgG1/SGMI-2L-Igλ) inhibits C5b-C9 deposition, as described in Example 3;
FIGURE 10 illustrates a chimeric chicken/human antibody comprising bioactive peptides fused to the N-terminus of the heavy chain variable region (A); and/or the N-terminus of the light chain variable region (B); and/or the C-terminus of the heavy chain constant region (C); and/or the C-terminus of the light chain constant region (D), as described in Example 4;
FIGURE 11 graphically illustrates the inhibitory activity of chimeric chicken/human antibodies comprising bioactive SGMI-1 or SGMI-2 peptides fused to the N- or C-terminus of the heavy or light chain, demonstrating that all of the peptide-mAb fusions inhibit C5b-C9 deposition, as described in Example 4;
FIGURE 12 is a schematic diagram adapted from Schwaeble et al., Immunobiol 205:455-466 (2002), as modified by Yongqing et al., BBA 1824:253 (2012), illustrating the MASP-2 and MAp19 protein domains and the exons encoding the same;
FIGURE 13 is a schematic diagram adapted from Schwaeble et al., Immunobiol 205:455-466 (2002), as modified by Yongqing et al., BBA 1824:253 (2012), illustrating the MASP-1, MASP-3 and MAp44 protein domains and the exons encoding the same;
FIGURE 14A and 14B shows an amino acid sequence alignment of the most active scFv clones, revealing two distinct groups belonging to VH2 and VH6 gene family, respectively, as described in Example 5;
FIGURE 15A and 15B shows an amino acid sequence alignment of the scFv clones 17D20, 17N16, 18L16 and 4D9, as described in Example 5;
FIGURE 16 illustrates a MASP-2 antibody scaffold comprising one or more SGMI peptides fused to the N-terminus of the heavy chain variable region (A); and/or the N-terminus of the light chain variable region (B); and/or the C-terminus of the heavy chain constant region (C); and/or the C-terminus of the light chain constant region (D), as described in Example 7;
FIGURE 17A illustrates a MASP-2 scFv antibody scaffold and SGMI peptide fused to the N-terminus of the heavy chain variable region and/or to the C-terminus of the light chain variable region, as described in Example 7;
FIGURE 17B illustrates a MASP-2 scFv antibody scaffold comprising an SGMI peptides fused to the N-terminus of the light chain variable region and/or to the C-terminus of the heavy chain variable region, as described in Example 7;
FIGURE 18 illustrates representative MASP-2 antibody (M2)-SGMI fusions as described in Example 7
FIGURE 19 graphically illustrates the results of a C3b deposition assay carried out in 10% mouse serum with a representative naked MASP-2 (HL-M2) scaffold antibody (mAb#6) in comparison to MASP-2 antibody (M2)-SGMI fusions comprising SGMI-1 or SGMI-2, as described in Example 7;
FIGURE 20A graphically illustrates the results of a C4 deposition assay carried out in 10% human plasma with a representative naked MASP-2 (HL-M2) scaffold antibody (mAb#6) in comparison to MASP-2 antibody (M2)-SGMI fusions comprising SGMI-1 or SGMI-2, as described in Example 7;
FIGURE 20B graphically illustrates the results of a C4 deposition assay carried out in 10% human plasma with a representative naked MASP-2 (HL-M2) scaffold antibody (mAb#6) in comparison to MASP-2 antibody (M2)-SGMI fusions comprising SGMI-1 or SGMI-2, as described in Example 7;

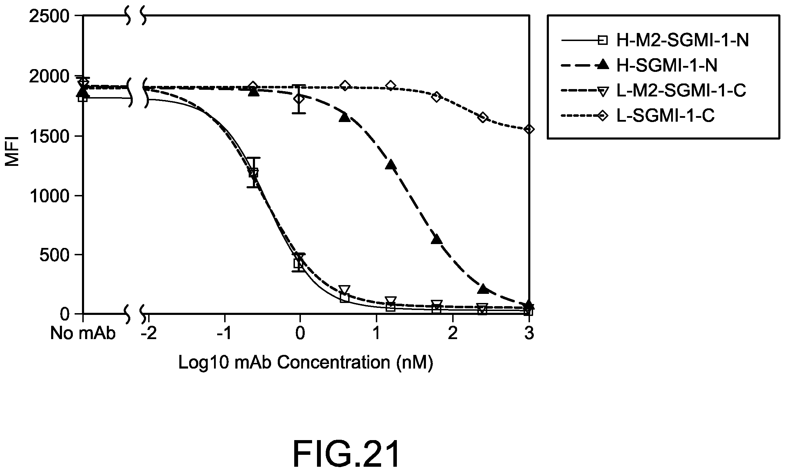

FIGURE 21 graphically illustrates the results of a C3b deposition assay carried out in 10% human serum with a non-specific chimeric chicken/human antibody comprising the SGMI-1 peptide fused to the C-terminus of the light chain constant region (L-SGMI-1-C); or a non-specific chimeric/human antibody comprising the SGMI-1 peptide fused to the N-terminus of the heavy chain variable region (H-SGMI-1-N) in comparison to the counterpart MASP-2 antibody (M2)-SGMI-1 fusions, showing synergistic lectin pathway inhibition when MASP-2 antibody and SGMI-1 are fused into the same antibody, as described in Example 7;
FIGURE 22A shows a plot of the concentration of a representative naked MASP-2 (HL-M2) scaffold antibody (mAb#6) or MASP-2 antibody (M2)-SGMI-1 fusions (pharmacokinetic (PK) data set) versus lectin pathway activity (C3b deposition; pharmacodynamics (PD) data set), wherein pharmacodynamics data from the untreated mice were used to provide common "0 nM antibody" data points for all conditions. Data from all time points were combined for each treatment group, as described in Example 8; and
FIGURE 22B shows a plot of the concentration of a representative naked MASP-2 (HL-M2) scaffold antibody (mAb#6) or MASP-2 antibody (M2)-SGMI-2 fusions (pharmacokinetic (PK) data set) versus lectin pathway activity (C3b deposition; pharmacodynamics (PD) data set), wherein pharmacodynamics data from the untreated mice were used to provide common "0 nM antibody" data points for all conditions. Data from all time points were combined for each treatment group, as described in Example 8.

### DESCRIPTION OF THE SEQUENCE LISTING

SEQ ID NO:1 human MASP-1 cDNA;
SEQ ID NO:2 human MASP-1 protein (with leader sequence);
SEQ ID NO:3 human MASP-2 cDNA;
SEQ ID NO:4 human MASP-2 protein (with leader sequence);
SEQ ID NO:5: SGMI peptide core sequence;
SEQ ID NO:6 SGMI-1L peptide (full length);
SEQ ID NO:7 SGMI-1M peptide (medium truncated version);
SEQ ID NO:8 SGMI-1S peptide (short truncated version);
SEQ ID NO:9 SGMI-2L peptide (full length);
SEQ ID NO:10 SGMI-2M peptide (medium truncated version);
SEQ ID NO: 11 SGMI-2S peptide (short truncated version);
SEQ ID NO:12 human IgG1-Fc polypeptide;
SEQ ID NO:13 peptide linker #1 (12aa);
SEQ ID NO:14: peptide linker #2 (10aa);
SEQ ID NO:15: nucleic acid encoding polypeptide fusion comprising the human IL-2-signal sequence, SGMI-1L, linker#1, and human IgG1-Fc;
SEQ ID NO:16: mature polypeptide fusion comprising SGMI-1L, linker#1 and human IgG1-Fc (SGMI-1Fc);
SEQ ID NO:17: nucleic acid encoding polypeptide fusion comprising the human IL-2-signal sequence, SGMI-2L, linker#1 and human IgG1-Fc;
SEQ ID NO:18: mature polypeptide fusion comprising SGMI-2L, linker#1 and human IgG1-Fc (SGMI-2Fc);
SEQ ID NO:19: SGMI-1 forward primer;
SEQ ID NO:20: SGMI-1 reverse primer;
SEQ ID NO:21: SGMI-2 forward primer;
SEQ ID NO:22: SGMI-2 reverse primer;
SEQ ID NO:23: parent DTLacO (clone #1) chicken heavy chain variable region (DTLacO VH);
SEQ ID NO:24: conserved FR-1 region from chicken heavy chain variable region;
SEQ ID NO:25: conserved FR-2 region from chicken heavy chain variable region;
SEQ ID NO:26: conserved FR-3 region from chicken heavy chain variable region;
SEQ ID NO:27: conserved FR-3 flanking region adjacent to CDR-H3 from chicken heavy chain variable region;
SEQ ID NO:28: conserved FR-4 region from chicken heavy chain variable region;
SEQ ID NO:29: conserved FR-4 flanking region adjacent to CDR-H3 from chicken heavy chain variable region;
SEQ ID NO:30: Parent DTLacO (clone #1) chicken light chain variable region (DTLacO VL);
SEQ ID NO:31: conserved FR-1 region from chicken light chain variable region;
SEQ ID NO:32: conserved FR-1 flanking region adjacent to CDR-L1 from chicken light chain variable region;
SEQ ID NO:33: conserved FR-2 region from chicken light chain variable region;
SEQ ID NO:34: conserved FR-2 flanking region adjacent to CDR-L1 from chicken light chain variable region;
SEQ ID NO:35: conserved FR-3 region from chicken light chain variable region;
SEQ ID NO:36: conserved FR-4 region from chicken light chain variable;
SEQ ID NO:37-46: peptide linkers
SEQ ID NO:47: human IgG1 constant region (CH1-hinge-CH2-CH3);
SEQ ID NO:48: human lambda light chain constant region;
SEQ ID NO:49: polynucleotide encoding the polypeptide comprising the human VH signal sequence, the SGMI-1L-bearing chicken VH sequence and the human IgG1 constant region (pcDNA3-SGMI-1L-IgG1);
SEQ ID NO:50: mature polypeptide comprising the SGMI-1L-bearing chicken VH region and the human IgG1 constant region (Ab-SGMI-1L-IgG1);
SEQ ID NO:51: polynucleotide encoding the polypeptide comprising the human VH signal sequence, the SGMI-1M-bearing chicken VH sequence and the human IgG1 constant region (pcDNA3-SGMI-1M-IgG1);
SEQ ID NO:52: mature polypeptide comprising the SGMI-1M-bearing chicken VH region and the human IgG1 constant region (Ab-SGMI-1M-IgG1);
SEQ ID NO:53: polynucleotide encoding the polypeptide comprising the human VH signal sequence, the SGMI-1S-bearing chicken VH sequence and the human IgG1 constant region (pcDNA3-SGMI-1S-IgG1);
SEQ ID NO:54: mature polypeptide comprising the SGMI-IS-bearing chicken VH region and the human IgG1 constant region (Ab-SGMI-1S-IgG1);
SEQ ID NO:55: polynucleotide encoding the polypeptide comprising the human VH signal sequence, the SGMI-1-L1-bearing chicken VH sequence and the human IgG1 constant region (pcDNA3-SGM1-1-L1-IgG1);
SEQ ID NO:56: mature polypeptide comprising the SGMI-1-L1-bearing chicken VH region and the human IgG1 constant region (Ab-SGMI-1-L1-IgG1);
SEQ ID NO:57: polynucleotide encoding the polypeptide comprising the human VH signal sequence, the SGMI-1-L2-bearing chicken VH sequence and the human IgG1 constant region (pcDNA3-SGMI-1-L2-IgG1);
SEQ ID NO:58: mature polypeptide comprising the SGMI-1-L2-bearing chicken VH region and the human IgG1 constant region (Ab-SGMI-I-L2-IgGl);
SEQ ID NO:59: polynucleotide encoding the polypeptide comprising the human VH signal sequence, the SGMI-1-L3-bearing chicken VH sequence and the human IgG1 constant region (pcDNA3-SGMI-1-L3-IgG1);
SEQ ID NO:60: mature polypeptide comprising the SGMI-I-L3-bearing chicken VH region and the human IgG1 constant region (Ab-SGMI-I-L3-IgGl);
SEQ ID NO:61: polynucleotide encoding the polypeptide comprising the human VH signal sequence, the SGMI-1-L4-bearing chicken VH sequence and the human IgG1 constant region (pcDNA3-SGMI-1-L4-IgG1);
SEQ ID NO:62: mature polypeptide comprising the SGMI-1-L4-bearing chicken VH region and the human IgG1 constant region (Ab-SGMI-1-L4-IgG1);
SEQ ID NO:63: polynucleotide encoding the polypeptide comprising the human VH signal sequence, the SGMI-I-L5-bearing chicken VH sequence and the human IgG1 constant region (pcDNA3-SGMI-1-L5-IgG1);
SEQ ID NO:64: mature polypeptide comprising the SGMI-I-L5-bearing chicken VH region and the human IgG1 constant region (Ab-SGMI-1-L5-IgG1);
SEQ ID NO:65: polynucleotide encoding the polypeptide comprising the human VH signal sequence, the SGMI-1-L6-bearing chicken VH sequence and the human IgG1 constant region (pcDNA3-SGMI-1-L6-IgG1);
SEQ ID NO:66: mature polypeptide comprising the SGMI-1-L6-bearing chicken VH region and the human IgG1 constant region (Ab-SGMI-1-L6-IgG1);
SEQ ID NO:67: polynucleotide encoding the polypeptide comprising the human VH signal sequence, the SGMI-1-L7-bearing chicken VH sequence and the human IgG1 constant region (pcDNA3-SGMI-1-L7-IgG1);
SEQ ID NO:68: mature polypeptide comprising the SGMI-1-L7-bearing chicken VH region and the human IgG1 constant region (Ab-SGMI-1-L7-IgG1);
SEQ ID NO:69: polynucleotide encoding the polypeptide comprising the human VH signal sequence, the SGMI-1-L8-bearing chicken VH sequence and the human IgG1 constant region (pcDNA3-SGMI-1-L8-IgG1);
SEQ ID NO:70: mature polypeptide comprising the SGMI-1-L8-bearing chicken VH region and the human IgG1 constant region (Ab-SGMI-1-L8-IgG1);
SEQ ID NO:71: polynucleotide encoding the polypeptide comprising the human VH signal sequence, the SGMI-1-L9-bearing chicken VH sequence and the human IgG1 constant region (pcDNA3-SGMI-1-L9-IgG1);
SEQ ID NO:72: mature polypeptide comprising the SGMI-1-L9-bearing chicken VH region and the human IgG1 constant region (Ab-SGMI-1-L9-IgG1);
SEQ ID NO:73: polynucleotide encoding the polypeptide comprising the human VH signal sequence, the SGMI-1-L10-bearing chicken VH sequence and the human IgG1 constant region (pcDNA3-SGMI-1-L10-IgG1);
SEQ ID NO:74: mature polypeptide comprising the SGMI-1-L10-bearing chicken VH region and the human IgG1 constant region (Ab-SGMI-1-L10-IgG1);
SEQ ID NO:75: polynucleotide encoding the polypeptide comprising the human VH signal sequence, the SGMI-1-L11-bearing chicken VH sequence and the human IgG1 constant region (pcDNA3-SGMI-1-L11-IgG1);
SEQ ID NO:76: mature polypeptide comprising the SGMI-1-L11-bearing chicken VH region and the human IgG1 constant region (Ab-SGMI-1-L11-IgG1);
SEQ ID NO:77: polynucleotide encoding the polypeptide comprising the human VH signal sequence, the SGMI-1-L12-bearing chicken VH sequence and the human IgG1 constant region (pcDNA3-SGMI-1-L12-IgG1);
SEQ ID NO:78: mature polypeptide comprising the SGMI-1-L12-bearing chicken VH region and the human IgG1 constant region (Ab-SGMI-I-LI2-IgGl);
SEQ ID NO:79: polynucleotide encoding the polypeptide comprising the human VL signal sequence, the SGMI-2L-bearing chicken VL sequence and the human Igλ constant region (pcDNA3-SGMI-2L-Igk);
SEQ ID NO:80: mature polypeptide comprising the SGMI-2L-bearing chicken VL region and the human Igλ constant region (Ab-SGMI-2L-Igλ);
SEQ ID NO:81: polynucleotide encoding the polypeptide comprising the human VL signal sequence, the SGMI-2M-bearing chicken VL sequence and the human Igλ constant region (pcDNA3-SGMI-2M-Igλ);
SEQ ID NO:82: mature polypeptide comprising the SGMI-2M-bearing chicken VL region and the human Igλ constant region (Ab-SGMI-2M-Igλ);
SEQ ID NO:83: polynucleotide encoding the polypeptide comprising the human VL signal sequence, the SGMI-2S-bearing chicken VL sequence and the human Igλ constant region (pcDNA3-SGMI-2S-Ig λ);
SEQ ID NO:84: mature polypeptide comprising the SGMI-2S-bearing chicken VL region and the human Igλ constant region (Ab-SGMI-2S-Igk);
SEQ ID NO:85: polynucleotide encoding the polypeptide comprising the SGMI-IL-bearing chicken VL region and the human Igλ constant region (pcDNA3-SGMI-1L-Igλ);
SEQ ID NO:86: mature polypeptide comprising the SGMI-1L-bearing chicken VL region and the human Igλ constant region (Ab-SGMI-1L-Igλ);
SEQ ID NO:87: polynucleotide encoding a polypeptide comprising the SGMI-1M-bearing chicken VL region and the human Igλ constant region (pcDNA3-SGMI-1M-Igλ);
SEQ ID NO:88: mature polypeptide comprising the SGMI-1M-bearing chicken VL region and the human Igλ constant region (Ab-SGMI-1M-Igλ);
SEQ ID NO:89: polynucleotide encoding a polypeptide comprising the SGMI-IS-bearing chicken VL region and the human Igλ constant region (pcDNA3-SGMI-1S-IgA);
SEQ ID NO:90: mature polypeptide comprising the SGMI-IS-bearing chicken VL region and the human Igλ constant region (Ab-SGMI-1S-Igλ);
SEQ ID NO:91: DTLacO chicken (clone #2) heavy chain variable region (DTLacO VH);
SEQ ID NO:92: DTLacO chicken (clone#2) light chain variable region (DTLacO VL);
SEQ ID NO:93: polynucleotide encoding the polypeptide comprising the human VH signal sequence, the SGMI-1-fused chicken VH sequence, and the human IgG1 constant region (pcDNA3-IgG1-S10);
SEQ ID NO:94: mature polypeptide comprising the SGMI-1-fused chicken VH region and the human IgG1 constant region (Ab-IgG1-S10);
SEQ ID NO:95: polynucleotide encoding the polypeptide comprising the human VH signal sequence, the SGMI-2-fused chicken VH sequence, and the human IgG1 constant region (pcDNA3-IgG1-S20);
SEQ ID NO:96: mature polypeptide comprising the SGMI-2-fused chicken VH region and the human IgG1 constant region (Ab-IgG1-S20);
SEQ ID NO:97: polynucleotide encoding the polypeptide comprising the human VH signal sequence, the chicken VH sequence, and the SGMI-1-fused human IgG1 constant region (pcDNA3-IgG1-S01);
SEQ ID NO:98: mature polypeptide comprising the chicken VH region and the SGMI-1-fused human IgG1 constant region (Ab-IgG1-S01);
SEQ ID NO:99: polynucleotide encoding the polypeptide comprising the human VH signal sequence, the chicken VH sequence, and the SGMI-2-fused human IgG1 constant region (pcDNA3-IgG1-S02);
SEQ ID NO: 100: mature polypeptide comprising the chicken VH region and the SGMI-2-fused human IgG1 constant region (Ab-IgGl-S02);
SEQ ID NO: 101: polynucleotide encoding the polypeptide comprising the human VL signal sequence, the SGMI-1-fused VL sequence and the human Igλ constant region (pcDNA3-Igλ-S10);
SEQ ID NO: 102: mature polypeptide comprising the SGMI-1-fused chicken VL region and the human Igλ constant region (Ab-Igλ-S10);
SEQ ID NO: 103: polynucleotide encoding the polypeptide comprising the human VL signal sequence, the SGMI-2-fused VL sequence and the human Igλ constant region (pcDNA3-Igλ-S20);
SEQ ID NO: 104: mature polypeptide comprising the SGMI-2-fused chicken VL region and the human Igλ constant region (Ab-Igk-S20);
SEQ ID NO: 105: polynucleotide encoding the polypeptide comprising the human VL signal sequence, the chicken VL sequence, and the SGMI-1-fused human Igλ constant region (pcDNA3-Igk-S01);
SEQ ID NO: 106: mature polypeptide comprising the chicken VL region, and the SGMI-1-fused human Igλ constant region (Ab-Igλ-S01;
SEQ ID NO: 107: polynucleotide encoding the polypeptide comprising the human VL signal sequence, the chicken VL sequence, and the SGMI-2-fused human Igλ constant region (pcDNA3-Igλ-S02); and
SEQ ID NO 108: mature polypeptide comprising the chicken VL region, and the SGMI-2-fused human Igλ constant region (Ab-Igλ-S02);
   **MASP-2 MONOCLONAL ANTIBODIES: VH chains**
SEQ ID NO:109 17D20mc heavy chain variable region (VH) polypeptide
SEQ ID NO:110 DNA encoding 17D20_dc35VH21N11VL heavy chain variable region (VH) (without signal peptide)
SEQ ID NO:111 17D20_dc35VH21N11VL heavy chain variable region (VH) polypeptide
SEQ ID NO:112 17N16mc heavy chain variable region (VH) polypeptide
   **MASP-2 MONOCLONAL ANTIBODIES: VL chains**
SEQ ID NO:113 17D20mc light chain variable region (VL) polypeptide
SEQ ID NO:114 DNA encoding 17D20_dc21N11VL light chain variable region (VL) (without signal peptide)
SEQ ID NO:115 17D20_dc21N11VL light chain variable region (VL) polypeptide
SEQ ID NO:116 17N16mc light chain variable region (VL) polypeptide
SEQ ID NO:117 DNA encoding 17N16_dc17N9 light chain variable region (VL) (without signal peptide)
SEQ ID NO:118 17N16_dc17N9 light chain variable region (VL) polypeptide **MASP-2 MONOCLONAL ANTIBODIES HEAVY CHAIN CDRS**
SEQ ID NOS:119-122 CDR-H1
SEQ ID NOS:123-126 CDR-H2
SEQ ID NOS:127-131 CDR-H3
   **MASP-2 MONOCLONAL ANTIBODIES LIGHT CHAIN CDRS**
SEQ ID NOS: 132-136 CDR-L1
SEQ ID NOS:137-141 CDR-L2
SEQ ID NOS:142-145 CDR-L3
SEQ ID NO:146 : consensus heavy chain CDR-H3 of 17D20m and d3521N11
SEQ ID NO: 147: consensus light chain CDR-L1 of 17D20m and d3521N11
SEQ ID NO:148: consensus light chain CDR-L1 of 17N16m and d17N9
SEQ ID NO:149: consensus light chain CDR-L2 of 17D20m, d3521N11, 17N16m and d17N9
SEQ ID NO:150: consensus light chain CDR-L3 of 17N16m and d17N9
SEQ ID NO:151: 17D20 clone in scFv format
SEQ ID NO:152: 18L16 clone in scFv format
SEQ ID NO:153: 4D9 clone in scFv format
SEQ ID NO:154: 17L20 clone in scFv format
SEQ ID NO:155: 17N16 clone in scFv format
SEQ ID NO:156: 3F22 clone in scFv format
SEQ ID NO:157: 9P13 clone in scFv format
SEQ ID NO:158: cDNA encoding wild-type IgG4
SEQ ID NO:159: wild-type IgG4 polypeptide
SEQ ID NO:160: cDNA encoding IgG4 hinge mutant S228P
SEQ ID NO:161: IgG4 mutant S228P polypeptide
SEQ ID NO:162: cDNA encoding wild-type IgG2
SEQ ID NO:163: wild-type IgG2 polypeptide
SEQ ID NO:164: NimoAb101: Heavy chain variable region (rat)
SEQ ID NO:165: NimoAb101: Light chain variable region (rat)
SEQ ID NO:166: NimoAb101: CDR-H1
SEQ ID NO:167: NimoAb101: CDR-H2
SEQ ID NO:168: NimoAb101: CDR-H3
SEQ ID NO:169: NimoAb101:CDR-L1
SEQ ID NO:170: NimoAb101:CDR-L2
SEQ ID NO:171 NimoAb 101:CDR-L3
SEQ ID NO:172-173: peptide linkers
SEQ ID NO:174: polynucleotide encoding the polypeptide comprising the VH-M2ab6-SGMI-1-N and the human IgG4 constant region with hinge mutation
SEQ ID NO:175: mature polypeptide comprising the VH-M2ab6-SGMI-1-N and the human IgG4 constant region with hinge mutation
SEQ ID NO:176: polynucleotide encoding the polypeptide comprising the VH-M2ab6-SGMI-2-N and the human IgG4 constant region with hinge mutation
SEQ ID NO:177: mature polypeptide comprising the VH-M2b6-SGMI-2-N and the human IgG4 constant region with hinge mutation
SEQ ID NO:178: polynucleotide encoding the polypeptide comprising the VH-M2ab6-SGMI-1-C and the human IgG4 constant region with hinge mutation
SEQ ID NO: 179: mature polypeptide comprising the VH-M2ab6-SGMI-1-C and the human IgG4 constant region with hinge mutation
SEQ ID NO: 180: polynucleotide encoding the polypeptide comprising the VH-M2ab 6-SGMI-2-C and the human IgG4 constant region with hinge mutation
SEQ ID NO: 181: mature polypeptide comprising the VH-M2ab6-SGMI-2-C and the human IgG4 constant region with hinge mutation
SEQ ID NO: 182: polynucleotide encoding the polypeptide comprising the VL-M2ab6-SGMI-1-N and the human Ig lambda constant region
SEQ ID NO:183: mature polypeptide comprising the VL-M2ab6-SGMI-1-N and the human Ig lambda constant region
SEQ ID NO: 184: polynucleotide encoding the polypeptide comprising the VL-M2ab6-SGMI-2-N and the human Ig lambda constant region
SEQ ID NO: 185: mature polypeptide comprising the VL-M2ab6-SGMI-2-N and the human Ig lambda constant region
SEQ ID NO: 186: polynucleotide encoding the polypeptide comprising the VL-M2ab6-SGMI-1-C and the human Ig lambda constant region
SEQ ID NO:187: mature polypeptide comprising the VL-M2ab6-SGMI-1-C and the human Ig lambda constant region
SEQ ID NO: 188: polynucleotide encoding the polypeptide comprising the VL-M2ab6-SGMI-2-C and the human Ig lambda constant region
SEQ ID NO: 189: mature polypeptide comprising the VL-M2ab6-SGMI-2-C and the human Ig lambda constant region
SEQ ID NO: 190: H-DT40-Ab-SGMI-1-N
SEQ ID NO: 191: L-DT40-Ab-SGMI-1-C
SEQ ID NO: 192-195: additional peptide linkers

### DETAILED DESCRIPTION

### I. DEFINITIONS

Unless specifically defined herein, all terms used herein have the same meaning as would be understood by those of ordinary skill in the art of the present invention. The following definitions are provided in order to provide clarity with respect to the terms as they are used in the specification and claims to describe the present invention.

As used herein, an "antibody" is an immunoglobulin molecule capable of specific binding to a target, such as a carbohydrate, polynucleotide, lipid, polypeptide, etc., through at least one epitope recognition site, located in the variable region (also referred to herein as the variable domain) of the immunoglobulin molecule. In some cases, the antibody as disclosed herein comprises a chicken variable region and further comprises a bioactive peptide amino acid sequence engrafted into a complementarity-determining region (CDR) region. In some cases, the antibody as disclosed herein comprises a human or non-human (e.g., mouse, rat, chicken, camelids, synthetic, etc.) variable region and further comprises a bioactive peptide fused to the amino and/or carboxy terminal region of the light and/or heavy chain. Examples of synthetic variable regions are provided in Holliger and Hudson, Nature Biotechnology, vol 23 (9): 1126-1136, 2005. It is not intended that the term "antibody" be limited as regard to the source of the antibody or manner in which it is made (e.g., by hybridoma, phage selection, recombinant expression, transgenic animal, peptide synthesis, etc.). As used herein, the term antibody encompasses not only intact polyclonal or monoclonal antibodies, but also fragments thereof (such as a single variable region antibody (dAb), or other known antibody fragments such as Fab, Fab', F(ab')₂, Fv and the like, single chain (ScFv), synthetic variants thereof, naturally occurring variants, fusion proteins comprising an antibody portion with an antigen-binding fragment of the required specificity, humanized antibodies, chimeric antibodies, and any other modified configuration of the immunoglobulin molecule that comprises an antigen-binding site or fragment (epitope recognition site) of the required specificity. "Diabodies", multivalent or multispecific fragments constructed by gene fusion (WO94/13804; P. Holliger et al, Proc. Natl. Acad. Sci. USA 90 6444-6448, 1993) are also a particular form of antibody contemplated herein. Minibodies comprising a scFv joined to a CH3 domain are also included herein (S. Hu et al, Cancer Res., 56, 3055-3061, 1996). See e.g., Ward, E. S. et al., Nature 341, 544-546 (1989); Bird et al, Science, 242, 423-426, 1988; Huston et al, PNAS USA, 85, 5879-5883, 1988); PCT/US92/09965; WO94/13804; P. Holliger et al, Proc. Natl. Acad. Sci. USA 90 6444-6448, 1993; Y. Reiter et al, Nature Biotech, 14, 1239-1245, 1996; S. Hu et al, Cancer Res., 56, 3055-3061, 1996. Nanobodies and maxibodies are also contemplated (see, e.g., U.S. 6,765,087, U.S. 6,838,254, WO06/079372, WO/2010037402).

As used herein, the term "hypervariable region" refers to the amino acid residues of an antibody that are responsible for antigen binding. The hypervariable region generally comprises amino acid residues from a "complementary determining region" or "CDR" (i.e., from around about residues 24-34 (L1), 50-56 (L2) and 89-97 (L3) in the light chain variable domain, and around about 31-35 (H1), 50-65 (H2) and 95-102 (H3) in the heavy chain variable domain when numbering in accordance with the Kabat numbering system as described in Kabat, et al., Sequences of Proteins of Immunological Interest, 5th Ed. Public Health Service, National Institutes of Health, Bethesda, Md (1991)); and/or those residues from a "hypervariable loop" (i.e., residues 24-34 (L1), 50-56 (L2) and 89-97 (L3) in the light chain variable domain, and 26-32 (H1), 52-56 (H2) and 95-102 (H3) in the heavy chain variable domain when numbered in accordance with the Chothia numbering system, as described in Chothia and Lesk, J. Mol. Biol. 196:901-917 (1987)); and/or those residues from a "hypervariable loop"/CDR (e.g., residues 27-38 (L1), 56-65 (L2) and 105-117 (L3) in the VL, and 27-38 (H1), 56-65 (H2), and 105-117 (H3) in the VH when numbered in accordance with the IMGT numbering system as described in Lefranc, J.P., et al., Nucleic Acids Res 27:209-212; Ruiz, M., et al., Nucleic Acids Res 28:219-221 (2000)).

As used herein, the term "engrafted into a CDR region" refers to introducing a bioactive peptide sequence into at least one CDR region of a variable region of a heavy or light chain comprising chicken framework regions (FR1, FR2, FR3 and FR4) parental generic heavy or light chain, wherein the flanking framework regions remain intact, and wherein either the entire native CDR sequence is replaced with the bioactive peptide, or at least one amino acid, at least two, at least three, at least four, at least five, or more, up to all the amino acid residues of the native CDR sequence are retained as linker sequences flanking the bioactive peptide in the heavy or light chain variable region comprising the engrafted bioactive peptide.

As used herein, the term 'fused onto a light or heavy chain" refers to fusing a bioactive peptide sequence at the amino terminal region or at the carboxy terminal region of a heavy chain or light chain of an antibody comprising a human or non-human (e.g., mouse, rat, chicken, camelids, synthetic, etc) variable region.

The term "antigen-binding fragment" as used herein refers to a polypeptide fragment that contains at least one CDR of an immunoglobulin heavy and/or light chain that binds to an antigen of interest, including a polypeptide fragment that contains at least one bioactive peptide engrafted into a CDR, or a bioactive peptide fused to a light chain or heavy chain, wherein the polypeptide fragment binds to a target of the bioactive peptide, such as MASP-1 or MASP-2. In this regard, an antigen-binding fragment of the herein described antibodies may comprise 1, 2, 3, 4, 5, or all 6 CDRs of a VH and VL sequence set forth herein, wherein the antibodies bind a target of a bioactive peptide of interest, such as MASP-1 or MASP-2. An antigen-binding fragment of the herein described MASP-1 or MASP-2-specific antibodies is capable of binding to MASP-1 or MASP-2. In certain cases, binding of an antigen-binding fragment prevents or inhibits binding of a target of a bioactive peptide of interest (e.g., a GPCR ligand to its receptor), interrupting the biological response resulting from ligand binding to the receptor. In certain cases, the antigen-binding fragment binds specifically to and/or inhibits or modulates the biological activity of a target of a bioactive peptide of interest. In certain cases, the antigen-binding fragment inhibits complement activation. In certain cases, the antigen-binding fragment inhibits lectin pathway complement activation. In certain cases, the antigen-binding fragment binds specifically to and/or inhibits or modulates the biological activity of human MASP-1 and/or human MASP-2. In certain cases, the antigen-binding fragment refers to a polypeptide fragment that contains at least one CDR of an immunoglobulin heavy and/or light chain that bind to MASP-2 (e.g., human MASP-2). In one case, an antigen-binding fragment of the herein described antibodies may comprise 1, 2, 3, 4, 5 or all 6 CDRs of a VH and VL sequence set forth herein from antibodies that bind MASP-2. An antigen-binding fragment of the herein described MASP-2 specific antibodies is capable of binding to MASP-2. In certain cases, the antigen-binding fragment binds specifically to and/or inhibits or modulates the biological activity of human MASP-2 and/or human MASP-1. In certain cases, an antigen-binding fragment inhibits MASP-2 and/or MASP-1-dependent complement activation.

The term "antigen" refers to a molecule or a portion of a molecule capable of being bound by a selective binding agent, such as an antibody of interest, including a target molecule or a portion of a molecule capable of being bound by a bioactive peptide of interest, and/or additionally capable of being used in an animal to produce antibodies capable of binding to an epitope of that antigen. An antigen may have one or more epitopes.

As used herein, an "epitope" refers to the site on a protein (e.g., a target of an antibody or bioactive peptide (e.g., SGMI peptide) such as a MASP-1 or MASP-2 protein) that is bound by an antibody. "Overlapping epitopes" include at least one (e.g., two, three, four, five, or six) common amino acid residue(s), including linear and nonlinear epitopes. In certain cases, epitope determinants include chemically active surface groupings of molecules such as amino acids, sugar side chains, phosphoryl or sulfonyl, and may in certain cases have specific three-dimensional structural characteristics, and/or specific charge characteristics. In certain cases, an antibody is said to specifically bind a protein target when it preferentially recognizes its target protein in a complex mixture of proteins and/or macromolecules. An antibody is said to specifically bind a target protein (also referred to as a target antigen) when the equilibrium dissociation constant is less than or equal to 10⁻⁶ M, or less than or equal to 10⁻⁷ M, or less than or equal to 10⁻⁸ M. In some cases, the equilibrium dissociation constant may be less than or equal to 10⁻⁹ M or less than or equal to 10⁻¹⁰ M.

The proteolytic enzyme papain preferentially cleaves IgG molecules to yield several fragments, two of which (the F(ab) fragments) each comprise a covalent heterodimer that includes an intact antigen-binding site. The enzyme pepsin is able to cleave IgG molecules to provide several fragments, including the F(ab')₂ fragment which comprises both antigen-binding sites. An Fv fragment for use according to certain cases of the present invention can be produced by preferential proteolytic cleavage of an IgM, and on rare occasions of an IgG or IgA immunoglobulin molecule. Fv fragments are, however, more commonly derived using recombinant techniques known in the art. The Fv fragment includes a non-covalent V_{H}::V_{L} heterodimer including an antigen-binding site which retains much of the antigen recognition and binding capabilities of the native antibody molecule. See e.g., Inbar et al. (1972) Proc. Nat. Acad. Sci. USA 69:2659-2662; Hochman et al. (1976) Biochem 15:2706-2710; and Ehrlich et al. (1980) Biochem 19:4091-4096.

In certain cases, single chain Fv or scFV antibodies are contemplated. For example, Kappa bodies (III et al., Prot. Eng. 10: 949-57 (1997); minibodies (Martin et al., EMBO J. 13: 5305-9 (1994); diabodies (Holliger et al., PNAS 90: 6444-8 (1993); or Janusins (Traunecker et al., EMBO J. 10: 3655-59 (1991) and Traunecker et al. Int. J. Cancer Suppl. 7: 51-52 (1992), may be prepared using standard molecular biology techniques following the teachings of the present application with regard to selecting antibodies having the desired specificity. In still other cases, bispecific or chimeric antibodies may be made that encompass the antibodies comprising engrafted bioactive peptides and/or bioactive peptide (e.g., SGMI) antibody fusions of the present disclosure. For example, a chimeric antibody may comprise CDRs and framework regions from different antibodies, while bispecific antibodies may be generated that bind specifically to the target of a first bioactive peptide (e.g., a first SGMI peptide such as SGMI-1) through one binding domain and to a target of a second bioactive peptide (e.g., a second SGMI peptide such as SGMI-2) through a second binding domain. In another case, bi-specific and/or tri-specific antibodies may be generated that bind to the target of the parent antibody through one binding domain and to a target of the first and/or second bioactive peptide through a second and/or third binding domain introduced by the presence of the bioactive peptide. These antibodies may be produced through recombinant molecular biological techniques or may be physically conjugated together.

A single chain Fv (scFv) polypeptide is a covalently linked V_{H}::V_{L} heterodimer which is expressed from a gene fusion including V_{H}- and V_{L}-encoding genes linked by a peptide-encoding linker. Huston et al. (1988) Proc. Nat. Acad. Sci. USA 85(16):5879-5883. A number of methods have been described to discern chemical structures for converting the naturally aggregated--but chemically separated--light and heavy polypeptide chains from an antibody variable (V) region into an scFv molecule which will fold into a three dimensional structure substantially similar to the structure of an antigen-binding site. See, e.g., U.S. Pat. Nos. 5,091,513 and 5,132,405, to Huston et al.; and U.S. Pat. No. 4,946,778, to Ladner et al. A dAb fragment of an antibody consists of a VH domain (Ward, E. S. et al., Nature 341, 544-546 (1989)).

In certain cases, an antibody as herein disclosed (e.g., a MASP-1 or MASP-2 - specific antibody) is in the form of a diabody. Diabodies are multimers of polypeptides, each polypeptide comprising a first domain comprising a binding region of an immunoglobulin light chain and a second domain comprising a binding region of an immunoglobulin heavy chain, the two domains being linked (e.g. by a peptide linker) but unable to associate with each other to form an antigen binding site: antigen binding sites are formed by the association of the first domain of one polypeptide within the multimer with the second domain of another polypeptide within the multimer (WO94/13804).

Where bispecific antibodies are to be used, these may be conventional bispecific antibodies, which can be manufactured in a variety of ways (Holliger, P. and Winter G. Current Opinion Biotechnol. 4, 446-449 (1993)), e.g. prepared chemically or from hybrid hybridomas, or may be any of the bispecific antibody fragments mentioned above. Diabodies and scFv can be constructed without an Fc region, using only variable regions, potentially reducing the effects of anti-idiotypic reaction.

Bispecific diabodies, as opposed to bispecific whole antibodies, may also be particularly useful because they can be readily constructed and expressed in *E. coli.* Diabodies (and many other polypeptides such as antibody fragments) of appropriate binding specificities can be readily selected using phage display (WO94/13804) from libraries. If one arm of the diabody is to be kept constant, for instance, with a specificity directed against antigen X, then a library can be made where the other arm is varied and an antibody of appropriate specificity selected. Bispecific whole antibodies may be made by knobs-into-holes engineering (J. B. B. Ridgeway et al, Protein Eng., 9, 616-621, 1996).

In certain cases, the antibodies described herein may be provided in the form of a UniBody^{®}. A UniBody^{®} is an IgG4 antibody with the hinge region removed (see GenMab Utrecht, The Netherlands; see also, e.g., US20090226421). This proprietary antibody technology creates a stable, smaller antibody format with an anticipated longer therapeutic window than current small antibody formats. IgG4 antibodies do not activate the complement system. Fully human IgG4 antibodies may be modified by eliminating the hinge region of the antibody to obtain half-molecule fragments having distinct stability properties relative to the corresponding intact IgG4 (GenMab, Utrecht). Halving the IgG4 molecule leaves only one area on the UniBody^{®} that can bind to cognate antigens (e.g., disease targets) and the UniBody^{®} therefore binds univalently to only one site on target cells. For certain cancer cell surface antigens, this univalent binding may not stimulate the cancer cells to grow as may be seen using bivalent antibodies having the same antigen specificity, and hence UniBody^{®} technology may afford treatment options for some types of cancer that may be refractory to treatment with conventional antibodies. The UniBody^{®} is about half the size of a regular IgG4 antibody. This small size can be a great benefit when treating some forms of cancer, allowing for better distribution of the molecule over larger solid tumors and potentially increasing efficacy.

In certain cases, the antibodies of the present disclosure may take the form of a nanobody. Nanobodies are encoded by single genes and are efficiently produced in almost all prokaryotic and eukaryotic hosts e.g. *E. coli* (see e.g. U.S. Pat. No. 6,765,087), molds (for example *Aspergillus* or *Trichoderma*) and yeast (for example *Saccharomyces, Kluyvermyces, Hansenula* or *Pichia* (see e.g. U.S. Pat. No. 6,838,254). The production process is scalable and multi-kilogram quantities of nanobodies have been produced. Nanobodies may be formulated as a ready-to-use solution having a long shelf life. The Nanoclone method (see eg. WO 06/079372) is a proprietary method for generating Nanobodies against a desired target, based on automated high-throughput selection of B-cells.

In certain cases, antibodies and antigen-binding fragments thereof as described herein include a heavy chain and a light chain CDR set, respectively interposed between a heavy chain and a light chain framework region (FR) set which provide support to the CDRs and define the spatial relationship of the CDRs relative to each other. As used herein, the term "CDR set" refers to the three hypervariable regions of a heavy or light chain V region. Proceeding from the N-terminus of a heavy or light chain, these regions are denoted as "CDR1," "CDR2," and "CDR3" respectively. An antigen-binding site, therefore, includes six CDRs, comprising the CDR set from each of a heavy and a light chain V region. A polypeptide comprising a single CDR, (e.g., a CDR1, CDR2 or CDR3) is referred to herein as a "molecular recognition unit." Crystallographic analysis of a number of antigen-antibody complexes has demonstrated that the amino acid residues of CDRs form extensive contact with bound antigen, wherein the most extensive antigen contact is with the heavy chain CDR3. Thus, the molecular recognition units are primarily responsible for the specificity of an antigen-binding site.

As used herein, the term "FR set" refers to the four flanking amino acid sequences which frame the CDRs of a CDR set of a heavy or light chain V region. Some FR residues may contact bound antigen; however, FRs are primarily responsible for folding the V region into the antigen-binding site, particularly the FR residues directly adjacent to the CDRs. Within FRs, certain amino residues and certain structural features are very highly conserved. In this regard, all V region sequences contain an internal disulfide loop of around 70-90 amino acid residues. When the V regions fold into a binding-site, the CDRs are displayed as projecting loop motifs which form an antigen-binding surface. It is generally recognized that there are conserved structural regions of FRs which influence the folded shape of the CDR loops into certain "canonical" structures--regardless of the precise CDR amino acid sequence. Further, certain FR residues are known to participate in non-covalent interdomain contacts which stabilize the interaction of the antibody heavy and light chains.

As used herein, the term "chicken framework region or a variant thereof' refers to the FR regions of a chicken antibody, and conserved variants thereof, for example as disclosed herein and further described in Wu et al., J. Immunol 188:322-333 (2012), hereby incorporated herein by reference.

The structures and locations of immunoglobulin variable regions may be determined by reference to Kabat, E. A. et al, Sequences of Proteins of Immunological Interest. 4th Edition. US Department of Health and Human Services. 1987, and updates thereof, now available on the Internet (immuno.bme.nwu.edu).

A "monoclonal antibody" refers to a homogeneous antibody population wherein the monoclonal antibody is comprised of amino acids (naturally occurring and non-naturally occurring) that are involved in the selective binding of an epitope. Monoclonal antibodies are highly specific, being directed against a single epitope. The term "monoclonal antibody" encompasses not only intact monoclonal antibodies and full-length monoclonal antibodies, but also fragments thereof (such as Fab, Fab', F(ab')₂, Fv), single chain (ScFv), variants thereof, fusion proteins comprising an antigen-binding portion, humanized monoclonal antibodies, chimeric monoclonal antibodies, and any other modified configuration of the immunoglobulin molecule that comprises an antigen-binding fragment (epitope recognition site) of the required specificity and the ability to bind to an epitope. It is not intended to be limited as regards the source of the antibody or the manner in which it is made (e.g., by hybridoma, phage selection, recombinant expression, transgenic animals, etc.). The term includes whole immunoglobulins as well as the fragments etc. described above under the definition of "antibody."

"Humanized" antibodies refer to a chimeric molecule, generally prepared using recombinant techniques, having an antigen-binding site derived from an immunoglobulin from a non-human species (e.g., a chicken) and the remaining immunoglobulin structure of the molecule based upon the structure and/or sequence of a human immunoglobulin. The antigen-binding site may comprise either complete variable regions fused onto constant domains or only the CDRs grafted (including CDRs comprising engrafted bioactive peptide sequences) onto appropriate framework regions in the variable regions. Epitope binding sites may be wild type or modified by one or more amino acid substitutions. This eliminates the constant region as an immunogen in human individuals, but the possibility of an immune response to the foreign variable region remains (LoBuglio, A. F. et al., (1989) Proc Natl Acad Sci USA 86:4220-4224; Queen et al., PNAS (1988) 86:10029-10033; Riechmann et al., Nature (1988) 332:323-327).

In certain cases, the antibodies of the present disclosure may be chimeric antibodies. In this regard, in one case, a chimeric antibody is comprised of an antigen-binding fragment of an antibody comprising a bioactive peptide sequence engrafted into a CDR of a variable region operably linked or otherwise fused to a heterologous Fc portion of a different antibody, or fused to the N- or C- terminus of the heavy or light chain. In certain cases, the heterologous Fc domain is of human origin. In other cases, the heterologous Fc domain may be from a different Ig class from the parent antibody, including IgA (including subclasses IgA1 and IgA2), IgD, IgE, IgG (including subclasses IgG1, IgG2, IgG3, and IgG4), and IgM. In further cases, the heterologous Fc domain may be comprised of CH2 and CH3 domains from one or more of the different Ig classes. As noted above with regard to humanized antibodies, the antigen-binding fragment of a chimeric antibody may comprise only one or more of the CDRs of the antibodies described herein (e.g., 1, 2, 3, 4, 5, or 6 CDRs of the antibodies described herein), or may comprise an entire variable region (VL, VH or both).

In certain cases, an antibody comprising a bioactive peptide sequence comprises one or more of the CDRs of the antibodies described herein. In certain cases, an antibody comprising a bioactive peptide sequence comprises one or more of the CDRs of the MASP-2 specific antibodies described herein. Further in this regard, it has been shown in some cases that the transfer of only the VH-CDR3 of an antibody can be done while still retaining desired specific binding (Barbas et al., PNAS (1995) 92: 2529-2533). See also, McLane et al., PNAS (1995) 92:5214-5218, Barbas et al., J. Am. Chem. Soc. (1994) 116:2161-2162.

As used herein, the term "MASP-2-dependent complement activation" comprises MASP-2-dependent activation of the lectin pathway, which occurs under physiological conditions (i.e., in the presence of Ca⁺⁺) leading to the formation of the C3 convertase C4b2a and upon accumulation of the C3 cleavage product C3b subsequently to the C5 convertase C4b2a(C3b)n.

As used herein, the term "MASP-1-dependent complement activation" comprises MASP-1 dependent activation of the lectin pathway, which occurs under physiological conditions (i.e., in the presence of Ca⁺⁺) leading to the formation of the C3 convertase C4b2a and upon accumulation of the C3 cleavage product C3b subsequently to the C5 convertase C4b2a(C3b)n.

As used herein, the term "lectin pathway" refers to complement activation that occurs via the specific binding of serum and non-serum carbohydrate-binding proteins including mannan-binding lectin (MBL), CL-11 and the ficolins (H-ficolin, M-ficolin, or L-ficolin).

As used herein, the term "MASP-2 inhibitory antibody" refers to any MASP-2 antibody, or MASP-2 binding fragment thereof, that binds to or directly interacts with MASP-2 and effectively inhibits MASP-2-dependent complement activation. MASP-2 inhibitory antibodies useful in the method of the invention may reduce MASP-2-dependent complement activation by greater than 20%, such as greater than 30%, or greater than 40%, or greater than 50%, or greater than 60%, or greater than 70%, or greater than 80%, or greater than 90%, or greater than 95%.

As used herein, the term "MASP-1 inhibitory antibody" refers to any MASP-1 antibody, or MASP-1 binding fragment thereof, that binds to or directly interacts with MASP-1 and effectively inhibits MASP-1-dependent complement activation. MASP-1 inhibitory antibodies useful in the method of the invention may reduce MASP-1-dependent complement activation by greater than 20%, such as greater than 30%, or greater than 40%, or greater than 50%, or greater than 60%, or greater than 70%, or greater than 80%, or greater than 90%, or greater than 95%.

As used herein, the term "MASP-2 blocking antibody" refers to MASP-2 inhibitory antibodies that reduce MASP-2-dependent complement activation by greater than 90%, such as greater than 95%, or greater than 98% (i.e., resulting in MASP-2 complement activation of only 10%, such as only 9%, or only 8%, or only 7%, or only 6%, such as only 5% or less, or only 4%, or only 3% or only 2% or only 1%).

As used herein, the term "MASP-1 blocking antibody" refers to MASP-1 inhibitory antibodies that reduce MASP-1-dependent complement activation by greater than 90%, such as greater than 95%, or greater than 98% (i.e., resulting in MASP-1 complement activation of only 10%, such as only 9%, or only 8%, or only 7%, or only 6%, such as only 5% or less, or only 4%, or only 3% or only 2% or only 1%).

As used herein, the term "variant" antibody sequence refers to a molecule which differs in amino acid sequence from a "parent" or reference antibody amino acid sequence by virtue of addition, deletion, and/or substitution of one or more amino acid residue(s) in the parent antibody sequence. In one case, a variant antibody sequence refers to a molecule which contains one or more framework regions that are identical to the parent framework domains, except for a combined total of 1, 2, 3, 4, 5, 6, 7, 8 9 or 10 amino acid substitutions within the framework regions of the heavy chain variable region, and/or up to a combined total of 1, 2, 3, 4, 5, 6, 7, 8, 9, or 10 amino acid substitutions with said framework regions of the light chain variable region. In some cases, the amino acid substitutions are conservative sequence modifications. In some cases, the variant framework region(s) of the variable light chain and/or the variable heavy chain comprise or consist of an amino acid sequence having at least 85% identity, such as least 86%, or at least 87%, or at least 88% or at least 89%, or at least 90%, or at least 91%, or at least 92%, or at least 93%, or at least 94% or at least 95%, or at least 96%, or at least 97%, or at least 98% or at least 99% or 100% identity with at least one or more of the chicken framework regions VL-FR1, VL-FR2, VL-FR3 and VL-FR4 amino acid sequences set forth in SEQ ID NO:s 31, 33, 35 and 36, respectively; or with at least one or more of the chicken framework regions VH-FR-1, VH-FR2, VH-FR3 and VH-FR4 amino acid sequences set forth in SEQ ID NO:s 24, 25, 26, and 28, respectively.

In some cases, the variant framework region(s) of the variable light chain and/or the variable heavy chain comprise or consist of an amino acid sequence having at least 85% identity, such as least 86%, or at least 87%, or at least 88% or at least 89%, or at least 90%, or at least 91%, or at least 92%, or at least 93%, or at least 94% or at least 95%, or at least 96%, or at least 97%, or at least 98% or at least 99% or 100% identity with at least one or more of the MASP-2 specific antibody variable region sequences set forth in TABLES 6, 8, 9, 10, 11, 12, 13, 14, 17, 18 and 19.

As used herein, the term "MASP-2 scaffold antibody" refers to an antibody that binds to MASP-2 which is encoded by an amino acid sequence used for the preparation of an antibody comprising a bioactive peptide that inhibits complement activation, such as a MASP-2 antibody comprising an SGMI peptide sequence.

As used herein, the term "parent chicken antibody" refers to an antibody which is encoded by an amino acid sequence used for the preparation of the variant comprising a bioactive peptide engrafted into or onto at least one of the variable region of the heavy or light chain. The parent antibody has a chicken framework region and, if present, typically has human antibody constant region(s).

As used herein, the amino acid residues are abbreviated as follows: alanine (Ala;A), asparagine (Asn;N), aspartic acid (Asp;D), arginine (Arg;R), cysteine (Cys;C), glutamic acid (Glu;E), glutamine (Gln;Q), glycine (Gly;G), histidine (His;H), isoleucine (Ile;I), leucine (Leu;L), lysine (Lys;K), methionine (Met;M), phenylalanine (Phe;F), proline (Pro;P), serine (Ser;S), threonine (Thr;T), tryptophan (Trp;W), tyrosine (Tyr;Y), and valine (Val;V).

In the broadest sense, the naturally occurring amino acids can be divided into groups based upon the chemical characteristic of the side chain of the respective amino acids. By "hydrophobic" amino acid is meant either Ile, Leu, Met, Phe, Trp, Tyr, Val, Ala, Cys or Pro. By "hydrophilic" amino acid is meant either Gly, Asn, Gln, Ser, Thr, Asp, Glu, Lys, Arg or His. This grouping of amino acids can be further subclassed as follows. By "uncharged hydrophilic" amino acid is meant either Ser, Thr, Asn or Gln. By "acidic" amino acid is meant either Glu or Asp. By "basic" amino acid is meant either Lys, Arg or His.

As used herein the term "conservative amino acid substitution" is illustrated by a substitution among amino acids within each of the following groups: (1) glycine, alanine, valine, leucine, and isoleucine, (2) phenylalanine, tyrosine, and tryptophan, (3) serine and threonine, (4) aspartate and glutamate, (5) glutamine and asparagine, and (6) lysine, arginine and histidine.

As used herein, the term "isolated antibody" refers to an antibody that has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred cases, the antibody will be purified (1) to greater than 95% by weight of antibody as determined by the Lowry method, and most preferably more than 99% by weight; (2) to a degree sufficient to obtain at least 15 residues of N-terminal or internal amino acid sequence by use of a spinning cup sequenator; or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

As used herein, an "isolated nucleic acid molecule" is a nucleic acid molecule (e.g., a polynucleotide) that is not integrated in the genomic DNA of an organism. For example, a DNA molecule that encodes a growth factor that has been separated from the genomic DNA of a cell is an isolated DNA molecule. Another example of an isolated nucleic acid molecule is a chemically-synthesized nucleic acid molecule that is not integrated in the genome of an organism. A nucleic acid molecule that has been isolated from a particular species is smaller than the complete DNA molecule of a chromosome from that species.

As used herein, a "nucleic acid molecule construct" is a nucleic acid molecule, either single- or double-stranded, that has been modified through human intervention to contain segments of nucleic acid combined and juxtaposed in an arrangement not existing in nature.

As used herein, an "expression vector" is a nucleic acid molecule encoding a gene that is expressed in a host cell. Typically, an expression vector comprises a transcription promoter, a gene, and a transcription terminator. Gene expression is usually placed under the control of a promoter, and such a gene is said to be "operably linked to" the promoter. Similarly, a regulatory element and a core promoter are operably linked if the regulatory element modulates the activity of the core promoter.

As used herein, the terms "approximately" or "about" in reference to a number are generally taken to include numbers that fall within a range of 5% in either direction (greater than or less than) of the number unless otherwise stated or otherwise evident from the context (except where such number would exceed 100% of a possible value). Where ranges are stated, the endpoints are included within the range unless otherwise stated or otherwise evident from the context.

As used herein the singular forms "a", "an" and "the" include plural aspects unless the context clearly dictates otherwise. Thus, for example, reference to "a cell" includes a single cell, as well as two or more cells; reference to "an agent" includes one agent, as well as two or more agents; reference to "an antibody" includes a plurality of such antibodies and reference to "a framework region" includes reference to one or more framework regions and equivalents thereof known to those skilled in the art, and so forth.

As used herein, "a subject" includes all mammals, including without limitation, humans, non-human primates, dogs, cats, horses, sheep, goats, cows, rabbits, pigs and rodents.

As used herein, the term "bioactive peptide" refers to a peptide having a biological activity.

The term "peptide" as used herein refers to a plurality of amino acids joined together in a linear chain via peptide bonds, including a dipeptide, tripeptide, oligopeptide and polypeptide. The term oligopeptide is typically used to describe peptides having from at least 2 to about 50 or more (e.g., from 2 amino acids to 60 amino acids in length, such as from about 5 to about 50 amino acids, such as from about 5 to about 40, or from about 5 to about 30 amino acids in length). Peptides larger than 60 amino acids are referred to herein as polypeptides or proteins.

As used herein, the term "bioactive" or "bioactivity" as used herein includes, but is not limited to, any type of interaction with another biomolecule, such as a protein, glycoprotein, carbohydrate, for example an oligosaccharide or polysaccharide, nucleotide, polynucleotide, fatty acid, hormone, enzyme, cofactor or the like, whether the interactions involve covalent or noncovalent binding. Bioactivity further includes interactions of any type with other cellular components or constituents including salts, ions, metals, nutrients, foreign or exogenous agents present in a cell such as viruses, phage and the like, for example binding, sequestration or transport-related interactions. Bioactivity of a peptide can be detected, for example, by observing phenotypic effects in a host cell in which it is expressed, or by performing an *in vitro* assay for a particular bioactivity, such as affinity binding to a target molecule, alteration of an enzymatic activity, or the like. Examples of bioactive peptides include antimicrobial peptides and peptide drugs. Antimicrobial peptides are peptides that adversely affect a microbe such as a bacterium, virus, protozoan, or the like. Antimicrobial peptides include, for example, inhibitory peptides that slow the growth of a microbe, microbiocidal peptides that are effective to kill a microbe (e.g., bacteriocidal and virocidal peptide drugs, sterilants, and disinfectants), and peptides effective to interfere with microbial reproduction, host toxicity, or the like. Peptide drugs for therapeutic use in humans or other animals include, for example, antimicrobial peptides that are not prohibitively toxic to the patient, and peptides designed to elicit, speed up, slow down, or prevent various metabolic processes in the host such as insulin, oxytocin, calcitonin, gastrin, somatostatin, anticancer peptides, and the like.

As used herein, the term "wherein the isolated antibody has at least substantially the same biological activity as the unmodified bioactive peptide" refers to wherein the isolated antibody comprising the bioactive peptide sequence has at least 70%, or at least 80%, or at least 85%, or at least 90% or at least 95%, or at least 98%, or at least 99% of the biological activity as compared to the original, unmodified form of the corresponding bioactive peptide.

Each case in this specification is to be applied *mutatis mutandis* to every other case unless expressly stated otherwise.

Standard techniques may be used for recombinant DNA, oligonucleotide synthesis, and tissue culture and transformation (e.g., electroporation, lipofection). Enzymatic reactions and purification techniques may be performed according to manufacturer's specifications or as commonly accomplished in the art or as described herein. These and related techniques and procedures may be generally performed according to conventional methods well known in the art and as described in various general and more specific references that are cited and discussed throughout the present specification. See *e.g*., Sambrook et al., 2001, MOLECULAR CLONING: A LABORATORY MANUAL, 3d ed., Cold Spring Harbor Laboratory Press, Cold Spring Harbor, N.Y.; Current Protocols in Molecular Biology (Greene Publ. Assoc. Inc. & John Wiley & Sons, Inc., NY, NY); Current Protocols in Immunology (Edited by: John E. Coligan, Ada M. Kruisbeek, David H. Margulies, Ethan M. Shevach, Warren Strober 2001 John Wiley & Sons, NY, NY); or other relevant Current Protocol publications and other like references. Unless specific definitions are provided, the nomenclature utilized in connection with, and the laboratory procedures and techniques of, molecular biology, analytical chemistry, synthetic organic chemistry, and medicinal and pharmaceutical chemistry described herein are those well known and commonly used in the art. Standard techniques may be used for recombinant technology, molecular biological, microbiological, chemical syntheses, chemical analyses, pharmaceutical preparation, formulation, and delivery, and treatment of patients.

It is contemplated that any case discussed in this specification can be implemented with respect to any method, kit, reagent, or composition of the present disclosure, and vice versa. Furthermore, compositions of the invention can be used to achieve methods of the disclosure.

### II. Overview

Bioactive peptides are peptides (i.e., from 2 to 60 amino acid residues in length, such as from about 5 to about 50 amino acids, such as from about 5 to about 40 amino acids in length, such as from about 5 to about 30 amino acids in length, or such as a peptide having a length of no more than 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, or 20 amino acid residues) that elicit a biological activity. In accordance with some cases of the disclosure, the bioactive peptides comprise an amino acid sequence that inhibits complement activation. In further cases, bioactive peptides comprise an SGMI core sequence (e.g., a bioactive peptide comprising an SGMI core sequence (set forth as SEQ ID NO:5) and having a length of from 10 to 60 amino acid residues in length, such as from about 10 to about 50 amino acids, such as from about 10 to about 40 amino acids in length, such as from about 10 to about 30 amino acids in length, or such as a peptide having a length of no more than 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11 or 10 amino acid residues) that inhibit MASP-1 and/or MASP-2 activity. For example, the bioactive peptides SGMI-1 (set forth as SEQ ID NO:6) and SGMI-2 (set forth as SEQ ID NO:9) are each 36 amino acid residues in length and are highly specific inhibitors of MASP-1 and MASP-2, respectively. However, as peptide they have limited potential for use in biological studies and therapeutic applications. For example, peptide instability within the biological system of interest often occurs, as evidenced by the unwanted degradation of potential peptide drugs by proteases and/or peptidases in the host cells.

In order to engineer bioactive peptides that inhibit complement activation, such as bioactive peptides comprising an SGMI core sequence (e.g., SGMI-1 and/or SGMI-2), for use as therapeutic agents, the inventors have generated bioactive peptide-bearing antibodies and fragments thereof by engrafting amino acid sequences encoding bioactive peptides, such as bioactive peptides comprising an SGMI core sequence (e.g., SGMI-1 and/or SGMI-2) into, or fused onto, various antibody scaffolds as follows: (1) fused onto the amino terminus of human IgG1 Fc region to create an Fc-fusion protein, as described in Example 2; (2) engrafted into various complementarity-determining regions (CDR) of a non-specific chimeric chicken (variable regions) - human (IgG1 and Igλ constant regions) antibody, as described in Example 3; (3) fused onto the amino or carboxy termini of the heavy and/or light chains of a non-specific chimeric chicken (variable regions)- human (IgG1 and Igλ constant regions) antibody, as described in Example 4, and (4) fused onto the amino or carboxy termini of the heavy and/or the light chains of a human antibody, such as a human MASP-2 antibody, as described in Examples 7 and 8.

Using the methods described herein, the inventors have produced bioactive peptide-bearing antibodies and fragments thereof which surprisingly have at least substantially the same biological activity of the bioactive peptide when measured *in vitro,* with the advantages of increased stability for use as a therapeutic agent in a living subject. For example, as further described herein, in accordance with various cases of the disclosure, the inventors have generated isolated antibodies comprising bioactive peptides that inhibit complement activation, such as, for example, SGMI peptide-bearing MASP-2 antibodies and fragments thereof by fusing the SGMI core peptide amino acid sequences (e.g., SGMI-1 and/or SGMI-2) onto the amino or carboxy termini of the heavy and/or light chains of a human MASP-2 antibody, as described in Example 7. Using the methods described herein, the inventors have produced SGMI peptide-bearing MASP-2 antibodies and fragments thereof which surprisingly have enhanced inhibitory activity, as compared to the naked MASP-2 scaffold antibody that does not contain the SGMI peptide sequence, when measured in a C3b or C4b deposition assay using human serum, as described in Example 7, and also have enhanced inhibitory activity as compared to the naked MASP-2 scaffold antibody when measured in a mouse model *in vivo.*

### III. Bioactive peptide-bearing Antibodies

In accordance with the foregoing, in one aspect, the invention provides a method of making a bioactive peptide-bearing antibody, the method comprising (a) engrafting the amino acid sequence of at least one bioactive peptide of interest into (i) at least one of CDR-H1, CDR-H2 or CDR-H3 of a heavy chain variable region comprising chicken framework regions and/or (ii) at least one of CDR-L1, CDR-L2 or CDR-L3 of the light chain variable region comprising chicken framework regions, and (b) determining whether the peptide-bearing antibody has at least substantially the same or increased biological activity as compared to the isolated bioactive peptide.

The method in accordance with this aspect of the invention may be used to generate a bioactive peptide-bearing antibody, wherein the antibody comprises the amino acid sequence of any bioactive peptide of interest. Bioactive peptides have been isolated from a variety of systems, exhibit a wide range of actions, and have been utilized as therapeutic agents in the field of medicine and as diagnostic tools in both basic and applied research. The mode of action of bioactive peptides has been found to be due to the interaction of the bioactive peptide with a specific protein target. The bioactive peptide acts by binding to and either activating or inactivating its protein target with extremely high specificities. Binding constants of bioactive peptides for their protein targets typically have been determined to be in the nanomolar (nM) range with binding constants as potent as picomolar range having been reported.

The methods of this aspect of the invention may be used to generate an antibody comprising an amino acid sequence with any bioactive peptide. Exemplary bioactive peptides for use in the methods of the invention include (i) bioactive peptides that inhibit complement activation, (ii) bioactive peptides that inhibit medically-important proteases, (iii) neuropeptides (iv) bioactive peptides that inhibit or activate neuropeptide activity, (v) peptide hormones, (vi) bioactive peptides that inhibit or activate peptide hormone activity, (vii) peptides that are ligands for Class A GPCRs, (viii) bioactive peptides that inhibit or activate Class A GPCRs, (ix) Class B GPCR ligands, and (x) bioactive peptides that inhibit or activate Class B GPCRs.

For example, medically-important proteases that are inhibited by bioactive peptides include, but are not limited to: Gamma-secretase, PAR-1, PAR-2, PAR-3, Cathepsin, Incretin, Dipeptidyl peptidase IV, Angiotensin-converting enzyme, Calpain, Caspase-3, Carboxypeptidase, Thrombin, and proteases in the clotting cascade and complement pathways. Examples of complement pathway serine protease inhibitors (e.g., MASP-1, MASP-2 inhibitors), include the bioactive peptide inhibitors SGMI-1 and SGMI-2.

Examples of neuropeptides include, but are not limited to: N-Acetylaspartylglutamic acid, agouti-related peptide, alpha-endorphin, Big dynorphin, Bombesin, Bombesin-like peptides, Carbetocin, Cocaine-and-amphetamine regulated transcript (CART), Cholecystokinin, Corazonin, Corticotropin-like intermediate peptide, Cortistatin, Demoxytocin, Dynorphin A, Dynorphin B, Eledoisin, Encephalin, Galanin, Galanin-like peptide, Galmic, Galnon, Gamma-endorphin, Ghrelin, Hemopressin, Kisspeptin, Neurokinin B, Neuromedin B, Neuromedin N, Neuromedin S, Neuromedin U, Neuromedin S, Neuromedin Y, Neuropeptide Y, Neurotensin, Nociceptin, Opiorphin, Orexin, Orexin-A, Oxytocin, Physalaemin, Preprotachykinin, Proctolin, Proenkephalin, Proopiomelanocortin, Protein episteme, Relaxin-3, RVD-Pα, Somatostatin, Substance P, TAC1, Tacchykinin peptides, TRH, Vasopressin, Vasotocin, VIP, and VGF.

Examples of peptide hormones include, but are not limited to: Activin and inhibin, Adiponectin, Adipose-derived hormones, Adrenocorticotropic hormone, Afamelanotide, Agouti gene, Agouti signaling peptide, Allatostatin, Amylin, Amylin family, Angiotensin, Atrial natriuretic peptide, Big gastrin, Bovine somatotropin, Bradykinin, Brain-derived neurotrophic factor, Calcitonin, cholecystokinin, Ciliary neurotrophic factor, CJC-1293, CJC-1295, Corticotropin-releasing hormone, Cosyntropin, Crustacean neurohormone family, Endothelian, Enteroglucagon, FGF15, GFG15/19, Follicle-stimulating hormone, Gastrin, Gastroinhibitory peptide, Ghrelin, Glucagon, Glucagon-like peptide-1, Gonadotropin, Gonadotropin-preparations, Gonadotropin-releasing hormone, Granulocyte-colony-stimulating factor, Growth hormone, Growth-hormone-releasing hormone, Hepcidin, Human chorionic gonadotropin, Human placental lactogen, Incretin, Insulin, Insulin analog, Insulin aspart, Insulin degludec, Insulin glargine, Insulin lispro, Insulin-like growth factor, Insulin-like growth factor-1, Insulin-like growth factor-2, Leptin, Liraglutide, Little gastrin I, Luteinizing hormone, Melanocortin, Melanocyte-stimulating hormone, Alpha-Melanocyte-stimulating hormone, Melanotan II, Minigastrin, N-terminal prohormone of brain natriuretic peptide, Nerve growth factor, Neurotrophin-3, Neurotrophin-4, NPH insulin, Obestatin, Orexin, Osteocalcin, Pancreatic hormone, Parathyroid hormone, Peptide hormone, Peptide YY, Plasma renin activity, Pramlintide, Preprohormone, Prolactin, Relaxin, Relaxin family peptide hormone, Renin, Salcatonin, Secretin, Secretin family peptide hormone, Sincalide, Teleost leptins, Temporin, Tesamorelin, Thyroid-stimulating hormone, Thyrotropin-releasing hormone, Urocortin, Urocortin II, Urocortin III, Vasoactive intestinal peptide, and Vitellogenin.

Examples of Class B GPCR ligands include, but are not limited to: VIP (28aa), PACAP (38aa), and CRF1 (41aa).

Tables 1 and 2 list representative bioactive peptides suitable for use in the methods of the invention.

**TABLE 1: Representative Bioactive Peptides Utilized in Medicine**

| **Name** | **Isolated from** | **Size (amino acid residues)** | **Therapeutic Use** |
|---|---|---|---|
| Angiotensin II | Human Plasma | 8 | Vasoconstrictor |
| Bradykinin | Human Plasma | 9 | Vasodilator |
| Caerulein | From Skin | 10 | Choleretic Agent |
| Calcitonin | Human Parathyroid Gland | 32 | Calcium Regulator |
| Cholecystokinin | Porcine Intestine | 33 | Choleretic Agent |
| Corticotropin | Porcine Pituitary Gland | 39 | Hormone |
| Eledoisin | Octopod Venom | 11 | Hypotensive Agent |
| Gastrin | Porcine Stomach | 17 | Gastric Activator |
| Glucagon | Porcine Pancreas | 29 | Antidiabetic Agent |
| Gramicidin D | Bacillus brevis | 11 | Antibacterial Agent |
| Insulin | Canine Pancreas | | Antidiabetic Agent |
| Insulin A | | 21 | |
| Insulin B | | 30 | |
| Kallidin | Human Plasma | 10 | Vasodilator |
| Luteinizing Hormone Releasing Factor | Bovine Hypothalamus | 10 | Hormone Stimulator |
| Melittin | Bee Venom | 26 | Antirheumatic Agent |
| Oxytocin | Bovine Pituitary Gland | 9 | Oxytocic Agent |
| Secretin | Canine Intestine | 27 | Hormone |
| Sermorelin | Human Pancreas | 29 | Hormone Stimulator |
| Somatostatin | Bovine Hypothalamus | 14 | Hormone Inhibitor |
| Vasopressin | Bovine Pituitary Gland | 9 | Antidiuretic Agent |

**TABLE 2: Representative Bioactive Peptides Utilized in Applied Research**

| **Name** | **Isolated from** | **Size (amino acid residues)** | **Biological activity** |
|---|---|---|---|
| Atrial Natriuretic Peptide | Rat Atria | 28 | Natriuretic Agent |
| Peptide Bombesin | Frog Skin | 14 | Gastric Activator |
| Conantokin G | Snail Venom | 17 | Neurotransmitter |
| Conotoxin G1 | Snail Venom | 13 | Neuromuscular Inhibitor |
| Defensin HNP-1 | Human Neutrophils | 30 | Antimicrobial Agent |
| Delta Sleep-Inducing Peptide | Rabbit Brain | 9 | Neurological Affector |
| Dermaseptin | Frog Skin | 34 | Antimicrobial Agent |
| Dynorphin | Porcine Brain | 17 | Neurotransmitter |
| EETI II | *Ecballium elaterium* seeds | 29 | Protease Inhibitor |
| Endorphin | Human Brain | 30 | Neurotransmitter |
| Enkephalin | Human Brain | 5 | Neurotransmitter |
| Histatin 5 | Human Saliva | 24 | Antibacterial Agent |
| Mastoparan | Vespid Wasps | 14 | Mast Cell Degranulator |
| Magainin 1 | Frog Skin | 23 | Antimicrobial Agent |
| Melanocyte | Porcine Pituitary | 13 | Hormone Stimulator |
| Motilin | Canine Intestine | 22 | Gastric Activator |
| Neurotensin | Bovine Brain | 13 | Neurotransmitter |
| Physalaemin | Frog Skin | 11 | Hypotensive Agent |
| Substance P | Horse Intestine | 11 | Vasodilator |
| Vasoactive Intestinal Peptide | Porcine Intestine | 28 | Hormone |

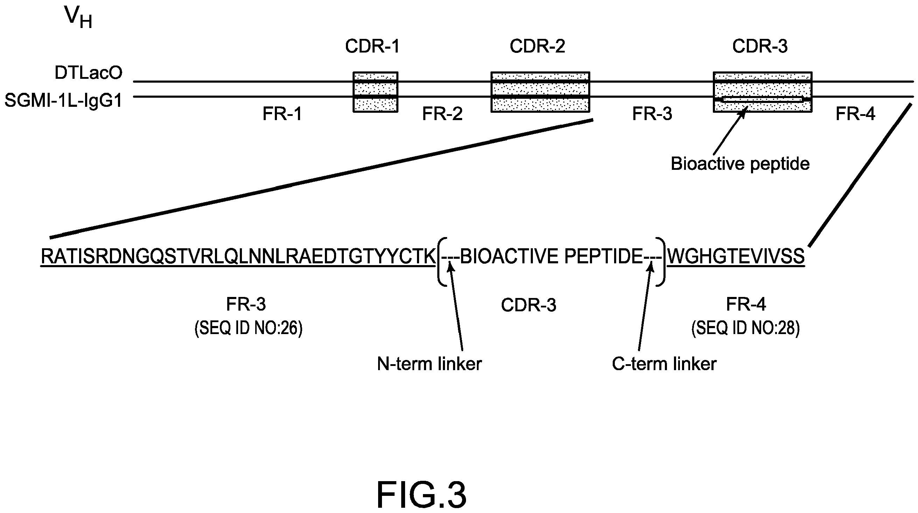

In accordance with the methods of the disclosure, an amino acid sequence of a bioactive peptide of interest is engrafted into at least one CDR region of a variable region of a heavy chain (e.g., a heavy chain comprising one or more chicken framework regions (VH-FR1, VH-FR2, VH-FR3, VH-FR4)), or is engrafted into at least one CDR region of a variable region of a light chain (e.g., a light chain comprising one or more chicken framework regions (VL-FR1, VL-FR2, VL-FR3,VL-FR4)), such as a heavy chain or light chain variable region from a parental chicken generic (i.e., non-specific) antibody, as described in Example 3 and illustrated in FIGURES 3-6. The bioactive peptide is engrafted into a CDR such that the flanking framework regions adjacent the CDR in the variable heavy or light chain remain intact. In some cases, the entire native CDR sequence of the generic parental antibody is removed and replaced with the bioactive peptide sequence.

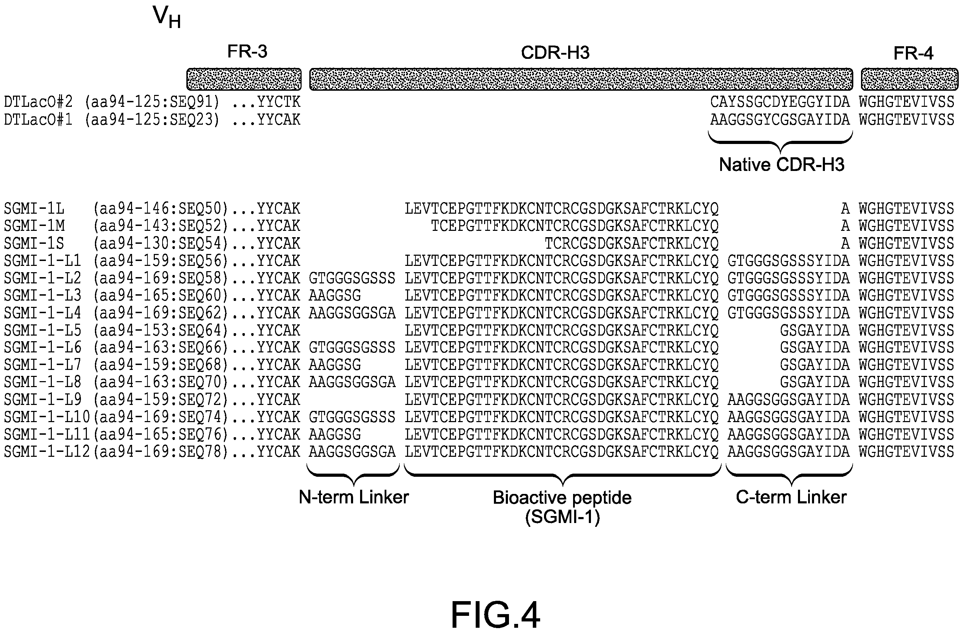

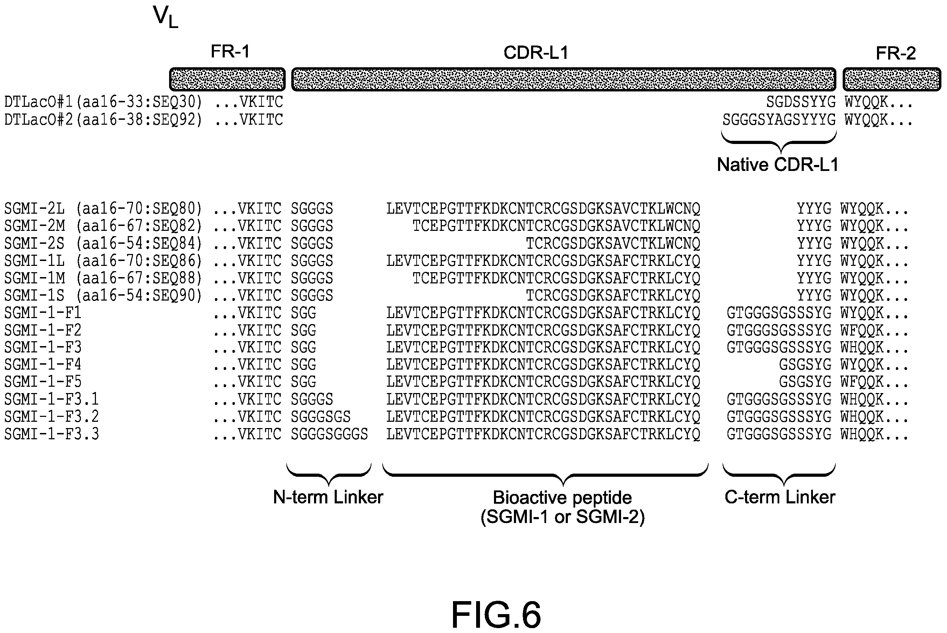

As shown in FIGURES 3-6, in some cases, at least one peptide linker sequence (typically from 1 amino acid residue to 20 amino acid residues in length) is included between the CDR-engrafted bioactive peptide amino acid sequence and one or both of the framework region(s) adjacent the bioactive peptide-bearing antibody. The peptide linker may be any flexible linker sequence, such as a flexible linker sequence shown in TABLE 4. In some cases, as illustrated in FIGURES 4 and 6, native CDR amino acid residues from the parental antibody are used to form a linker on one or both flanking regions of the bioactive peptide adjacent the framework regions. In some cases, at least one amino acid, or at least two, at least three, at least four, at least five, or more, up to all the amino acid residues of the native CDR sequence are retained as linker sequences flanking the bioactive peptide in the heavy or light chain variable region comprising the engrafted bioactive peptide.

In some cases, the bioactive peptide sequence is engrafted into a heavy chain variable region of an antibody, wherein the heavy chain variable region comprises a region having general formula (I):
N-X-B-Y-C (I) wherein:
N is an amino terminal region of the heavy chain variable region,
X is a flexible amino acid linker region and consists of 0, 1, 2, 3, 4, 5, 6, 7, 8, 9, 10, 11, 12, 13, 14, 15, 16, 17, 18, 19 or 20 amino acids;
B is a bioactive peptide amino acid sequence and consists of an amino acid sequence of no more than 60, or consists of no more than 50 amino acid residues to a minimum of at least 3 amino acid residues;
Y is a flexible amino acid linker region and consists of 0, 1, 2, 3, 4, 5, 6, 7, 8, 9, 10, 11, 12, 13, 14, 15, 16, 17, 18, 19 or 20 amino acids; and
C is a carboxy terminal region of the heavy chain variable region, and
wherein at least one of the following applies:
   N comprises FR-1, set forth as SEQ ID NO:24, or a variant thereof, and C comprises FR-2, set forth as SEQ ID NO:25, or a variant thereof; or
   N comprises FR-2, set forth as SEQ ID NO:25, or a variant thereof, and C comprises FR-3, set forth as SEQ ID NO:26, or a variant thereof; or
   N comprises FR-3, set forth as SEQ ID NO:26, or a variant thereof, or flanking SEQ ID NO:27, and C comprises FR-4, set forth as SEQ ID NO:28, or a variant thereof, or flanking SEQ ID NO:29.

In one case, a bioactive peptide sequence is engrafted into a heavy chain variable region of an antibody, wherein N comprises FR-3, set forth as SEQ ID NO:26, or a variant thereof, or flanking SEQ ID NO:27, and C comprises FR-4, set forth as SEQ ID NO:28, or a variant thereof, or flanking SEQ ID NO:29.

In one case, the heavy chain comprising one or more framework regions (eg., VH-FR1, VH-FR2, VH-FR3, VH-FR4) and at least one bioactive peptide engrafted into a CDR further comprises the human IgG1 constant region, set forth as SEQ ID NO:47, or a variant thereof.

In some cases, the bioactive peptide sequence is engrafted into a light chain variable region of an antibody, wherein the light chain variable region comprises a region having general formula (II):
N-X-B-Y-C (II) wherein:
N is an amino terminal region of the light chain variable region,
X is a flexible amino acid linker region and consists of 0, 1, 2, 3, 4, 5, 6, 7, 8, 9, 10, 11, 12, 13, 14, 15, 16, 17, 18, 19 or 20 amino acids;
B is a bioactive peptide amino acid sequence and consists of an amino acid sequence of no more than 60, or consists of no more than 50 amino acid residues to a minimum of at least 3 amino acid residues;
Y is a flexible amino acid linker region and consists of 0, 1, 2, 3, 4, 5, 6, 7, 8, 9, 10, 11, 12, 13, 14, 15, 16, 17, 18, 19 or 20 amino acids; and
C is a carboxy terminal region of the light chain variable region, and
wherein at least one of the following applies:
   N comprises FR-1, set forth as SEQ ID NO:31, or a variant thereof, or flanking SEQ ID NO:32, and C comprises FR-2, set forth as SEQ ID NO:33, or a variant thereof, or flanking SEQ ID NO:34; or
   N comprises FR-2, set forth as SEQ ID NO:33, or a variant thereof, and C comprises FR-3, set forth as SEQ ID NO:35, or a variant thereof; or
   N comprises FR-3, set forth as SEQ ID NO:35, or a variant thereof, and C comprises FR-4, set forth as SEQ ID NO:36, or a variant thereof.

In one case, a bioactive peptide is engrafted into a light chain variable region of an antibody, wherein N comprises FR-1, set forth as SEQ ID NO:31, or a variant thereof, or flanking SEQ ID NO:32, and C comprises FR-2, set forth as SEQ ID NO:33, or a variant thereof, or flanking SEQ ID NO:34.

In one case, the light chain comprising one or more framework regions (VL-FR1, VL-FR2, VL-FR3, VL-FR4) and at least one bioactive peptide engrafted into a CDR further comprises the human lambda light chain, set forth as SEQ ID NO:48, or a variant thereof.

In one case, the methods according to this aspect of the invention comprise engrafting a bioactive peptide comprising an SGMI core amino acid sequence into at least one of the heavy chain variable region and/or light chain variable region (e.g., a heavy chain variable region and/or a light chain variable region comprising chicken framework regions), wherein the SGMI core amino acid sequence comprises:
X₁CTX₂X₃X₄CX₅Q (SEQ ID NO:5)
wherein:
X₁ is F or V,
X₂ is R or K,
X₃ is K or L,
X₄ is L or W, and
X₅ is Y or N; and
wherein the bioactive peptide inhibits the activity of at least one of MASP-1 or MASP-2.

In one case, the method comprises engrafting a bioactive peptide selected from the group consisting of SEQ ID NO:6 to SEQ ID NO:11.

In one case, the method comprises engrafting a bioactive peptide that inhibits the activity of MASP-1, wherein the bioactive peptide is at least one of SEQ ID NO: 6 to 8.

In one case, the method comprises engrafting a bioactive peptide that inhibits the activity of MASP-2, wherein the bioactive peptide is at least one of SEQ ID NO: 9 to 11.

Also described herein is an isolated antibody, or antigen-binding fragment thereof, comprising one or more bioactive peptide amino acid sequence(s), wherein at least one of the bioactive peptide amino acid sequence is engrafted into at least one of: (i) a light chain variable region comprising chicken framework regions and/or (ii) a heavy chain variable region comprising chicken framework regions. In some cases, a bioactive peptide amino acid sequence is engrafted into at least one of CDR-H1, CDR-H2 or CDR-H3 of a heavy chain variable region comprising chicken framework regions. In some cases, the bioactive peptide amino acid sequence is engrafted into at least one of CDR-L1, CDR-L2 or CDR-L3 of a light chain variable region comprising chicken framework regions. Various cases of the isolated antibodies or antigen-binding fragments thereof comprising the one or more bioactive peptide amino acid sequences engrafted into one or more CDR regions of a heavy and/or light chain are generated according to the methods as described herein.

In one case, the isolated antibody or antigen binding fragment thereof comprises a bioactive peptide amino acid sequence comprising an SGMI core sequence set forth as SEQ ID NO:5. In one case, the isolated antibody or fragment thereof comprises a bioactive peptide sequence engrafted into a CDR, wherein the bioactive peptide sequence comprises or consists of at least one of SEQ ID NO:6 to SEQ ID NO:11.

In one case, the isolated antibody or antigen binding fragment thereof comprises at least one of SEQ ID NO:50, 52, 54, 56, 58, 60, 62, 64, 66, 68, 70, 72, 74, 76, 78, 80, 82, 84, 86, 88, or SEQ ID NO:90, or a variant thereof having at least 85%, or at least 88%, or at least 90%, or at least 92%, or at least 95%, or at least 98% identity to SEQ ID NO:50, 52, 54, 56, 58, 60, 62, 64, 66, 68, 70, 72, 74, 76, 78, 80, 82, 84, 86, 88, or SEQ ID NO:90. In another case, a nucleic acid molecule is provided that encodes the isolated antibody or antigen fragment thereof, the nucleic acid molecule comprising at least one of SEQ ID NO:49, 51, 53, 55, 57, 59, 61, 63, 65, 67, 69, 71, 73, 75, 77, 79, 81, 83, 85, 87 or SEQ ID NO:89, or a variant thereof having at least 85%, or at least 88%, or at least 90%, or at least 92%, or at least 95%, or at least 98% identity to SEQ ID NO:49, 51, 53, 55, 57, 59, 61, 63, 65, 67, 69, 71, 73, 75, 77, 79, 81, 83, 85, 87 or SEQ ID NO:89.

Also described herein is a method of making a bioactive peptide-bearing antibody, comprising (a) fusing the amino acid sequence of at least one bioactive peptide of interest onto: (i) an amino terminal region of at least one of: a light chain variable region and/or a heavy chain variable region, and/or (ii) a carboxy terminal region of at least one of: a light chain constant region and/or a heavy chain constant region; and (b) determining whether the peptide-bearing antibody has at least substantially the same or increased biological activity as compared to the isolated bioactive peptide. In some cases, the heavy chain variable region and/or the light chain variable region comprise non-human regions. In some cases, the heavy chain variable region and/or the light chain variable region comprise chicken regions. In some cases, the heavy chain variable region and/or the light chain variable region comprise or consist of human regions. In some cases, the bioactive peptide-bearing antibody inhibits complement activation. In some cases, the bioactive peptide-bearing antibody inhibits the lectin pathway of complement activation. In some cases, the bioactive peptide-bearing antibody inhibits the activity of at least one of MASP-1 and/or MASP-2. In some cases, the heavy chain variable region and/or the light chain variable region comprise one or more CDRs that specifically bind to MASP-2.

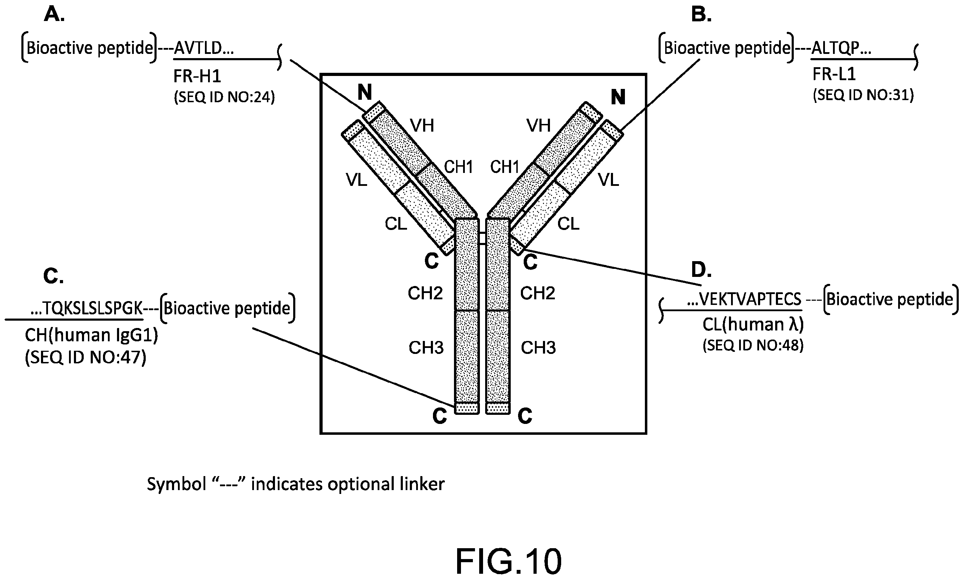

Such methods may be carried out with any bioactive peptide of interest, such as the exemplary bioactive peptides described herein. FIGURES 10 16, 17 and 18 are schematic diagrams illustrating the various cases of bioactive-peptide-bearing antibodies that may be generated using the methods of this aspect of the invention, as further described in Examples 4 and 7.

In some cases, the method of the disclosure comprises fusing the amino acid sequence of a bioactive peptide of interest to the amino terminal region of at least one of a light chain variable region (e.g., a light chain variable region comprising non-human (e.g., rat, mouse, chicken, camelid, synthetic, etc.) or human regions) and/or a heavy chain variable region (e.g., a heavy chain variable region comprising non-human (e.g., rat, mouse, chicken, camelid, synthetic, etc.) or human regions).

In some cases, the method of the disclosure comprises fusing the amino acid sequence of a bioactive peptide of interest to the carboxy terminal region of at least one of: a light chain constant region and/or a heavy chain constant region.

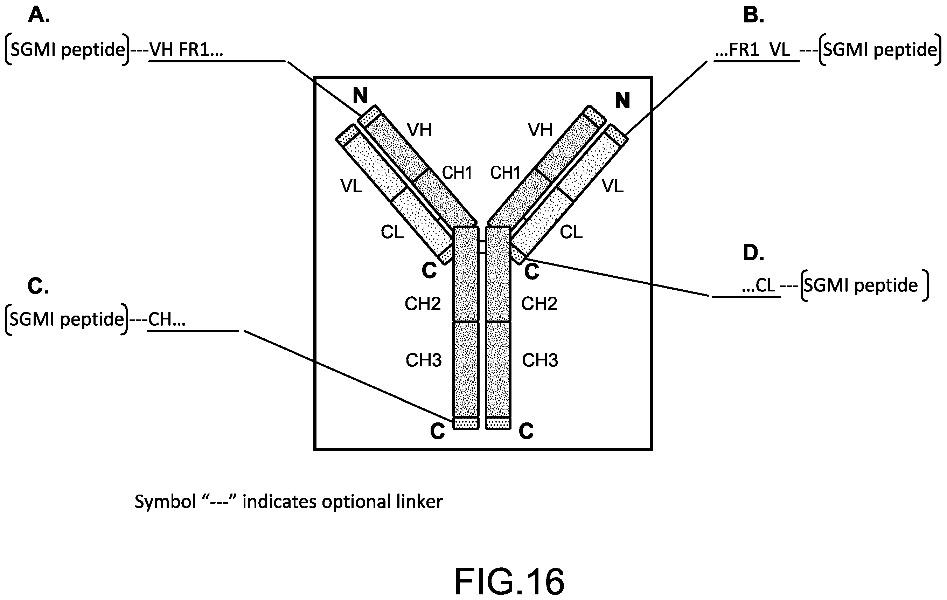

As shown in FIGURES 10 and 16, in some cases, at least one peptide linker sequence (typically from 1 amino acid residue to 20 amino acid residues) is included between the bioactive peptide sequence and the amino terminus of the light or heavy chain region, or between the bioactive peptide sequence and the carboxy terminus of the light or heavy constant region.

In some cases, a bioactive peptide of interest is fused to the amino terminus of a heavy chain variable region comprising the chicken framework regions VH-FR1, VH-FR-2, VH-FR-3 and VH-FR-4 amino acid sequences set forth as SEQ ID NO:24, 25, 26 and 28, respectively, or variants thereof. In some cases, the heavy chain further comprises a human IgG1 constant region, for example, as set forth as SEQ ID NO:47, or a variant thereof.

In some cases, a bioactive peptide of interest is fused to the carboxy terminus of a heavy chain constant region, wherein the heavy chain further comprises a variable region comprising the chicken framework regions VH-FR1, VH-FR-2, VH-FR-3 and VH-FR-4 amino acid sequences set forth as SEQ ID NO:24, 25, 26 and 28, respectively, or variants thereof.

In some cases, a bioactive peptide of interest is fused to the amino terminus of a light chain variable region comprising the chicken framework regions VL-FR1, VL-FR2, VL-FR3, VL-FR4 amino acid sequences set forth as SEQ ID NO:31, 33, 35 and 36, respectively, or variants thereof. In some cases, the light chain further comprises a human lambda light chain constant region, for example, as set forth as SEQ ID NO:48.

In some cases, such methods comprise fusing a bioactive peptide comprising an SGMI core amino acid sequence onto at least one of a heavy and/or light chain comprising non-human (e.g., rat, mouse, chicken, camelid, synthetic, etc.) or human regions, wherein the SGMI core amino acid sequence comprises:
X₁CTX₂X₃X₄CX₅Q (SEQ ID NO:5)
wherein:
X₁ is F or V,
X₂ is R or K,
X₃ is K or L,
X₄ is L or W, and
X₅ is Y or N; and
wherein the bioactive peptide inhibits the activity of at least one of MASP-1 or MASP-2.

In one case, the method comprises fusing a bioactive peptide selected from the group consisting of SEQ ID NO:6 to SEQ ID NO:11.

In one case, the method comprises fusing a bioactive peptide that inhibits the activity of MASP-1, wherein the bioactive peptide is at least one of SEQ ID NO: 6 to 8.

In one case, the method comprises fusing a bioactive peptide that inhibits the activity of MASP-2, wherein the bioactive peptide is at least one of SEQ ID NO:9 to 11.

In accordance with the foregoing, in some cases, the disclosure provides a method of making a bioactive peptide-bearing antibody that is an inhibitor of the lectin pathway wherein the heavy chain variable region and/or the light chain variable region comprise one or more CDRs that specifically bind to MASP-2 and the antibody inhibits MASP-2 activity.

Also described herein is a method of making an SGMI peptide-bearing MASP-2 antibody (i.e., an antibody scaffold comprising one or more CDRs that specifically bind to MASP-2), comprising (a) fusing the amino acid sequence of an SGMI core peptide sequence onto at least one of (i) the amino terminal region of at least one of: a light chain variable region and/or a heavy chain variable region; or (ii) the carboxy terminal region of at least one of: a light chain variable region and/or a heavy chain variable region; or (iii) the carboxy terminal region of at least one of: a light chain human constant region and/or a heavy chain human constant region; and (b) determining whether the antibody fusion is capable of inhibiting MASP-2 activity.

Such methods may be used to generate a SGMI peptide sequence-bearing MASP-2 antibody, wherein the antibody fusion is capable of inhibiting MASP-2-dependent complement activation.

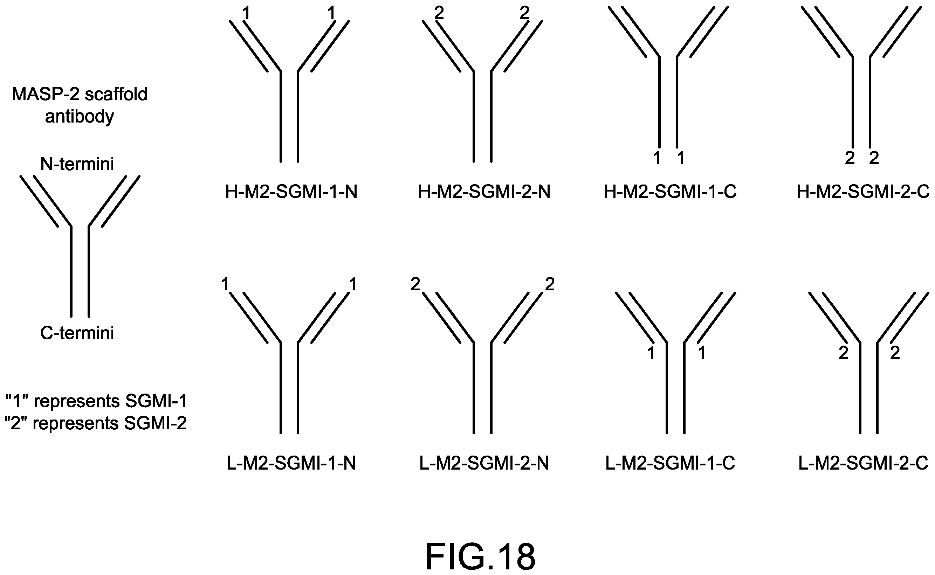

As shown in FIGURE 16, in some cases, an optional peptide linker sequence (typically from 1 amino acid residue to 20 amino acid residues in length, e.g., a flexible peptide sequence that consists of 1, 2, 3, 4, 5, 6, 7, 8, 9, 10, 11, 12, 13, 14, 15, 16, 17, 18, 19 or 20 amino acids) is included between the SGMI peptide amino acid sequence and the adjacent MASP-2 scaffold antibody sequence (e.g., the amino terminal region of at least one of: a light chain variable region and/or a heavy chain variable region; or the carboxy terminal region of at least one of: a light chain variable region and/or a heavy chain variable region; or the carboxy terminal region of at least one of: a light chain human constant region and/or a heavy chain human constant region). The peptide linker may be any flexible linker sequence, such an exemplary linker sequence provided herein, set forth as SEQ ID NO:13, 172 or 173. FIGURE 18 is a schematic diagram illustrating the various cases of the SGMI-peptide bearing MASP-2 antibodies that may be generated using the methods of this disclosure, as further described in Example 7.

Such methods may comprise fusing an SGMI core amino acid sequence onto at least one of the following regions of an antibody that specifically binds MASP-2: (i) the amino terminal region of at least one of: a light chain variable region and/or a heavy chain variable region; or (ii) the carboxy terminal region of at least one of: a light chain variable region and/or a heavy chain variable region; or (iii) the carboxy terminal region of at least one of: a light chain human constant region and/or a heavy chain human constant region, wherein the SGMI core amino acid sequence comprises:
X₁CTX₂X₃X₄CX₅Q (SEQ ID NO:5)
wherein:
X₁ is F or V,
X₂ is R or K,
X₃ is K or L,
X₄ is L or W, and
X₅ is Y or N; and
wherein the SGMI peptide sequence inhibits the activity of at least one of MASP-1 or MASP-2.

In one case, the method comprises fusing an SGMI peptide sequence selected from the group consisting of SEQ ID NO:6 to SEQ ID NO:11.

In one case, the method comprises fusing an SGMI peptide sequence that inhibits the activity of MASP-1, wherein the SGMI peptide sequence is at least one of SEQ ID NO: 6 to 8.

In one case, the method comprises fusing an SGMI peptide sequence that inhibits the activity of MASP-2, wherein the SGMI peptide sequence is at least one of SEQ ID NO: 9 to 11.

In some cases, the MASP-2 scaffold antibody without the SGMI peptide specifically binds to MASP-2 but may have weak or no MASP-2 inhibitory activity, and the MASP-2-SGMI fusion provides, or increases, MASP-2 inhibitory activity (e.g., inhibits lectin pathway activation). In some cases, the MASP-2 scaffold antibody specifically binds to MASP-2 and has an initial level of MASP-2 inhibitory activity, and the MASP-2-SGMI fusion has enhanced inhibitory activity as compared to the scaffold antibody (e.g., a statistically significant improvement in inhibitory activity, such as inhibition of lectin pathway activation, as compared to the scaffold antibody without the SGMI peptide). Non-limiting examples of suitable MASP-2 inhibitory antibody heavy chain variable regions and light chain variable regions are provided in TABLES 18 and 19, respectively.

In one case, the SGMI core peptide sequence is fused to the amino terminal region of at least one of a light chain variable region comprising one or more CDRs that bind MASP-2 and/or a heavy chain variable region comprising one or more CDRs that bind MASP-2.

In one case, the SGMI core peptide sequence is fused to the carboxy terminal region of at least one of: a light chain variable region comprising one or more CDRs that bind MASP-2 and/or to the carboxy terminal region of a heavy chain variable region comprising one or more CDRs that bind MASP-2.

In one case, the SGMI core peptide sequence is fused to the carboxy terminal region of at least one of a light chain human constant region and/or a heavy chain human constant region of an antibody that specifically binds to MASP-2.

In one case, the SGMI core peptide sequence is fused to at least one of a heavy chain variable region and/or a light chain variable region comprising one or more CDRs that specifically recognize human MASP-2 (set forth as SEQ ID NO:4).

In one case, the SGMI core peptide sequence is fused to a monoclonal MASP-2 antibody, or antigen binding fragment thereof, that specifically binds to human MASP-2 and inhibits or blocks MASP-2-dependent complement activation. MASP-2 inhibitory antibodies may effectively inhibit or effectively block the MASP-2-dependent complement activation system by inhibiting or blocking the biological function of MASP-2. For example, an inhibitory antibody may effectively inhibit or block MASP-2 protein-to-protein interactions, interfere with MASP-2 dimerization or assembly, block Ca²⁺ binding, or interfere with the MASP-2 serine protease active site.

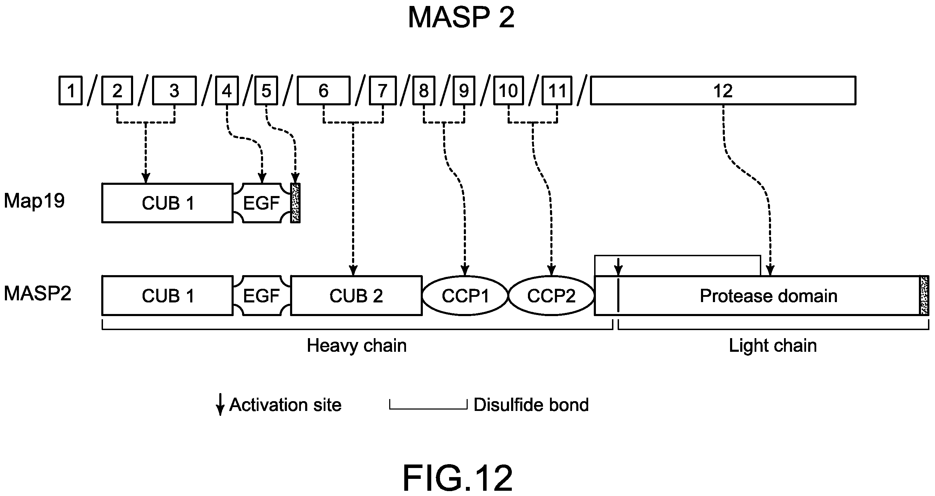

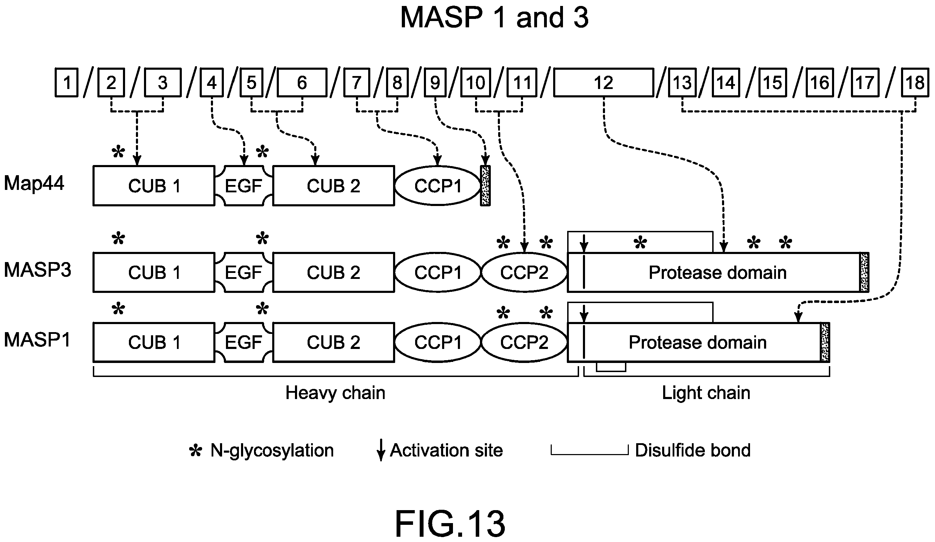

As shown in FIGURES 12 and 13, the MASP-2 polypeptide exhibits a molecular structure similar to MASP-1, MASP-3, and C1r and C1s, the proteases of the C1 complement system. The cDNA molecule set forth in SEQ ID NO:3 encodes a representative example of MASP-2 (consisting of the amino acid sequence set forth in SEQ ID NO:4) and provides the human MASP-2 polypeptide with a leader sequence (aa 1-15) that is cleaved in the process of secretion, resulting in the mature form of human MASP-2. As shown in FIGURE 12, the human *MASP2* gene encompasses twelve exons. The human MASP-2 cDNA is encoded by exons B, C, D, F, G, H, I, J, K and L. Those skilled in the art will recognize that the sequences disclosed in SEQ ID NO:3 and SEQ ID NO:4 represent single alleles of human MASP-2, and that allelic variation and alternative splicing are expected to occur. Allelic variants of the nucleotide sequences shown in SEQ ID NO:3 and SEQ ID NO:4, including those containing silent mutations and those in which mutations result in amino acid sequence changes, are within the scope of the present invention. Allelic variants of the MASP-2 sequence can be cloned by probing cDNA or genomic libraries from different individuals according to standard procedures.

The domains of the human MASP-2 protein (SEQ ID NO:4) are shown in FIGURE 12, and include an N-terminal C1r/C1s/sea urchin VEGF/bone morphogenic protein (CUBI) domain, an epidermal growth factor-like domain, a second CUB domain (CUBII), as well as a tandem of complement control protein domains CCP1 and CCP2, and a serine protease domain. Alternative splicing of the MASP-2 gene results in MAp19. MAp19 is a nonenzymatic protein containing the N-terminal CUB1-EGF region of MASP-2 with four additional residues (EQSL).

Several proteins have been shown to bind to, or interact with MASP-2 through protein-to-protein interactions. For example, MASP-2 is known to bind to, and form Ca²⁺ dependent complexes with, the lectin proteins MBL, H-ficolin and L-ficolin. Each MASP-2/lectin complex has been shown to activate complement through the MASP-2-dependent cleavage of proteins C4 and C2 (Ikeda, K., et al., J. Biol. Chem. 262:7451-7454, 1987; Matsushita, M., et al., J. Exp. Med. 176:1497-2284, 2000; Matsushita, M., et al., J. Immunol. 168:3502-3506, 2002). Studies have shown that the CUB1-EGF domains of MASP-2 are essential for the association of MASP-2 with MBL (Thielens, N.M., et al., J. Immunol. 166:5068, 2001). It has also been shown that the CUB1-EGF-CUBII domains mediate dimerization of MASP-2, which is required for formation of an active MBL complex (Wallis, R., et al., J. Biol. Chem. 275:30962-30969, 2000). Therefore, MASP-2 inhibitory antibodies can be identified that bind to or interfere with MASP-2 target regions known to be important for MASP-2-dependent complement activation.

MASP-2 antibodies for use in generating the SGMI-MASP-2 antibody fusions of the invention may bind to an epitope on one of the following MASP-2 polypeptide domains. The CUBI domain of human MASP-2 (aa 16-136 of SEQ ID NO:4); or the CUBI/EGF domains of human MASP-2 (aa 16-181 of SEQ ID NO:4); or the CUBI/EGF/CUBII domains of human MASP-2 (aa 16-292 of SEQ ID NO:4), or the EGF domain of human MASP-2 (aa 137-181 of SEQ ID NO:4), or the CCPI/CCPII/SP domains of human MASP-2 (aa 290-686 aa of SEQ ID NO:4), or the CCPI/CCPII domains of human MASP-2 (aa 293-444 of SEQ ID NO:4), or the CCPI domain of human MASP-2 (aa 293-362 of SEQ ID NO:4), or the CCPII domain of human MASP-2 (aa 363-444 of SEQ ID NO:4), or the CCPII/SP domains of human MASP-2 (aa 363- 686 of SEQ ID NO:4), or the SP domain of human MASP-2 (aa 444-6686 of SEQ ID NO:4).

In one case, the MASP-2 inhibitory scaffold antibodies for use in the invention bind to a portion of the full length human MASP-2 protein (SEQ ID NO:4), such as CUBI, EGF, CUBII, CCPI, CCPII, or SP domain of MASP-2. In some cases, the MASP-2 inhibitory antibodies of the invention bind to an epitope in the CCP1 domain of human MASP-2 (aa 293-362 of SEQ ID NO:4). For example, inhibitory MASP-2 antibodies (e.g., mAb#6) have been identified that only bind to MASP-2 fragments containing the CCP1 domain and inhibit MASP-2 dependent complement activation, as described in Example 5.

In some cases, MASP-2 scaffold antibodies for use in the invention specifically bind to human MASP-2 (set forth as SEQ ID NO:4, encoded by SEQ ID NO:3), with an affinity of at least ten times greater than to other antigens in the complement system. In some cases, the MASP-2 scaffold antibodies specifically bind to human MASP-2 with a binding affinity of at least 100 times greater than to other antigens in the complement system.

In some cases, the MASP-2 scaffold antibodies for use in the invention specifically bind to human MASP-2 with a K_{D} (dissociation constant) of less than about 100 nM, or less than about 50 nM, or less than about 25 nM, or less than about 10 nM, or less than about 5 nM, or less than or equal to about 1 nM, or less than or equal to 0.1nM. The binding affinity of the MASP-2 antibodies can be determined using a suitable binding assay known in the art, such as an ELISA assay.

Also described herein is an isolated antibody, or antigen-binding fragment thereof, comprising one or more bioactive peptide amino acid sequence(s), wherein at least one bioactive peptide amino acid sequence is fused to at least one of (i) the amino terminal region of at least one of: a light chain variable region and/or a heavy chain variable region; or (ii) the carboxy terminal region of at least one of: a light chain constant region and/or a heavy chain constant region, wherein the antibody has at least substantially the same or increased biological activity as compared to the isolated bioactive peptide.

In some cases, the heavy chain variable region and/or the light chain variable region comprises or consists of human regions. In some cases, the heavy chain variable region and/or the light chain variable region comprises non-human (e.g., rat, mouse, chicken, camelid, synthetic, etc.) regions. In some cases, the bioactive peptide-bearing antibody inhibits complement activation. In some cases, the bioactive peptide-bearing antibody inhibits the lectin pathway of complement activation. In some cases, the bioactive -peptide bearing antibody inhibits the activity of at least one of MASP-1 and/or MASP-2. In some cases, the heavy chain variable region and/or the light chain variable region comprises one or more CDRs that specifically bind to MASP-2.

Various cases of the isolated antibodies or fragments thereof comprising the one or more bioactive peptide amino acids fused to the amino terminal region of a light or heavy chain variable region, or fused to the carboxy terminal region of a light chain constant region or a heavy chain constant region are generated according to the methods as described herein.

In one case, the isolated antibody or antigen binding fragment thereof is an SGMI peptide-bearing antibody comprising a bioactive peptide amino acid sequence comprising an SGMI core sequence set forth as SEQ ID NO:5. In one case, the isolated antibody or fragment thereof comprises a bioactive peptide fused onto the amino terminal region of a light or heavy chain variable region, or fused to the carboxy terminal region of a light chain constant region or a heavy chain constant region, wherein the bioactive peptide sequence comprises or consists of at least one of SEQ ID NO:6 to SEQ ID NO:11.

In one case, the isolated antibody or antigen binding fragment thereof comprises at least one of SEQ ID NO:94, 96, 98, 100, 102, 104, 106, or SEQ ID NO:108, or a variant thereof having at least 85%, or at least 88%, or at least 90%, or at least 92%, or at least 95%, or at least 98% identity to SEQ ID NO:94, 96, 98, 100, 102, 104, 106, or SEQ ID NO: 108. In another case, a nucleic acid molecule is provided that encodes the isolated antibody or antigen fragment thereof, the nucleic acid molecule comprising at least one of SEQ ID NO:93, 95, 97, 99, 101, 103, 105 or SEQ ID NO: 107, or a variant thereof having at least 85%, or at least 88%, or at least 90%, or at least 92%, or at least 95%, or at least 98% identity to SEQ ID NO:93, 95, 97, 99, 101, 103, 105 or SEQ ID NO: 107.

Also described herein is an isolated antibody, or antigen binding fragment thereof, comprising: (i) a heavy chain variable region and/or a light chain variable region comprising one or more CDRs that specifically bind to MASP-2; and (ii) at least one SGMI core peptide sequence comprising an amino acid sequence according to: X₁CTX₂X₃X₄CX₅Q (SEQ ID NO:5) wherein: X₁ is F or V; X₂ is R or K; X₃ is K or L; X₄ is L or W; and X₅ is Y or N; and wherein the SGMI core peptide sequence is fused to at least one of: (a) the amino terminal region of at least one of: a light chain variable region and/or a heavy chain variable region; or (b) the carboxy terminal region of at least one of: a light chain variable region and/or a heavy chain variable region; or (c) the carboxy terminal region of at least one of: a light chain human constant region and/or a heavy chain human constant region; and wherein the antibody fusion protein inhibits MASP-2 dependent lectin pathway complement activation. The antibodies in accordance with this aspect of the disclosure are also referred to herein as "SGMI peptide-bearing MASP-2 antibodies." Various cases of the SGMI peptide-bearing MASP-2 antibodies, or antigen-binding fragments thereof are generated according to the methods as described herein.

### Potency of SGMIpeptide-bearing MASP-2 Antibodies

In one case, an SGMI peptide-bearing MASP-2 antibody fusion is sufficiently potent to inhibit MASP-2 dependent complement activation at an IC₅₀ ≤ 30 nM, preferably less than or about 20 nM, or less than about 10 nM or less than about 5 nM, or less than or equal to about 3nM, or less than or equal to about 1 nM when measured in 1% serum.

In one case, an SGMI peptide-bearing MASP-2 antibody fusion is sufficiently potent to inhibit MASP-2 dependent complement activation at an IC₅₀ ≤ 30 nM, preferably less than or about 20 nM, or less than about 10 nM or less than about 5 nM, or less than or equal to about 3nM, or less than or equal to about 1 nM, when measured in 90% serum.

The inhibition of MASP-2-dependent complement activation is characterized by at least one of the following changes in a component of the complement system that occurs as a result of administration of an SGMI peptide-bearing MASP-2 antibody fusion: the inhibition of the generation or production of MASP-2-dependent complement activation system products C4a, C3a, C5a and/or C5b-9 (MAC) (measured, for example, as described in Example 2 of US Patent No. 7,919,094) as well as their catabolic degradation products (e.g., C3desArg), the reduction of C4 cleavage and C4b deposition (measured, for example, as described in Example 4) and its subsequent catabolic degradation products (e.g., C4bc or C4d), or the reduction of C3 cleavage and C3b deposition (measured, for example, as described in Example 4), or its subsequent catabolic degradation products (e.g., C3bc, C3d, etc.).

In some cases, the SGMI peptide-bearing MASP-2 antibody fusion proteins of the invention are capable of inhibiting C3 deposition in full serum to less than 80%, such as less than 70%, such as less than 60%, such as less than 50%, such as less than 40%, such as less than 30%, such as less than 20%, such as less than 15%, such as less than 10% of control C3 deposition.

In some cases, the SGMI peptide-bearing MASP-2 antibody fusion proteins of the invention are capable of inhibiting C4 deposition in full serum to less than 80%, such as less than 70%, such as less than 60%, such as less than 50%, such as less than 40%, such as less than 30%, such as less than 20%, such as less than 15%, such as less than 10% of control C4 deposition.

In some cases, the SGMI peptide-bearing MASP-2 antibody fusion proteins selectively inhibit MASP-2 complement activation (i.e., bind to MASP-2 with at least 100-fold or greater affinity than to Clr or Cls), while leaving the Clq-dependent complement activation system functionally intact (*i.e.,* at least 80%, or at least 90%, or at least 95%, or at least 98%, or 100% of the classical pathway activity is retained).

In some cases, the subject SGMI peptide-bearing MASP-2 antibody fusion proteins have the following characteristics: (a) high affinity for human MASP-2 (e.g., a K_{D} of 10 nM or less, preferably a K_{D} of 1nM or less), and (b) inhibit MASP-2 dependent complement activity in 90% human serum with an IC₅₀ of 10nM or less, more preferably an IC₅₀ of 1nM or less).

In some cases, the MASP-2 antibody scaffold is fully human. In some cases, the MASP-2 antibody scaffold is humanized and comprises a MASP-2-binding site derived from an immunoglobulin from a non-human species (e.g., a rodent, such as mouse or rat, or a chicken).

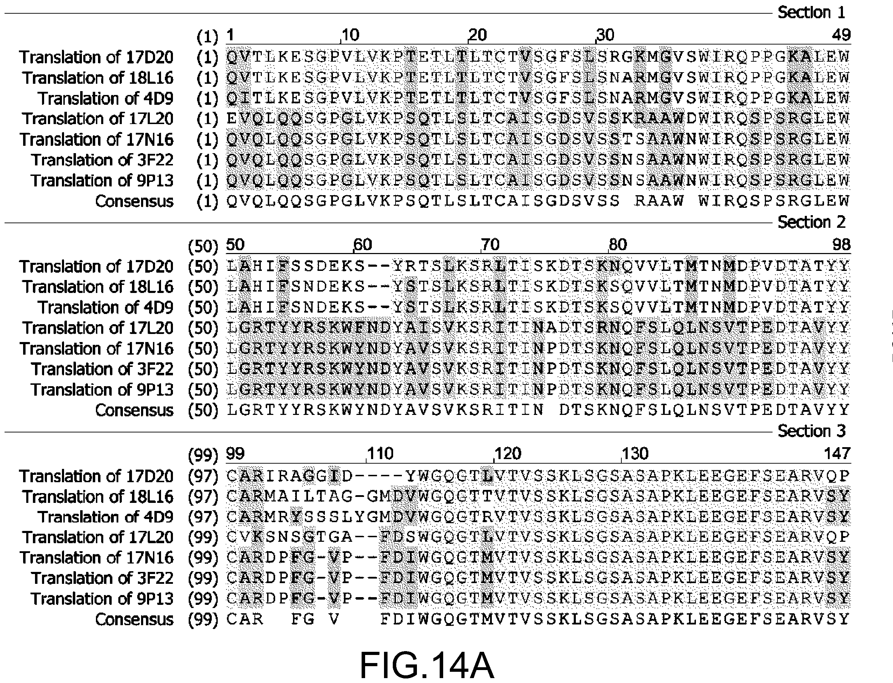

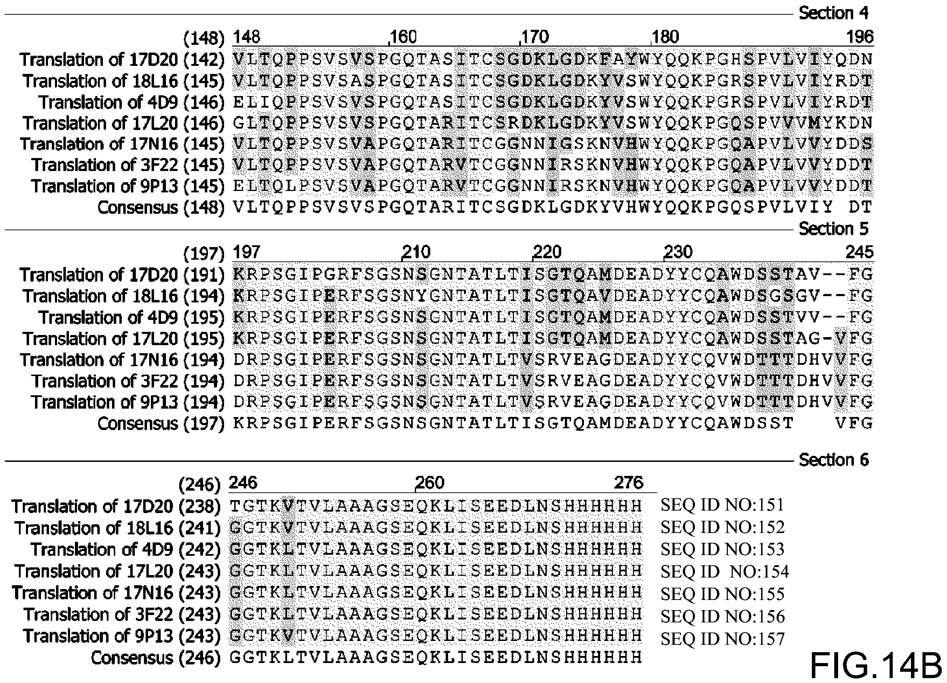

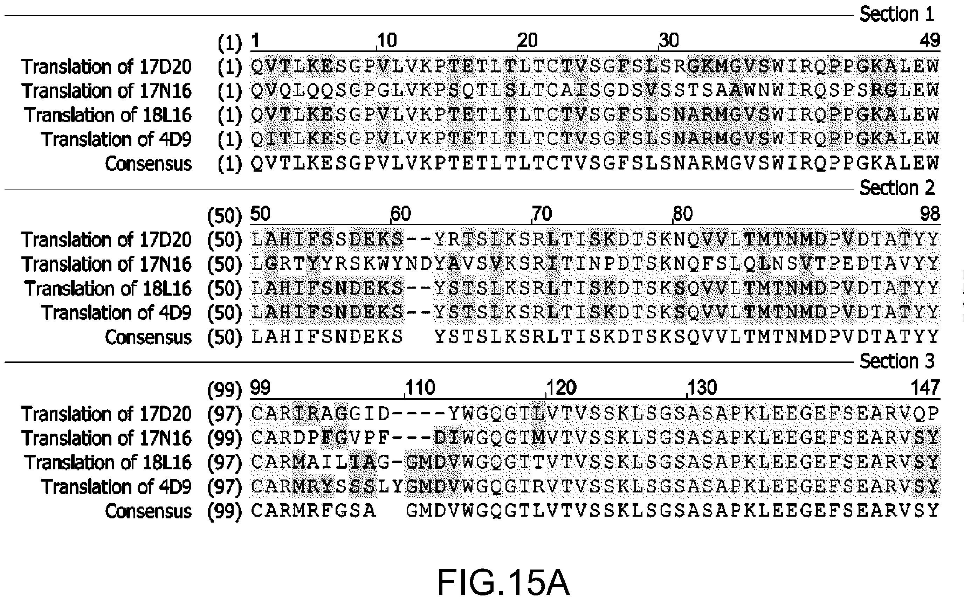

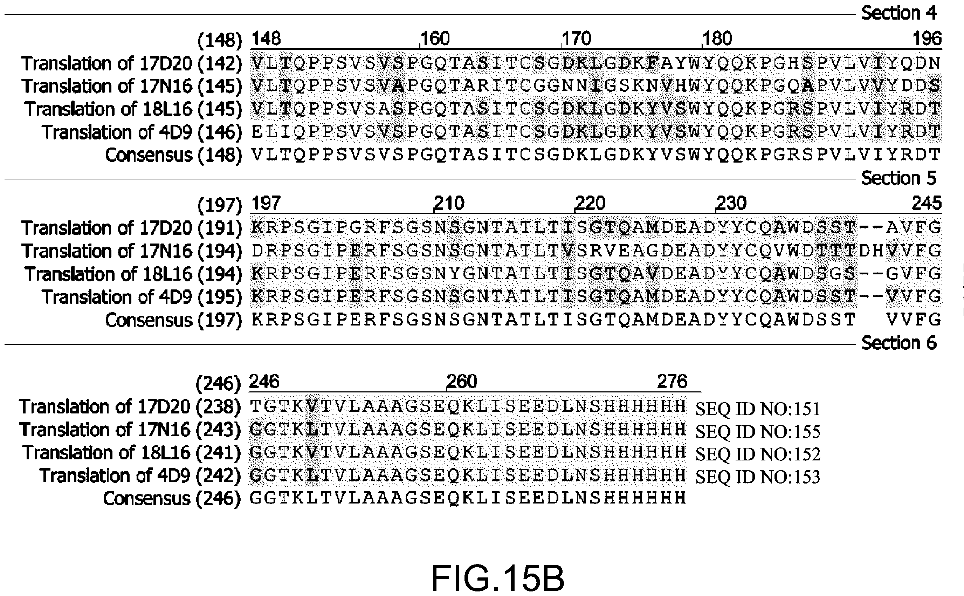

As described in Example 5, fully human antibodies have been identified that bind with high affinity to MASP-2 and inhibit lectin-mediated complement activation while leaving the classical (Clq-dependent) pathway component of the immune system intact. The variable light and heavy chain fragments of the antibodies have been sequenced, isolated and analyzed in both a scFv format and in a full length IgG format. FIGURES 14A and 14B show an amino acid sequence alignment of seven scFv anti-MASP-2 clones that were identified as having high binding affinity to MASP-2 and the ability to inhibit MASP-2 dependent activity. FIGURES 15A and 15B show an amino acid sequence alignment of four of the scFv mother clones 17D20, 17N16, 18L16 and 4D9, showing the framework regions and the CDR regions. The scFv mother clones 17D20 and 17N16 were subjected to affinity maturation, leading to the generation of daughter clones with higher affinity and increased potency as compared to the mother clones, as described in Example 5. The amino acid sequences of the heavy chain variable regions (VH) (aa 1-120) and the light chain variable regions (VL) (aa 148-250) of the scFv clones shown in FIGURES 14A and B and FIGURES 15A and B, and the resulting daughter clones, is provided below in TABLES 6, 8, 9, 10, 11, 12, 18 and 19. The amino acid sequences of exemplary SGMI peptide-bearing MASP-2 antibody fusion proteins are provided in TABLE 14.

Substitutable positions of a MASP-2 inhibitory antibody, as well the choice of amino acids that may be substituted into those positions, are revealed by aligning the heavy and light chain amino acid sequences of the MASP-2 inhibitory antibodies discussed above, and determining which amino acids occur at which positions of those antibodies. In one exemplary case, the heavy and light chain amino acid sequences of FIGURES 14A and B and FIGURES 15A and B are aligned, and the identity of amino acids at each position of the exemplary antibodies is determined. As illustrated in FIGURES 14A and B and FIGURES 15A and B (illustrating the amino acids present at each position of the heavy and light chains of the exemplary MASP-2 inhibitory antibodies), several substitutable positions, as well as the amino acid residues that can be substituted into those positions, are readily identified. In another exemplary case, the light chain amino acid sequences of the mother and daughter clones are aligned and the identity of amino acids at each position of the exemplary antibodies is determined in order to determine substitutable positions, as well as the amino acid residues that can be substituted into these positions.

In certain cases, a subject SGMI peptide-bearing MASP-2 antibody fusion protein comprises a heavy chain variable domain that is substantially identical (e.g., at least about 70%, at least 75%, at least about 80%, at least about 85%, at least about 90%, at least about 95%, or at least about 96% identical, or at least about 97% identical, or at least about 98% identical, or at least 99% identical), to that of any of the heavy chain variable domain sequences set forth in TABLE 18.

In some cases, a subject SGMI peptide-bearing MASP-2 antibody fusion protein comprises a heavy chain variable domain that is substantially identical (e.g., at least about 70%, at least 75%, at least about 80%, at least about 85%, at least about 90%, at least about 95%, or at least about 96% identical, or at least about 97% identical, or at least about 98% identical, or at least 99% identical) to 17D20 (VH), set forth as SEQ ID NO:109. In some cases, the subject SGMI peptide-bearing MASP-2 antibody fusion protein comprises a heavy chain variable domain that comprises SEQ ID NO:109.

In some cases, a subject SGMI peptide-bearing MASP-2 antibody fusion protein comprises a heavy chain variable domain that is substantially identical (e.g. at least about 70%, at least 75%, at least about 80%, at least about 85%, at least about 90%, at least about 95%, at least about 96% identical, at least about 97% identical, at least about 98% identical, or at least 99% identical) to SEQ ID NO: 111.

In some cases, a subject SGMI peptide-bearing MASP-2 antibody fusion protein comprises a heavy chain variable domain that is substantially identical (e.g., at least about 70%, at least 75%, at least about 80%, at least about 85%, at least about 90%, at least about 95%, or at least about 96% identical, or at least about 97% identical, or at least about 98% identical, or at least 99% identical) to SEQ ID NO: 112.

In some cases, a subject SGMI peptide-bearing MASP-2 antibody fusion protein comprises a light chain variable domain that is substantially identical (e.g., at least about 70%, at least 75%, at least about 80%, at least about 85%, at least about 90%, at least about 95%, or at least about 96% identical, or at least about 97% identical, or at least about 98% identical, or at least 99% identical), to that of any of the light chain variable domain sequences set forth in TABLE 19.

In some cases, a subject SGMI peptide-bearing MASP-2 antibody fusion protein comprises a light chain variable domain that is substantially identical (e.g., at least about 70%, at least 75%, at least about 80%, at least about 85%, at least about 90%, at least about 95%, or at least about 96% identical, or at least about 97% identical, or at least about 98% identical, or at least 99% identical) to SEQ ID NO: 113.

In some cases, a subject SGMI peptide-bearing MASP-2 antibody fusion protein comprises a light chain variable domain that is substantially identical (e.g., at least about 70%, at least 75%, at least about 80%, at least about 85%, at least about 90%, at least about 95%, or at least about 96% identical, or at least about 97% identical, or at least about 98% identical, or at least 99% identical) to SEQ ID NO:115.

In some cases, a subject SGMI peptide-bearing MASP-2 antibody fusion protein comprises a light chain variable domain that is substantially identical (e.g., at least about 70%, at least 75%, at least about 80%, at least about 85%, at least about 90%, at least about 95%, or at least about 96% identical, or at least about 97% identical, or at least about 98% identical, or at least 99% identical) to SEQ ID NO: 116.

In some cases, a subject SGMI peptide-bearing MASP-2 antibody fusion protein comprises a light chain variable domain that is substantially identical (e.g., at least about 70%, at least 75%, at least about 80%, at least about 85%, at least about 90%, at least about 95%, or at least about 96% identical, or at least about 97% identical, or at least about 98% identical, or at least 99% identical) to SEQ ID NO: 118.

In some cases, the SGMI peptide-bearing MASP-2 antibody fusion protein comprises a heavy or light chain that is encoded by a nucleotide sequence that hybridizes under high stringency conditions to a nucleotide sequence encoding a heavy or light chain, as set forth in SEQ ID NO:110 or SEQ ID NO: 114. High stringency conditions include incubation at 50°C or higher in 0. 1x SSC (15 mM saline/0.15mM sodium citrate).

In some cases, the SGMI peptide-bearing MASP-2 antibody fusion proteins comprise a heavy chain variable region comprising one or more CDRs (CDR1, CDR2 and/or CDR3) that are substantially identical (e.g., at least about 70%, at least 75%, at least about 80%, at least about 85%, at least about 90%, at least about 95%, or at least about 96% identical, or at least about 97% identical, or at least about 98% identical, or at least 99% identical), or comprise or consist of the identical sequence as compared to the amino acid sequence of the CDRs of any of the heavy chain variable sequences shown in FIGURES 14A and B or FIGURES 15A and B, or described below in TABLE 9.

In some cases, the SGMI peptide-bearing MASP-2 antibody fusion proteins comprise light chain variable region comprising one or more CDRs (CDR1, CDR2 and/or CDR3) that are substantially identical (e.g., at least about 70%, at least 75%, at least about 80%, at least about 85%, at least about 90%, at least about 95%, or at least about 96% identical, or at least about 97% identical, or at least about 98% identical, or at least 99% identical), or comprise or consist of the identical sequence as compared to the amino acid sequence of the CDRs of any of the light chain variable sequences shown in FIGURES 14A and B or FIGURES 15A and B, or described below in TABLE 11.

In some cases, the SGMI peptide-bearing MASP-2 antibody fusion proteins comprise a heavy chain variable region CDR-H3 sequence comprising an amino acid sequence set forth as SEQ ID NO:129 or SEQ ID NO:146 and conservative sequence modifications thereof.

In some cases, the SGMI peptide-bearing MASP-2 antibody fusion proteins comprise a light chain variable region CDR-L3 sequence comprising an amino acid sequence set forth as SEQ ID NO:142 or SEQ ID NO:150 and conservative sequence modifications thereof.

In some cases, the SGMI peptide-bearing MASP-2 antibody fusion proteins comprise a heavy chain variable region CDR-H2 sequence comprising an amino acid sequence set forth as SEQ ID NO:123 or SEQ ID NO:124, and conservative sequence modifications thereof.

In some cases, the SGMI peptide-bearing MASP-2 antibody fusion proteins comprise a heavy chain variable region CDR-H1 sequence comprising an amino acid sequence set forth as SEQ ID NO: 119 or SEQ ID NO: 120 and conservative modifications thereof.

In some cases, the SGMI peptide-bearing MASP-2 antibody fusion proteins comprise a light chain variable region CDR-L2 sequence comprising an amino acid sequence set forth as SEQ ID NO: 149 and conservative modifications thereof.

In some cases, the SGMI peptide-bearing MASP-2 antibody fusion proteins comprise a light chain variable region CDR-L1 sequence comprising an amino acid sequence set forth as SEQ ID NO:147 or SEQ ID NO:148 and conservative modifications thereof.

In some cases, the SGMI peptide-bearing MASP-2 antibody fusion proteins comprise an amino acid sequence that is substantially identical (e.g., at least about 70%, at least 75%, at least about 80%, at least about 85%, at least about 90%, at least about 95%, or at least about 96% identical, or at least about 97% identical, or at least about 98% identical, or at least 99% identical), or comprise or consist of the identical sequence as compared to an amino acid sequence described below in TABLE 14.

In some cases, the SGMI peptide-bearing MASP-2 antibody fusion proteins comprise an amino acid sequence that is substantially identical (e.g., at least about 70%, at least 75%, at least about 80%, at least about 85%, at least about 90%, at least about 95%, or at least about 96% identical, or at least about 97% identical, or at least about 98% identical, or at least 99% identical) or comprise the identical sequence as compared to an amino acid sequence set forth as SEQ ID NO:175, SEQ ID NO:177, SEQ ID NO:179, SEQ ID NO:181; SEQ ID NO:183, SEQ ID NO:185; SEQ ID NO:187 or SEQ ID NO:189.

In another case, a nucleic acid molecule is provided that encodes the SGMI peptide-bearing MASP-2 antibody fusion protein, the nucleic acid molecule comprising at least one of SEQ ID NO:174, 176, 178, 180, 182, 184, 186, or SEQ ID NO:188, or a variant thereof having at least 85%, or at least 88%, or at least 90%, or at least 92%, or at least 95%, or at least 98% identity to SEQ ID NO: 174, 176, 178, 180, 182, 184, 186, or SEQ ID NO:188.

Also described herein is an isolated monoclonal antibody or antigen-binding fragment thereof that binds to human MASP-2 and which further comprises at least one of SGMI-1 (set forth as SEQ ID NO:6) and/or SGMI-2 (set forth as SEQ ID NO:9), wherein said antibody or antigen binding fragment thereof inhibits C4 activation on a mannan-coated substrate with an IC₅₀ of 10nM or less in 1% human serum. In one case of this aspect, said antibody or antigen binding fragment thereof specifically recognizes at least part of an epitope recognized by (i) a reference antibody comprising a heavy chain variable region as set forth in SEQ ID NO:111 and a light chain variable region as set forth in SEQ ID NO:115, or (ii) a reference antibody produced by hybridoma cell line deposited in the European Collection of Cell Cultures (ECACC), Salisbury Wiltshire, United Kingdom, under the accession number 03050904.

In accordance with the foregoing, an antibody or antigen-binding fragment thereof which further comprises at least one of SGMI-1 and/or SGMI-2 according to certain preferred cases of the present application may be one that competes for binding to human MASP-2 with any antibody described herein which both (i) specifically binds to the antigen and (ii) comprises a VH and/or VL domain disclosed herein, or comprises a CDR-H3 disclosed herein, or a variant of any of these. Competition between binding members may be assayed easily *in vitro,* for example using ELISA and/or by tagging a specific reporter molecule to one binding member which can be detected in the presence of other untagged binding member(s), to enable identification of specific binding members which bind the same epitope or an overlapping epitope. Thus, there is presently provided a specific antibody or antigen-binding fragment thereof, comprising a human antibody antigen-binding site which competes with an antibody described herein that binds to human MASP-2, such as any one of the MASP-2 antibodies described in Example 5, Example 6 and Example 7, for binding to human MASP-2.

In one case, the disclosure provides a monoclonal antibody or antigen-binding fragment thereof that binds to human MASP-2 and which comprises SGMI-1 (set forth as SEQ ID NO:6), wherein the monoclonal antibody comprises an amino acid sequence selected from the group consisting of SEQ ID NO: 175, SEQ ID NO:182 and SEQ ID NO: 187, or a variant thereof having at least 85%, or at least 88%, or at least 90%, or at least 92%, or at least 95%, or at least 98% identity to SEQ ID NO: 175, SEQ ID NO: 182 and SEQ ID NO: 187.

In one case, the disclosure provides a monoclonal antibody or antigen-binding fragment thereof that binds to human MASP-2 and which comprises SGMI-2 (set forth as SEQ ID NO:9), wherein the monoclonal antibody comprises an amino acid sequence selected from the group consisting of SEQ ID NO:181, SEQ ID NO: 185 and SEQ ID NO: 189, or a variant thereof having at least 85%, or at least 88%, or at least 90%, or at least 92%, or at least 95%, or at least 98% identity to SEQ ID NO: 181, SEQ ID NO: 185 and SEQ ID NO: 189.

### Variant MASP-2 Inhibitory Antibodies

The above-described monoclonal MASP-2 antibodies may be modified to provide variant antibodies that inhibit MASP-2 dependent complement activation. The variant antibodies may be made by substituting, adding, or deleting at least one amino acid of an above-described monoclonal antibody. In general, these variant antibodies have the general characteristics of the above-described MASP-2 antibodies and contain at least the CDRs of one of the above-described antibodies, or, in certain cases, CDRs that are very similar to the CDRs of an above-described antibody.

In the preferred case, the variant comprises one or more amino acid substitution(s) in one or more hypervariable region(s) of the parent antibody. For example, the variant may comprise at least one, e.g., from about one to about ten, such as at least 1, at least 2, at least 3, at least 4, at least 5, at least 6, at least 7, at least 8, at least 9 or at least 10 substitutions, and preferably from about two to about six, substitutions in one or more CDR regions of the parent antibody. Ordinarily, the variant will have an amino acid sequence having at least 75% amino acid sequence identity with the parent antibody heavy or light chain variable domain sequences, more preferably at least 80%, more preferably at least 85%, more preferably at least 90%, and most preferably at least 95%, or at least 96%, or at least 97%, or at least 98%, or at least 99% identity. Identity or homology with respect to this sequence is defined herein as the percentage of amino acid residues in the candidate sequence that are identical with the parent antibody residues, after aligning the sequences and introducing gaps, if necessary, to achieve the maximum percent sequence identity. The variant retains the ability to bind human MASP-2 and preferably has properties which are superior to those of the parent antibody. For example, the variant may have a stronger binding affinity and/or an enhanced ability to inhibit or block MASP-2 dependent complement activation.

To analyze such properties, one should compare a Fab form of the variant to a Fab form of the parent antibody or a full length form of the variant to a full length form of the parent antibody, for example, since it has been found that the format of the anti-MASP-2 antibody impacts its activity in the biological activity assays disclosed herein. The variant antibody of particular interest herein is one which displays at least about 10-fold, preferably at least about 20-fold, and most preferably at least about 50-fold, enhancement in biological activity when compared to the parent antibody.

The antibodies may be modified to enhance desirable properties, such as it may be desirable to control serum half-life of the antibody. In general, complete antibody molecules have a very long serum persistence, whereas fragments (<60-80 kDa) are filtered very rapidly through the kidney. Hence, if long-term action of the MASP-2 antibody is desirable, the MASP-2 antibody is preferably a complete full length IgG antibody (such as IgG2 or IgG4), whereas if shorter action of the MASP-2 antibody is desirable, an antibody fragment may be preferred. As described in Example 7, it has been determined that an S228P substitution in the hinge region of IgG4 increases serum stability. Accordingly, in some cases, the subject SGMI peptide-bearing MASP-2 antibody fusion protein is a full length IgG4 antibody with an S228P substitution.

### Single Chain Antibodies

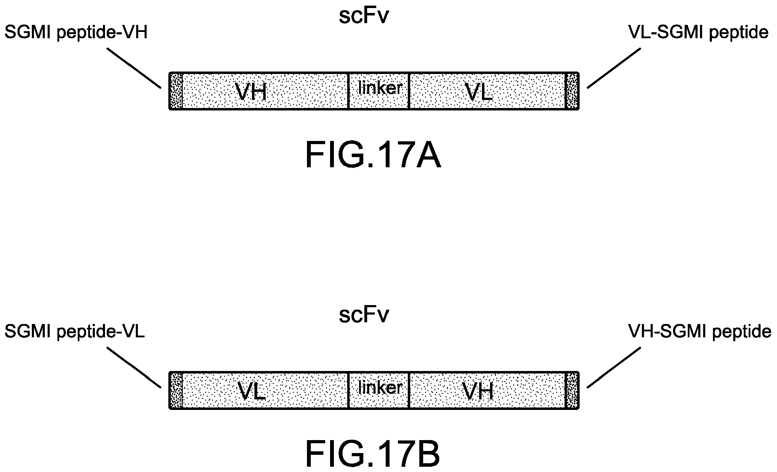

In one case, the SGMI peptide-bearing MASP-2 antibody fusion protein is a single chain antibody, defined as a genetically engineered molecule containing the variable region of the light chain, the variable region of the heavy chain, linked by a suitable polypeptide linker as a genetically fused single chain molecule, and further comprising an SGMI peptide, as illustrated in FIGURES 17A and 17B. Such single chain antibodies are also referred to as "single-chain Fv" or "scFv" antibody fragments. Generally, the Fv polypeptide further comprises a polypeptide linker between the VH and VL domains that enables the scFv to form the desired structure for antigen binding. The scFv antibodies that bind MASP-2 can be oriented with the variable light region either amino terminal to the variable heavy region or carboxyl terminal to it. Exemplary scFv antibodies of the invention are set forth in TABLE 6.

Also described herein is an isolated polypeptide comprising: (i) a region comprising an SGMI core sequence, the SGMI core sequence comprising an amino acid sequence according to: X₁CTX₂X₃X₄CX₅Q (SEQ ID NO:5), wherein:Xi is F or V, X₂ is R or K, X₃ is K or L, X₄ is L or W, and X₅ is Y or N; and (ii) a region comprising human IgG1 Fc, wherein the polypeptide inhibits the activity of at least one of MASP-1 or MASP-2.

In one case, the region comprising the human IgG1 Fc region is located at the amino terminus of the region comprising the SGMI core sequence. In another case, the region comprising the human IgG1 Fc region is located at the carboxy terminus of the region comprising the SGMI core sequence.

In one case, the region comprising the IgG1 Fc comprises or consists of SEQ ID NO:12, or a variant thereof.

In one case, the region comprising the SGMI core sequence comprises or consists of at least one of SEQ ID NO:6 to SEQ ID NO:11. In one case, the region comprising human IgG1 Fc is fused directly to at least one of SEQ ID NO:6 to SEQ ID NO:11. In one case, the polypeptide further comprises a linker region of from 1 amino acid residue to 20 amino acid residues, wherein the linker region is included between the region comprising the SGMI core sequence and the region comprising human IgG1 Fc. In one case, the linker sequence comprises at least one of SEQ ID NO: 13 or SEQ ID NO:14. In one case, the polypeptide comprises at least one of SEQ ID NO:16 or SEQ ID NO:18, or a variant thereof having at least 85%, or at least 88%, or at least 90%, or at least 92%, or at least 95%, or at least 98% identity to SEQ ID NO:16 or SEQ ID NO:18.

In another case, a nucleic acid molecule is provided that encodes the polypeptide, the nucleic acid molecule comprising at least one of SEQ ID NO:15 or SEQ ID NO:17, or a variant thereof having at least 85%, or at least 88%, or at least 90%, or at least 92%, or at least 95%, or at least 98% identity to SEQ ID NO:15 or SEQ ID NO:17.

### Methods for Producing Biopeptide-bearing Antibodies

The antibodies and polypeptides of the disclosure can be produced by standard recombinant genetic engineering methods, which are well known to those of skill in the art of molecular biology and immunology.

For recombinant production of a biopeptide-bearing antibody or fusion polypeptide of the invention, DNA sequences encoding the polypeptide components of a biopeptide-bearing antibody or fusion polypeptide (e.g., a bioactive peptide sequence and a heavy chain or light chain polypeptide sequence of interest) may be assembled using conventional methodologies. In one example, the components may be assembled separately and ligated into an appropriate expression vector. For example, the 3' end of the DNA sequence encoding one polypeptide component is ligated, with or without a peptide linker, to the 5' end of a DNA sequence encoding the second polypeptide component so that the reading frames of the sequences are in phase.

For recombinant production of a bioactive peptide sequence engrafted into a CDR region of a heavy chain variable region or a light chain variable region, the nucleic acid components may be assembled and ligated into an appropriate expression vector, with or without a peptide linker, such that the nucleic acid sequence encoding the bioactive peptide sequence is in phase with the nucleic acid sequence encoding the adjacent framework regions of the variable light chain or variable heavy chain.

As described herein, a peptide linker sequence may be employed to separate a bioactive peptide sequence (e.g., an SGMI peptide sequence) from a heterologous polypeptide sequence by some defined distance, for example a distance sufficient to ensure that the advantages of the invention are achieved, e.g., biological activity of the bioactive peptide engrafted into a CDR region, or fused onto an amino or carboxy terminal region of a heavy or light chain polypeptide. Such a peptide linker sequence may be incorporated into the bioactive peptide-bearing antibodies using standard techniques well known in the art. Suitable peptide linker sequences may be chosen based, for example, on the factors such as: (1) their ability to adopt a flexible extended conformation; and (2) their inability to adopt a secondary structure that could interfere with the activity of the bioactive peptide sequence. Illustrative peptide linker sequences, for example, may contain Gly, Asn and Ser residues. Other near neutral amino acids, such as Thr and Ala may also be used in the linker sequence. Amino acid sequences which may be usefully employed as linkers include those disclosed herein as well as those disclosed in Maratea et al., Gene 40:39-46, 1985; Murphy et al., Proc. Natl. Acad. Sci. USA 83:8258-8262, 1986; U.S. Pat. No. 4,935,233 and U.S. Pat. No. 4,751,180. The linker sequence may generally be from 1 to about 20 amino acids in length, for example.

The invention further includes nucleic acid molecules encoding the polypeptides of the invention as described herein. A vector that contains such a nucleic acid is also included. When the method is performed in a host cell, the host cell is first transformed or transfected with an exogenous nucleic acid encoding the stabilized polypeptide, then the polypeptides and antibodies are expressed and recovered. The host cells can be prokaryotic, such as bacteria, or eukaryotic, as described further herein.

In many cases, the nucleic acids encoding a subject monoclonal antibody are introduced directly into a host cell, and the cell incubated under conditions sufficient to induce expression of the encoded antibody.

In some cases, the invention provides a cell comprising a nucleic acid molecule encoding an antibody or polypeptide of the invention.

In some cases, the invention provides an expression cassette comprising a nucleic acid molecule encoding an antibody or polypeptide of the invention.

In some cases, the invention provides a method of producing an antibody or polypeptide of the invention comprising culturing a cell comprising a nucleic acid molecule encoding an antibody of the invention.

According to certain related cases there is provided a recombinant host cell which comprises one or more constructs as described herein; a nucleic acid encoding any antibody, CDR, VH or VL domain, or antigen-binding fragment thereof; and a method of production of the encoded product, which method comprises expression from encoding nucleic acid therefor. In some cases, there is provided a recombinant host cell which comprises a nucleic acid encoding an SGMI peptide-bearing MASP-2 antibody fusion protein, CDR, VH or VL domain, or antigen-binding fragment thereof; and a method of production of the encoded product, which method comprises expression from encoding nucleic acid therefor. Expression may conveniently be achieved by culturing under appropriate conditions recombinant host cells containing the nucleic acid. Following production by expression, an antibody or antigen-binding fragment thereof, may be isolated and/or purified using any suitable technique, and then used as desired.

For example, any cell suitable for expression of expression cassettes may be used as a host cell, for example, yeast, insect, plant, etc., cells. In many cases, a mammalian host cell line that does not ordinarily produce antibodies is used, examples of which are as follows: monkey kidney cells (COS cells), monkey kidney CVI cells transformed by SV40 (COS-7, ATCC CRL 165 1); human embryonic kidney cells (HEK-293, Graham et al., J. Gen Virol. 36:59 (1977)); baby hamster kidney cells (BHK, ATCC CCL 10); Chinese hamster ovary-cells (CHO, Urlaub and Chasin, Proc. Natl. Acad. Sci. (USA) 77:4216, (1980); mouse sertoli cells (TM4, Mather, Biol. Reprod. 23:243-251 (1980)); monkey kidney cells (CVI ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (hep G2, HB 8065); mouse mammary tumor (MMT 060562, ATCC CCL 51); TRI cells (Mather et al., Annals N.Y. Acad. Sci 383:44-68 (1982)); NIH/3T3 cells (ATCC CRL-1658); and mouse L cells (ATCC CCL-1). Additional cell lines will become apparent to those of ordinary skill in the art. A wide variety of cell lines are available from the American Type Culture Collection, 10801 University Boulevard, Manassas, Va. 20110-2209.

Methods of introducing nucleic acids into cells are well known in the art. Suitable methods include electroporation, particle gun technology, calcium phosphate precipitation, cationic lipid nucleic acid delivery, direct microinjection, and the like. The choice of method is generally dependent on the type of cell being transformed and the circumstances under which the transformation is taking place (i.e., *in vitro, ex vivo,* or *in vivo).* A general discussion of these methods can be found in Ausubel, et al., Short Protocols in Molecular Biology, 3d ed., Wiley & Sons, 1995. In some cases, lipofectamine and calcium mediated gene transfer technologies are used.

After the subject nucleic acids have been introduced into a cell, the cell is typically incubated, normally at 37°C, sometimes under selection, for a suitable time to allow for the expression of the antibody. In most cases, the antibody is typically secreted into the supernatant of the media in which the cell is growing in.

In mammalian host cells, a number of viral-based expression systems may be utilized to express a subject antibody. In cases where an adenovirus is used as an expression vector, the antibody coding sequence of interest may be ligated to an adenovirus transcription/translation control complex, e.g., the late promoter and tripartite leader sequence. This chimeric gene may then be inserted in the adenovirus genome by in vitro or in vivo recombination. Insertion in a non-essential region of the viral genome (e.g., region E1 or E3) will result in a recombinant virus that is viable and capable of expressing the antibody molecule in infected hosts. (e.g., see Logan & Shenk, Proc. Natl. Acad. Sci. USA 81:355-359 (1984)). The efficiency of expression may be enhanced by the inclusion of appropriate transcription enhancer elements, transcription terminators, etc. (see Bittner et al., Methods in Enzymol. 153:51-544 (1987)).

For long-term, high-yield production of recombinant antibodies, stable expression may be used. For example, cell lines, which stably express the antibody molecule, may be engineered. Rather than using expression vectors which contain viral origins of replication, host cells can be transformed with immunoglobulin expression cassettes and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into a chromosome and grow to form foci which in turn can be cloned and expanded into cell lines. Such engineered cell lines may be particularly useful in screening and evaluation of compounds that interact directly or indirectly with the antibody molecule.

Once an antibody molecule or fusion polypeptide of the invention has been produced, it may be purified by any method known in the art for purification of an immunoglobulin molecule, for example, by chromatography (e.g., ion exchange, affinity, particularly by affinity for the specific antigen after Protein A, and sizing column chromatography), centrifugation, differential solubility, or by any other standard technique for the purification of proteins. In many cases, antibodies are secreted from the cell into culture medium and harvested from the culture medium. For example, a nucleic acid sequence encoding a signal peptide may be included adjacent the coding region of the antibody or fragment. Such a signal peptide may be incorporated adjacent to the 5' end of the amino acid sequences set forth herein for the subject antibodies in order to facilitate production of the subject antibodies.

In one case, the antibodies comprising chicken framework regions according to certain cases of the present invention may be generated and/or optimized for required affinity or specificity using an *in vitro* system based on the DT40 chicken B cell lymphoma line. The DT40 chicken B cell lymphoma line has been used for antibody evolution *ex vivo* (Cumbers, S.J. et al. Nat Biotechnol 20: 1129-1134 (2002); Seo, H. et al. Nat Biotechnol 23:731-735 (2005).). DT40 cells command enormous potential V region sequence diversity, as they can access two distinct physiological pathways for diversification, gene conversion and somatic hypermutation, which create templated and nontemplated mutations, respectively (Maizels, N., Immunoglobulin gene diversification. Ann. Rev. Genet. 39:23-46 (2005)). However, the utility of DT40 cells for antibody evolution has been limited in practice because - as in other transformed B cell lines-diversification occurs at less than 1% the physiological rate. Diversification can be accelerated several-fold by disabling the homologous recombination pathway (Cumbers *et al., supra),* but cells thus engineered lose the ability to carry out efficient gene targeting. Diversification can also be accelerated by treatment of cells with the histone deacetylase inhibitor, trichostatin A (Seo *et al., supra),* but resulting mutations are exclusively templated, which limits potential diversity and may not produce antibodies of required affinity or specificity.

The DT40 cells used herein to generate antibodies are modified to accelerate the rate of immunoglobulin (Ig) gene diversification without sacrificing the capacity for further genetic modification or the potential for both gene conversion and somatic hypermutation to contribute to mutagenesis. This was accomplished by putting Ig gene diversification under control of the potent *E. coli* lactose operator/repressor regulatory network. Multimers consisting of approximately 100 polymerized repeats of the potent *E. coli* lactose operator (PolyLacO) were inserted upstream of the rearranged and expressed Igλ and IgH genes by homologous gene targeting. Regulatory factors fused to lactose repressor protein (LacI) can then be tethered to the LacO regulatory elements to regulate diversification, taking advantage of the high affinity (K_{D}=10⁻¹⁴ M) of lactose repressor for operator DNA. DT40 PolyLacO-λ_{R} cells, in which PolyLacO was integrated only at Igλ, exhibited a 5-fold increase in Ig gene diversification rate relative to the parental DT40 cells prior to any engineering (Cummings, W.J. et al. PLoS Biol 5, e246 (2007)). Diversification was further elevated in cells engineered to carry PolyLacO targeted to both the Igλ and the IgH genes ("DTLacO").

### Pharmaceutical Compositions

Also described herein are pharmaceutical compositions comprising the bioactive peptide-bearing antibodies and antigen-binding fragments thereof, as disclosed herein and a pharmaceutically acceptable carrier. In some cases, the disclosure provides compositions comprising bioactive peptide-bearing antibodies and antigen-binding fragments thereof capable of inhibiting complement activation. In some cases, the disclosure provides compositions comprising bioactive peptide-bearing antibodies and fragments thereof capable of inhibiting activation of the lectin complement pathway. In some cases, the disclosure provides compositions comprising SGMI peptide-bearing MASP-2 antibodies (e.g., SGMI-1 and/or SGMI-2) and fragments thereof capable of inhibiting activation of the lectin pathway.

In one case, the composition is formulated to specifically inhibit MASP-1 or MASP-2 activity. In one case, the composition is formulated to specifically inhibit MASP-1 activity. In one case, the composition is formulated to specifically inhibit MASP-2 activity. In one case, the composition is formulated to specifically inhibit MASP-1 and MASP-2 activity.

In one case, the disclosure provides a pharmaceutical composition comprising a bioactive peptide-bearing antibody that specifically binds to MASP-2 and inhibits MASP-2 dependent lectin pathway activation. In one case, the disclosure provides a pharmaceutical composition comprising a bioactive peptide-bearing antibody that specifically binds to MASP-1 and inhibits MASP-1 activity. In one case, the disclosure provides a pharmaceutical composition comprising a bioactive peptide-bearing bi-specific antibody that binds to MASP-1 and MASP-2 and inhibits MASP-1 and MASP-2 activity. In one case, the disclosure provides a pharmaceutical composition comprising a first bioactive peptide-bearing antibody that binds to MASP-1 and inhibits MASP-1 activity and a second bioactive peptide-bearing antibody that binds to MASP-2 and inhibits MASP-2 activity.

The carrier is non-toxic, biocompatible and is selected so as not to detrimentally affect the biological activity of the bioactive peptide-bearing antibody (and any other therapeutic agents combined therewith). Exemplary pharmaceutically acceptable carriers for polypeptides are described in U.S. Patent No. 5,211,657 to Yamada. The bioactive peptide-bearing antibodies and polypeptides may be formulated into preparations in solid, semi-solid, gel, liquid or gaseous forms such as tablets, capsules, powders, granules, ointments, solutions, depositories, inhalants and injections allowing for oral, parenteral or surgical administration. The disclosure also contemplates local administration of the compositions by coating medical devices and the like.

Suitable carriers for parenteral delivery via injectable, infusion or irrigation and topical delivery include distilled water, physiological phosphate-buffered saline, normal or lactated Ringer's solutions, dextrose solution, Hank's solution, or propanediol. In addition, sterile, fixed oils may be employed as a solvent or suspending medium. For this purpose, any biocompatible oil may be employed including synthetic mono- or diglycerides. In addition, fatty acids such as oleic acid find use in the preparation of injectables. The carrier and agent may be compounded as a liquid, suspension, polymerizable or non-polymerizable gel, paste or salve.

The carrier may also comprise a delivery vehicle to sustain (i.e., extend, delay or regulate) the delivery of the agent(s) or to enhance the delivery, uptake, stability or pharmacokinetics of the therapeutic agent(s). Such a delivery vehicle may include, by way of non-limiting example, microparticles, microspheres, nanospheres or nanoparticles composed of proteins, liposomes, carbohydrates, synthetic organic compounds, inorganic compounds, polymeric or copolymeric hydrogels and polymeric micelles. Suitable hydrogel and micelle delivery systems include the PEO:PHB:PEO copolymers and copolymer/cyclodextrin complexes disclosed in WO 2004/009664 A2 and the PEO and PEO/cyclodextrin complexes disclosed in U.S. Patent Application Publication No. 2002/0019369 A1. Such hydrogels may be injected locally at the site of intended action, or subcutaneously or intramuscularly to form a sustained release depot.

For intra-articular or intravenous delivery, the bioactive peptide-bearing antibodies or polypeptides may be carried in above-described liquid or gel carriers that are injectable, above-described sustained-release delivery vehicles that are injectable, or a hyaluronic acid or hyaluronic acid derivative.

For intrathecal (IT) or intracerebroventricular (ICV) delivery, appropriately sterile delivery systems (e.g., liquids; gels, suspensions, etc.) can be used to administer the present invention.

The compositions of the present invention may also include biocompatible excipients, such as dispersing or wetting agents, suspending agents, diluents, buffers, penetration enhancers, emulsifiers, binders, thickeners, flavoring agents (for oral administration).

To achieve high concentrations of the subject antibodies for local delivery, the antibodies may be formulated as a suspension of particulates or crystals in solution for subsequent injection, such as for intramuscular injection of a depot.

### Therapeutic Methods:

Also described herein are methods of inhibiting lectin pathway complement activation in a mammalian subject, such as a human subject, comprising administering a composition comprising a bioactive peptide-bearing antibody or bioactive peptide-bearing polypeptide fusion as disclosed herein to said human subject, wherein the bioactive peptide inhibits activation of the lectin complement pathway. As described herein, the bioactive peptides SGMI-1 and SGMI-2 block the lectin pathway of complement activation without affecting the classical pathway (Heja et al., 2012. Proc. Natl. Acad. Sci. 109:10498). As described in US Patent No. 7,919,094, US Patent No. 8,652,477, and co-pending U.S. Patent Application Serial No. 13/083,441 (published as US2011/0311549), U.S. Patent Application Serial No. 13/830,779 (published as US2013/0266559), U.S. Patent Application Serial No. 13/441,827 (published as US2012/0258095), U.S. Patent Application Serial No. 13/830,831 (published as US2013/0266560) and U.S. Patent Application Serial No. 13/464,334 (published as US2012/0282263), (each of which is assigned to Omeros Corporation, the assignee of the instant application), MASP-2-dependent lectin complement activation has been implicated as contributing to the pathogenesis of numerous acute and chronic disease states, including MASP-2-dependent complement-mediated vascular condition, an ischemia reperfusion injury, atherosclerosis, inflammatory gastrointestinal disorder, a pulmonary condition, an extracorporeal reperfusion procedure, a musculoskeletal condition, a renal condition, a skin condition, organ or tissue transplant, nervous system disorder or injury, a blood disorder, a urogenital condition, diabetes, chemotherapy or radiation therapy, malignancy, an endocrine disorder, a coagulation disorder, a thrombotic microangiopathy, or an ophthalmologic condition. Therefore, the lectin pathway inhibitory antibodies of the present invention may be used to treat the above-referenced diseases and conditions.

### Methods for inhibiting MASP-2-dependent lectin pathway complement activation and/or MASP-1-dependent lectin pathway complement activation

In one case, the disclosure provides a method for inhibiting MASP-2-dependent lectin complement activation and/or MASP-1-dependent lectin complement activation for treating, preventing, or reducing the severity of a lectin complement-mediated vascular condition, an ischemia reperfusion injury, atherosclerosis, inflammatory gastrointestinal disorder, a pulmonary condition, an extracorporeal reperfusion procedure, a musculoskeletal condition, a renal condition, a skin condition, organ or tissue transplant, nervous system disorder or injury, a blood disorder, a urogenital condition, diabetes, chemotherapy or radiation therapy, malignancy, an endocrine disorder, a coagulation disorder, a thrombotic microangiopathy, or an ophthalmologic condition, comprising administering a composition comprising a therapeutically effective amount of a bioactive peptide-bearing antibody (e.g., an SGMI-2 bearing antibody, such as an SGMI-2- MASP-2 antibody and/or an SGMI-1 bearing antibody, such as an SGMI-1 MASP-2 antibody, or an SGMI-1 and SGMI-2 bearing antibody) or antigen-binding fragment thereof, capable of inhibiting MASP-2-dependent lectin pathway activation and/or MASP-1-dependent lectin pathway activation to a subject in need thereof.

In some cases, the methods of this aspect of the disclosure are used to treat a subject suffering from a vascular condition, preferably a vascular condition selected from the group consisting of a cardiovascular condition, a cerebrovascular condition, a peripheral (e.g., musculoskeletal) vascular condition, a renovascular condition, a mesenteric/enteric vascular condition, revascularization to transplants and/or replants, vasculitis, Henoch-Schonlein purpura nephritis, systemic lupus erythematosus-associated vasculitis, vasculitis associated with rheumatoid arthritis, immune complex vasculitis, Takayasu's disease, dilated cardiomyopathy, diabetic angiopathy, Kawasaki's disease (arteritis), venous gas embolus (VGE), and restenosis following stent placement, rotational atherectomy and percutaneous transluminal coronary angioplasty (PTCA).

In some cases, the methods are used to treat a subject suffering from a condition associated with an ischemia-reperfusion injury, preferably an ischemia-reperfusion injury associated with aortic aneurysm repair, cardiopulmonary bypass, vascular reanastomosis in connection with organ transplants and/or extremity/digit replantation, stroke, myocardial infarction, and hemodynamic resuscitation following shock and/or surgical procedures.

In some cases, the methods are used for treating and/or preventing atherosclerosis in a subject in need thereof.

In some cases, the methods are used to treat a subject suffering from a condition associated with an inflammatory gastrointestinal disorder, preferably an inflammatory gastrointestinal disorder selected from the group consisting of pancreatitis, Crohn's disease, ulcerative colitis, irritable bowel syndrome and diverticulitis.

In some cases, the methods are used to treat a subject suffering from a pulmonary condition, preferably a pulmonary condition selected from the group consisting of acute respiratory distress syndrome, transfusion-related acute lung injury, ischemia/reperfusion acute lung injury, chronic obstructive pulmonary disease, asthma, Wegener's granulomatosis, antiglomerular basement membrane disease (Goodpasture's disease), meconium aspiration syndrome, bronchiolitis obliterans syndrome, idiopathic pulmonary fibrosis, acute lung injury secondary to burn, non-cardiogenic pulmonary edema, transfusion-related respiratory depression and emphysema.

In some cases, the methods are used for inhibiting lectin complement activation in a subject that has undergone, is undergoing, or will undergo an extracorporeal reperfusion procedure, preferably an extracorporeal reperfusion procedure selected from the group consisting of hemodialysis, plasmapheresis, leukopheresis, extracorporeal membrane oxygenator (ECMO), heparin-induced extracorporeal membrane oxygenation LDL precipitation (HELP) and cardiopulmonary bypass (CPB).

In some cases, the methods are used to treat a subject suffering from a musculoskeletal condition selected from the group consisting of osteoarthritis, rheumatoid arthritis, juvenile rheumatoid arthritis, gout, neuropathic arthropathy, psoriatic arthritis, spondyloarthropathy, crystalline arthropathy and systemic lupus erythematosus (SLE).

In some cases, the methods are used to treat a subject suffering from a renal condition selected from the group consisting of mesangioproliferative glomerulonephritis, membranous glomerulonephritis, membranoproliferative glomerulonephritis (mesangiocapillary glomerulonephritis), acute postinfectious glomerulonephritis (poststreptococcal glomerulonephritis), cryoglobulinemic glomerulonephritis, lupus nephritis, Henoch-Schonlein purpura nephritis and IgA nephropathy.

In some cases, the methods are used to treat a subject suffering from a skin condition selected from the group consisting of psoriasis, autoimmune bullous dermatoses, eosinophilic spongiosis, bullous pemphigoid, epidermolysis bullosa acquisita, herpes gestationis, thermal burn injury and chemical burn injury.

In some cases, the methods are used to treat a subject that has undergone, is undergoing, or will undergo an organ or tissue transplant procedure, preferably a transplant procedure selected from the group consisting of organ allotransplantation, organ xenotransplantation organ and tissue graft.

In some cases, the methods are used to treat a subject suffering from a nervous system disorder or injury selected from the group consisting of multiple sclerosis, myasthenia gravis, Huntington's disease, amyotrophic lateral sclerosis, Guillain Barre syndrome, reperfusion following stroke, degenerative discs, cerebral trauma, Parkinson's disease, Alzheimer's disease, Miller-Fisher syndrome, cerebral trauma and/or hemorrhage, demyellination and meningitis.

In some cases, the methods are used to treat a subject suffering from a blood disorder selected from the group consisting of sepsis, severe sepsis, septic shock, acute respiratory distress syndrome resulting from sepsis, systemic inflammatory response syndrome, hemorrhagic shock, hemolytic anemia, autoimmune thrombotic thrombocytopenic purpura and hemolytic uremic syndrome.

In some cases, the methods are used to treat a subject suffering from a urogenital condition selected from the group consisting of painful bladder disease, sensory bladder disease, chronic abacterial cystitis, interstitial cystitis, infertility, placental dysfunction and miscarriage and pre-eclampsia.

In some cases, the methods are used to treat a subject suffering from a condition associated with nonobese diabetes (Type-1 diabetes or Insulin-dependent diabetes mellitus) and/or complications associated with Type-1 or Type-2 (adult onset) diabetes, preferably a complication associated with Type 1 or Type 2 diabetes that is selected from the group consisting of angiopathy, neuropathy and retinopathy.

In some cases, the methods are used to inhibit the lectin complement pathway in a subject that has undergone, is undergoing, or will undergo chemotherapeutic treatment and/or radiation therapy. In some cases, the methods of this aspect of the invention are used to treat a subject suffering from a malignancy.

In some cases, the methods are used to treat a subject suffering from an endocrine disorder selected from the group consisting of Hashimoto's thyroiditis, stress, anxiety and hormonal disorders involving regulated release of prolactin, growth or other insulin-like growth factor and adrenocorticotropin from the pituitary.

In some cases, the methods are used to treat a subject suffering from a lectin complement mediated ophthalmologic condition, preferably age-related macular degeneration.

In some cases, the methods are used for treating, preventing, or reducing the severity of disseminated intravascular coagulation in a subject in need thereof, such as in a subject suffering from a disease or condition selected from the group consisting of sepsis, trauma, malignancy, transplant rejection, transfusion reaction, obstetric complication, vascular aneurysm, hepatic failure, heat stroke, burn, radiation exposure and severe toxic reaction.

In some cases, the methods are used to inhibit the lectin complement pathway in a subject suffering from paroxysmal nocturnal hemoglobinuria.

In some cases, the methods are used to inhibit the lectin pathway in a subject suffering from a thrombotic microangiopathy (TMA). In some cases, the methods are used for inhibiting the lectin pathway in a subject suffering from or at risk for developing atypical hemolytic uremic syndrome (aHUS). In some cases, the methods are used for inhibiting the lectin pathway in a subject suffering from or at risk for developing hemolytic uremic syndrome (HUS). In some cases, the methods are used for inhibiting the lectin pathway in a subject suffering from thrombotic thrombocytopenic purpura (TTP), or exhibiting symptoms consistent with a diagnosis of TTP.

In some cases, the methods are used for inhibiting the lectin pathway in a subject suffering from cryoglobulinemia.

In some cases, the methods are used for inhibiting the lectin pathway in a subject suffering from cold aggultinin disease.

In some cases, the methods are used for inhibiting the lectin pathway in a subject suffering from glaucoma.

In some cases, the methods are used for inhibiting the lectin pathway in a subject at risk for developing or suffering from acute radiation syndrome.

In some cases, the methods are used for inhibiting the lectin pathway in a subject suffering from, or at risk for developing a disease or disorder selected from the group consisting of dense deposit disease, pauci-immune necrotizing crescentic glomerulonephritis, traumatic brain injury, aspiration pneumonia, endophthalmitis, neuromyelitis optica and Behcet's disease.

### Methods for inhibiting lectin pathway complement activation and inhibiting

### MASP-1-dependent alternative pathway complement activation

Also described herein is a method for inhibiting lectin pathway complement activation and also inhibiting MASP-1-dependent alternative pathway complement activation for treating, preventing, or reducing the severity of Paroxysmal nocturnal hemoglobinuria (PNH), age-related macular degeneration, ischemia-reperfusion injury, arthritis, disseminated intravascular coagulation, thrombotic microangiopathy (including HUS, aHUS and TTP), asthma, dense deposit disease, pauci-immune necrotizing crescentic glomerulonephritis, traumatic brain injury, aspiration pneumonia, endophthalmitis, neuromyelitis optica and Behcet's disease comprising administering a composition comprising a therapeutically effective amount of a bioactive peptide-bearing antibody or bioactive peptide-bearing polypeptide fusion as disclosed herein, wherein the composition inhibits activation of the lectin complement pathway and inhibits MASP-1-dependent activation of the alternative pathway (e.g., an SGMI-1 bearing antibody, or an SGMI-1-MASP-2 antibody or an SGMI-1 and SGMI-2 bearing antibody) or antigen-binding fragment thereof, capable of inhibiting lectin pathway activation and MASP-1-dependent alternative pathway activation to a subject in need thereof.

Recent discoveries have linked MASP-1 to alternative pathway activation. MASP-1 can convert the alternative pathway activation enzyme factor D from its zymogen form into its enzymatically active form (see Takahashi et al., J Exp Med 207:29-37 (2010), Iwaki et al., J. Immunol 187:3751-58 (2011)). Furthermore, MASP-1 activates the zymogen form of MASP-3 (Megyeri et al., J. Biol. Chem. 288:8922-8934 (2013); Degn et al. J. Immunol. 189(8): 3957-69 (2012)), which itself can activate zymogen factor D as well as cleave factor B, another essential component of the alternative pathway, to its active form (Iwaki et al., J. Immunol. 187:3751-58 (2011)).

As described in co-pending U.S. Patent Application Serial No. 13/857,391 (published as US2013/0273053) and U.S. Patent Application Serial No. 13/921,139 (published as US2013/0344073) (each of which is assigned to Omeros Corporation, the assignee of the instant application), MASP-2 dependent lectin pathway activation and MASP-1-dependent alternative pathway complement activation have been implicated as contributing to the pathogenesis of numerous acute and chronic disease states, including Paroxysmal nocturnal hemoglobinuria, age-related macular degeneration, ischemia-reperfusion injury, arthritis, disseminated intravascular coagulation, thrombotic microangiopathy (including HUS, aHUS and TTP), asthma, dense deposit disease, pauci-immune necrotizing crescentic glomerulonephritis, traumatic brain injury, aspiration pneumonia, endophthalmitis, neuromyelitis optica and Behcet's disease.

Accordingly, in one case, disclosed herein is a method of inhibiting lectin pathway activation and MASP-1-dependent alternative pathway activation by administering a therapeutically effective amount of a composition comprising at least one of (i) a bioactive peptide bearing antibody capable of inhibiting MASP-1 activity (e.g., an antibody comprising SGMI-1), (ii) an SGMI-1-MASP-2 antibody, (iii) a SGMI-1 and SGMI-2 bearing antibody, or (iv) a first SGMI-1-bearing antibody and a second SGMI-2-bearing antibody, for the treatment of subject suffering from, or at risk for developing a disease or disorder selected from the group consisting of: Paroxysmal nocturnal hemoglobinuria, age-related macular degeneration, ischemia-reperfusion injury, arthritis, disseminated intravascular coagulation, thrombotic microangiopathy (including HUS, aHUS and TTP), asthma, dense deposit disease, pauci-immune necrotizing crescentic glomerulonephritis, traumatic brain injury, aspiration pneumonia, endophthalmitis, neuromyelitis optica and Behcet's disease.

### Delivery Methods

In accordance with various cases of the present disclosure, the composition is formulated for systemic delivery, such as, by intra-arterial, intravenous, intracranial, intramuscular, inhalational, nasal or subcutaneous administration.

As used herein, the terms "systemic delivery" and "systemic administration" are intended to include but are not limited to oral and parenteral routes including intramuscular (IM), subcutaneous, intravenous (IV), intra-arterial, inhalational, sublingual, buccal, topical, transdermal, nasal, rectal, vaginal and other routes of administration that effectively result in dispersal of the delivered antibody to a single or multiple sites of intended therapeutic action. Preferred routes of systemic delivery for the present compositions include intravenous, intramuscular, subcutaneous, and inhalational. It will be appreciated that the exact systemic administration route for selected agents utilized in particular compositions of the present invention will be determined in part to account for the agent's susceptibility to metabolic transformation pathways associated with a given route of administration.

The bioactive peptide-bearing antibodies and polypeptides can be delivered into a subject in need thereof by any suitable means. Methods of delivery include administration by oral, pulmonary, parenteral (e.g., intramuscular, intraperitoneal, intravenous (IV), or subcutaneous injection), inhalation (such as via a fine powder formulation), transdermal, nasal, vaginal, rectal, or sublingual routes of administration, and can be formulated in dosage forms appropriate for each route of administration.

The compositions of the present invention may be systemically administered on a periodic basis at intervals determined to maintain a desired level of therapeutic effect. For example, compositions may be administered, such as by subcutaneous injection, every two to four weeks or at less frequent intervals. The dosage regimen will be determined by the physician considering various factors that may influence the action of the combination of agents. These factors will include the extent of progress of the condition being treated, the patient's age, sex and weight, and other clinical factors. The dosage for each individual agent will vary as a function of the particular antibody that is included in the composition, as well as the presence and nature of any drug delivery vehicle (e.g., a sustained release delivery vehicle). In addition, the dosage quantity may be adjusted to account for variation in the frequency of administration and the pharmacokinetic behavior of the delivered agent(s).

Therapeutic efficacy of MASP-2 and MASP-1 inhibitory compositions and methods of the present disclosure in a given subject, and appropriate dosages, can be determined in accordance with complement assays well known to those of skill in the art. Complement generates numerous specific products. During the last decade, sensitive and specific assays have been developed and are available commercially for most of these activation products, including the small activation fragments C3a, C4a, and C5a and the large activation fragments iC3b, C4d, Bb, and sC5b-9. Most of these assays utilize antibodies that react with new antigens (neoantigens) exposed on the fragment, but not on the native proteins from which they are formed, making these assays very simple and specific. Most rely on ELISA technology, although radioimmunoassay is still sometimes used for C3a and C5a. These latter assays measure both the unprocessed fragments and their 'desArg' fragments, which are the major forms found in the circulation. Unprocessed fragments and C5a_{desArg} are rapidly cleared by binding to cell surface receptors and are hence present in very low concentrations, whereas C3a_{desArg} does not bind to cells and accumulates in plasma. Measurement of C3a provides a sensitive, pathway-independent indicator of complement activation. Alternative pathway activation can be assessed by measuring the Bb fragment. Detection of the fluid-phase product of membrane attack pathway activation, sC5b-9, provides evidence that complement is being activated to completion. Because both the lectin and classical pathways generate the same activation products, C4a and C4d, measurement of these two fragments does not provide any information about which of these two pathways has generated the activation products.

The inhibition of lectin-dependent complement activation is characterized by at least one of the following changes in a component of the complement system that occurs as a result of administration of an anti-MASP-2 antibody in accordance with the present invention: the inhibition of the generation or production of MASP-2-dependent complement activation system products C4b, C3a, C5a and/or C5b-9 (MAC), the reduction of C4 cleavage and C4b deposition, or the reduction of C3 cleavage and C3b deposition.

### Articles of Manufacture

Also described herein is an article of manufacture containing a bioactive peptide-bearing antibody, or antigen binding fragment thereof, or polypeptide as described herein (e.g., an SGMI-bearing MASP-2 antibody) in a unit dosage form suitable for therapeutic administration to a human subject, such as, for example, a unit dosage in the range of 1mg to 5000mg, such as from 1 mg to 2000mg, such as from 1mg to 1000 mg, such as 5mg, 10mg, 50mg, 100mg, 200mg, 500mg, or 1000mg. In some cases, the article of manufacture comprises a container and a label or package insert on or associated with the container. Suitable containers include, for example, bottles, vials, syringes, etc. The containers may be formed from a variety of materials such as glass or plastic. The container holds a composition which is effective for treating the condition and may have a sterile access port (for example, the container may be an intravenous solution bag or a vial having a stopper pierceable by a hypodermic injection needle). At least one active agent in the composition is the bioactive peptide-bearing antibody or antigen binding fragment thereof or polypeptide of the invention (e.g., an SGMI-peptide bearing MASP-2 antibody or antigen binding fragment thereof). The label or package insert indicates that the composition is used for treating the particular condition. The label or package insert will further comprise instructions for administering the antibody composition to the patient. Articles of manufacture and kits comprising combinatorial therapies described herein are also contemplated.

The following examples merely illustrate the best mode now contemplated for practicing the present disclosure.

### EXAMPLE 1

### Overview of the strategy for generating inhibitory MASP polypeptides by engrafting bioactive peptides into, or fusing bioactive peptides onto antibodies, or fragments thereof

Rationale: The generation of specific inhibitors of MASP-1 and MASP-2, termed SGMI-1 and SGMI-2, respectively, is described in Heja et al., J Biol Chem 287:20290 (2012) and Heja et al., PNAS 109:10498 (2012), each of which is hereby incorporated herein by reference. SGMI-1 and SGMI-2 are each 36 amino acid peptides which were selected from a phage library of variants of the *Schistocerca gregaria* protease inhibitor 2 in which six of the eight positions of the protease binding loop were fully randomized. Subsequent *in vitro* evolution yielded mono-specific inhibitors with single digit nM K_{I} values (Heja et al., J. Biol. Chem. 287:20290, 2012). Structural studies revealed that the optimized protease binding loop forms the primary binding site that defines the specificity of the two inhibitors. The amino acid sequences of the extended secondary and internal binding regions are common to the two inhibitors and contribute to the contact interface (Heja et al., 2012. J. Biol. Chem. 287:20290). Mechanistically, both SGMI-1 and SGMI-2 block the lectin pathway of complement activation without affecting the classical pathway (Heja et al., 2012. Proc. Natl. Acad. Sci. 109:10498).

The amino acid sequences of the SGMI-1 and SGMI-2 inhibitors are set forth below:

| | |
|---|---|
| SGMI-1-full-length: | LEVTCEPGTTFKDKCNTCRCGSDGKSAFCTRKLCYQ (SEQ ID NO:6) |
| SGMI-1-medium: | TCEPGTTFKDKCNTCRCGSDGKSAFCTRKLCYQ (SEQ ID NO:7) |
| SGMI-1-short: | TCRCGSDGKSAFCTRKLCYQ (SEQ ID NO:8) |
| SGMI-2-full-length: | LEVTCEPGTTFKDKCNTCRCGSDGKSAVCTKLWCNQ (SEQ ID NO:9) |
| SGMI-2-medium: | TCEPGTTFKDKCNTCRCGSDGKSAVCTKLWCNQ (SEQ ID NO: 10) |
| SGMI-2-short: | TCRCGSDGKSAVCTKLWCNQ (SEQ ID NO:11) |

The above SGMI sequences share a core SGMI sequence (underlined), which is set forth below as SEQ ID NO:5:
X₁CTX₂X₃X₄CX₅Q (SEQ ID NO:5)
wherein:
X₁ is F or V,
X₂ is R or K,
X₃ is K or L,
X₄ is L or W, and
X₅ is Y or N

The bioactive peptides (e.g., SGMI peptides derived from SGMI-1 (set forth as SEQ ID NOs:6-8) and SGMI-2 (set forth as SEQ ID NO:9-11)) are highly specific inhibitors of MASP-1 and MASP-2, respectively. However, as peptides they have limited potential for use in biological studies and therapeutic applications. To address these limitations, as described herein, the inventors have engrafted these bioactive peptide amino acid sequences (i.e., amino acid sequences encoding the bioactive peptides) into, or fused them onto, various scaffolds: (1) fused onto the amino terminus of human IgG1 Fc region to create an Fc-fusion protein, as described in Example 2; (2) engrafted into various complementarity-determining regions (CDR) of a non-specific chimeric chicken (variable regions) - human (IgG1 and Igλ constant regions) antibody, as described in Example 3; (3) fused onto the amino or carboxy termini of the heavy and/or light chains of a non-specific chimeric chicken (variable regions)- human (IgG1 and Igλ constant regions) antibody, as described in Example 4, and (4) fused onto the amino or carboxy termini of the heavy and/or the light chains of a human antibody, such as a human MASP-2 antibody, as described in Examples 7 and 8.

As described herein, introduction of a bioactive peptide sequence into or onto an antibody scaffold results in a product with at least the same or greater bioactivity as compared to the isolated bioactive peptide and with improved therapeutic properties, such as a longer half-life and antibody effector functions.

### EXAMPLE 2

This Example describes the generation of recombinant SGMI-Fc fusion proteins and demonstrates that these fusion proteins are able to inhibit the lectin pathway.

Methods: To express the SGMI-IgG1 Fc fusion proteins, polynucleotides encoding the SGMI-1 (SEQ ID NO:6) and SGMI-2 (SEQ ID NO:9) peptides were synthesized (DNA 2.0) and inserted into the expression vector pFUSE-hIgG1-Fc2 (InvivoGen) between nucleotide sequences encoding the IL-2 signal sequence and the human IgG1 Fc region (SEQ ID NO:12). In some cases, an optional flexible polypeptide linker (e.g., SEQ ID NO:13 or SEQ ID NO:14) was included between the SGMI peptide and the IgG1 Fc region.

### Exemplary Flexible Polypeptide Linker Sequences:

GTGGGSGSSSRS (SEQ ID NO:13)
GTGGGSGSSS (SEQ ID NO:14)

It is noted that in another case, the disclosure encompasses an alternative version of the SGMI-IgG1 Fc fusion proteins containing the IgG1 Fc region fused to the amino terminus of the SGMI peptides. It is further noted that in further cases, the disclosure encompasses alternative versions of the SGMI-IgG1 Fc fusion proteins comprising a bioactive peptide amino acid sequence comprising the core SGMI sequence (SEQ ID NO:5), and having a length of from at least 9 amino acid residues to 36 amino acid residues, including various truncated versions of SGMI-1 or SGMI-2 bioactive peptides (e.g., SGMI peptides comprising the core sequence of SEQ ID NO:5, such as any of SEQ ID NO:6 to SEQ ID NO:11).

The resulting constructs are described as follows:
A polynucleotide encoding the polypeptide fusion comprising the human IL-2 signal sequence, SGMI-1, linker and human IgG1-Fc (pFUSE-SGMI-1Fc), is set forth as SEQ ID NO:15, which encodes the mature polypeptide fusion comprising SGMI-1 (underlined), linker region (italicized) and human IgG1-Fc (together referred to as "SGMI-1Fc"), which is set forth as SEQ ID NO: 16.

### SEQ ID NO:16

LEVTCEPGTTFKDKCNTCRCGSDGKSAFCTRKLCYQGTGGGSGSSSRSDK
THTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESN
GQPENNYKTTPPVLDSDGSFFLYSKLTVDKSRWQQGNVFSCSVMHEALHNHYT
QKSLSLSPGK

A polynucleotide encoding the polypeptide fusion comprising the human IL-2 signal sequence, SGMI-2, linker and human IgG1-Fc (pFUSE-SGMI-2Fc), is set forth as SEQ ID NO:17, which encodes the mature polypeptide fusion comprising SGMI-2 (underlined), linker region (italicized) and human IgG1-Fc (together referred to as "SGMI-2Fc"), which is set forth as SEQ ID NO: 18:

### SEQ ID NO:18

LEVTCEPGTTFKDKCNTCRCGSDGKSAVCTKLWCNQGTGGGSGSSSRSDK
THTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESN
GQPENNYKTTPPVLDSDGSFFLYSKLTVDKSRWQQGNVFSCSVMHEALHNHYT
QKSLSLSPGK

### Production of Recombinant proteins:

Freestyle 293-F or Expi293F cells (Invitrogen) were transiently transfected according to the supplier's protocol with one of the two expression plasmids (pFUSE-SGMI-1Fc (SEQ ID NO: 15) and pFUSE-SGMI-2Fc (SEQ ID NO: 17). After four days of incubation at 37°C, the culture media were harvested. The Fc-fusion proteins were purified by Protein A affinity chromatography.

### Assays measuring activation of the lectin pathway.

The Wieslab^{®} Complement System Screen (Euro Diagnostic, Malmö, Sweden), MBL assay measures C5b-C9 deposition in conditions that isolated the lectin pathway. The assay was carried out according to the manufacturer's instructions with the Fc fusion proteins being tested at final concentrations of 400 nM.

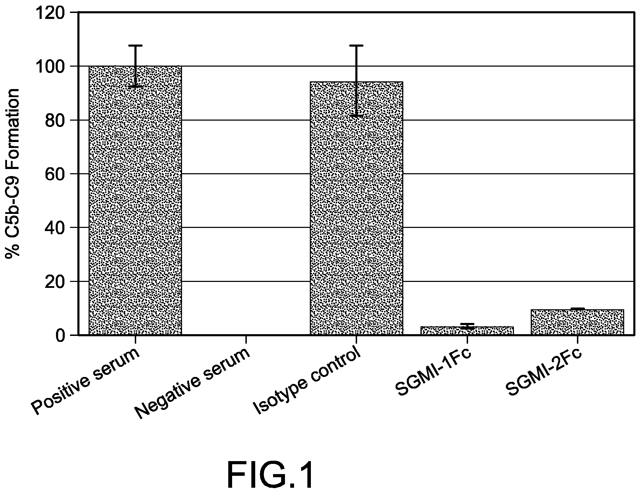

FIGURE 1 is a bar graph showing the inhibitory activity of the SGMI-1Fc (SEQ ID NO: 16) or SGMI-2Fc (SEQ ID NO:18) fusion proteins in comparison to the positive and negative sera provided with the assay kit, as well as an isotype control antibody. As shown in FIGURE 1, both SGMI-1Fc and SGMI-2Fc inhibit the activation of the lectin pathway, whereas the isotype control antibody does not.

The SGMI-1Fc and SGMI-2Fc fusion proteins were also tested for the ability to inhibit deposition of C3b from 1% serum on a mannan-coated 96-well plate, which is another measure of lectin pathway activity. SGMI-1Fc and SGMI-2Fc were pre-incubated with 1% normal human serum for one hour on ice before addition to wells coated with mannan (2 µg/well). C3b deposition was measured by ELISA as described in Schwaeble et al. PNAS 108:7523, 2011.

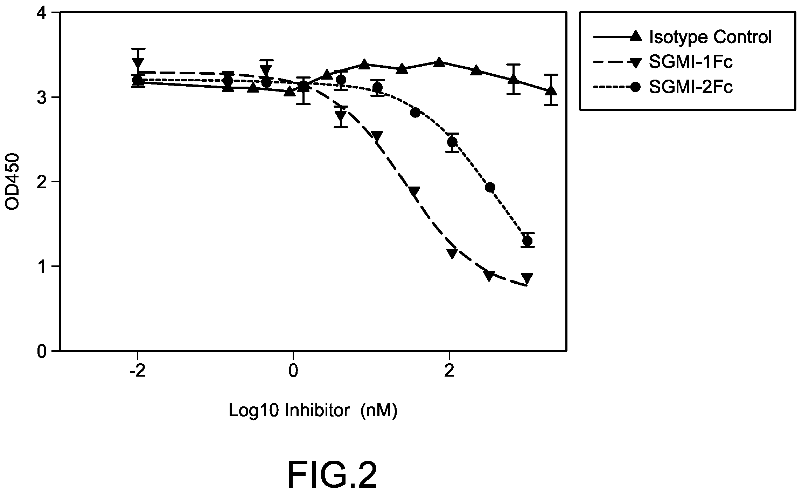

FIGURE 2 graphically illustrates the level of C3b deposition for 1% normal human serum plus isotype control, SGMI-1Fc or SGMI-2Fc over a concentration range of 0.15 to 1000 nM, demonstrating that both SGMI-1Fc and SGMI-2Fc inhibited C3b deposition from normal serum in mannan-coated ELISA wells, with IC50 values of approximately 27nM and 300nM, respectively.

These results demonstrate that the MASP-1 and MASP-2 inhibitory functions of the SGMI peptides are retained in the SGMI-1Fc and SGMI-2Fc fusion proteins.

### EXAMPLE 3

This Example describes the generation of chimeric chicken (V region)/human constant region) antibodies comprising a bioactive peptide amino acid sequence (e.g., SGMI-1 or SGMI-2) engrafted into at least one CDR region of a heavy chain variable region and/or at least one CDR region of a light chain variable region (e.g., CDR-H3 and/or CDR-L1).

### Background/Rationale:

A modified DT40 cell line, DTLacO, that permits reversible induction of diversification of a particular polypeptide, is described in WO2009029315 and US2010093033, each of which is hereby incorporated herein by reference. DT40 is a chicken B cell line that is known to constitutively mutate its heavy and light chain immunoglobulin (Ig) genes in culture. Like other B cells, this constitutive mutagenesis targets mutations to the V region of Ig genes, and thus, the CDRs of the expressed antibody molecules. Constitutive mutagenesis in DT40 cells takes place by gene conversion using as donor sequences an array of non-functional V gene segments (pseudo-V genes; ψV) situated upstream of each functional V region. Deletion of the ψV region was previously shown to cause a switch in the mechanism of diversification from gene conversion to somatic hypermutation, the mechanism commonly observed in human B cells. The DT40 chicken B cell lymphoma line has been shown to be a promising starting point for antibody evolution *ex vivo* (Cumbers, S.J. et al. Nat Biotechnol 20, 1129-1134 (2002); Seo, H. et al. Nat Biotechnol 23, 731-735 (2005)). DT40 cells proliferate robustly in culture, with an 8-10 hour doubling time (compared to 20-24 hr for human B cell lines), and they support very efficient homologous gene targeting (Buerstedde, J.M. et al. Embo J 9, 921-927 (1990)). DT40 cells command enormous potential V region sequence diversity given that they can access two distinct physiological pathways for diversification, gene conversion and somatic hypermutation, which create templated and nontemplated mutations, respectively (Maizels, N. Annu Rev Genet 39, 23-46 (2005)). Diversified heavy and light chain immunoglobulins (Igs) are expressed in the form of a cell-surface displayed IgM. Surface IgM has a bivalent form, structurally similar to an IgG molecule. Cells that display IgM with specificity for a particular antigen can be isolated by binding either immobilized soluble or membrane displayed versions of the antigen. However, utility of DT40 cells for antibody evolution has been limited in practice because - as in other transformed B cell lines-diversification occurs at less than 1% the physiological rate.

In the system used in this example, as described in WO2009029315 and US2010093033, the DT40 cells were engineered to accelerate the rate of Ig gene diversification without sacrificing the capacity for further genetic modification or the potential for both gene conversion and somatic hypermutation to contribute to mutagenesis. Two key modifications to DT40 were made to increase the rate of diversification and, consequently, the complexity of binding specificities in the library of cells (Yabuki et al., PLoS One 7:e36032, 2012). First, Ig gene diversification was put under the control of the potent *E. coli* lactose operator/repressor regulatory network. Multimers consisting of approximately 100 polymerized repeats of the potent *E. coli* lactose operator (PolyLacO) were inserted upstream of the rearranged and expressed Igλ, and IgH genes by homologous gene targeting. Regulatory factors fused to lactose repressor protein (LacI) can then be tethered to the LacO regulatory elements to regulate diversification, taking advantage of the high affinity (k_{D}=10⁻¹⁴ M) of lactose repressor for operator DNA. DT40 PolyLacO-λ_{R} cells, in which PolyLacO was integrated only at Igλ, exhibited a 5-fold increase in Ig gene diversification rate relative to the parental DT40 cells prior to any engineering (Cummings, W.J. et al. PLoS Biol 5, e246 (2007)). Diversification was further elevated in cells engineered to carry PolyLacO targeted to both the Igλ and the IgH genes ("DTLacO"). DTLacO cells were demonstrated to have diversification rates 2.5- to 9.2-fold elevated relative to the 2.8% characteristic of the parental DT40 PolyLacO-λ_{R} LacI-HP1 line. Thus, targeting PolyLacO elements to both the heavy and light chain genes accelerated diversification 21.7-fold relative to the DT40 parental cell line. Tethering regulatory factors to the Ig loci not only alters the frequency of mutagenesis, but also can change the pathway of mutagenesis creating a larger collection of unique sequence changes (Cummings et al. 2007; Cummings et al. 2008). Second, a diverse collection of sequence starting points for the tethered factor-accelerated Ig gene diversification was generated. These diverse sequence starting points were added to DTLacO by targeting rearranged Ig heavy-chain variable regions, isolated from a two month old chick, to the heavy chain locus. The addition of these heavy chain variable regions created a repertoire of 10⁷ new starting points for antibody diversification. Building these new starting points into the DTLacO cell line permits the identification of clones that bind a particular target, and then enable rapid affinity maturation by the tethered factors. Following affinity maturation, a full-length, recombinant chimeric IgG is made by cloning the matured, rearranged heavy- and light-chain variable sequences (VH and Vλ consisting of chicken framework regions and the CDRs) into expression vectors containing human IgG1 and lambda constant regions. These recombinant mAbs are suitable for *in vitro* and *in vivo* applications, and they serve as the starting point for humanization.

Through the use of the DTLacO system, the inventors have observed large inserts of more than 25 amino acids in CDR-H3 of the chicken heavy (VH) and CDR-L1 of the chicken light (VL) chain variable regions. In contrast, the average CDR-H3 size for mice and humans is much smaller (average size of 9 amino acids and 12 amino acids, respectively). Given the potential of these chicken CDRs to accommodate large blocks of sequence, the inventors tested the capacity of the CDRs to present the bioactive peptides SGMI-1 and SGMI-2 in an active conformation. The value of this strategy is several-fold: (1) the *in vivo* stability of an antibody is conferred to the SGMI-inhibitors, an important benefit for therapeutic applications; (2) integration of the VH and VL genes carrying a bioactive peptide, (such as the SGMI-1 or -2 sequence) into the DTLacO cell line provides the means for *ex vivo* mutagenesis and selection of V regions with greater affinity and potency; (3) engrafting a first bioactive peptide (e.g. SGMI-1) into one of the long CDRs and engrafting a second bioactive peptide (e.g. SGMI-2) into another of the long CDRs of an antibody, will create a bi-specific antibody that has two functional activities (e.g., inhibits MASP-1 and MASP-2). While this example describes the invention in the context of engrafting SGMI sequences into the CDR-H3 and/or CDR-L1 of the chicken variable regions and retaining inhibitory activity, it will be understood by one of skill in the art that results here establish a paradigm for the display and delivery of other bio-active peptides within CDRs of the variable light and/or heavy chain of antibodies comprising chicken variable regions.

### Methods:

To generate the chimeric chicken-human antibodies bearing bioactive peptides (SGMI-1 or SGMI-2) within CDR-H3 and/or CDR-L1, polynucleotides encoding the SGMI-1 and SGMI-2 peptides were inserted by In-Fusion cloning (Clontech primers shown in Table 3) into the pcDNA3 (Invitrogen)-based expression vectors of chicken-human chimeric heavy- and light-chain antibodies, described in WO2009029315 and US2010093033, incorporated herein by reference.

**TABLE 3: PCR primers used for cloning the SGMI-1 and SGMI-2 polynucleotides into chicken V-regions, resulting in SGMI-1L-IgG1 and SGMI-2L-Igλ chimeric antibodies.**

| **Primer** | **Sequence** |
|---|---|
| SGMI-1 Forward | CTACTGCGCCAAACTCGAGGTGACATGTGA (SEQ ID NO:19) |
| SGMI-1 Reverse | CGTGGCCCCATGCCTGGTAGCACAATTTCC (SEQ ID NO:20) |
| SGMI-2 Forward | CGGGGGTGGCAGC TTGGAAGTGACGTGTGA (SEQ ID NO:21) |
| SGMI-2 Reverse | AGCCATAATAGTA CTGGTTACACCAGAGCT (SEQ ID NO:22) |

### 1. SGMI-1 and SGMI-2 engrafted into the CDR-H3 of a parental chicken heavy chain variable region.

The DT40 chicken heavy chain variable region was chosen as the starting parental clone for use as a scaffold into which SGMI-1 or SGMI-2 peptide sequences were engrafted into the CDR-H3 region, as shown in FIGURES 3 and 4.

FIGURE 3 illustrates an exemplary parental (DTLacO) variable heavy chain polypeptide sequence compared to a modified version of the variable heavy chain polypeptide sequence comprising a bioactive peptide amino acid sequence engrafted within CDR-H3. As shown in FIGURE 3, the chicken heavy chain variable region contains three CDRs (CDR-H1, CDR-H2 and CDR-H3), flanked by four framework regions (FR-1, FR-2, FR-3 and FR-4). The inventors have surprisingly discovered that by engrafting a bioactive peptide (e.g. SGMI-1 or SGMI-2) into a CDR of the heavy chain variable region of the DT40 parental chicken antibody (which provides the antibody scaffold), the biological activity of the bioactive peptide was conferred to the parental antibody comprising the engrafted bioactive peptide sequence.

The parental chicken antibody provides the framework regions (FR1, FR2, FR3 and FR4) of the heavy and light chains, which are conserved between various clones. Any parental chicken antibody clone may be selected for use as a scaffold. In some cases, the parental chicken antibody clone may be selected based on desirable properties, such as stability.

Exemplary parental chicken heavy chain variable regions are provided below. As shown in FIGURE 4, although the native CDR regions vary between parental clones, the Framework regions between the CDRs are conserved in chicken, accordingly, a consensus FR-1, FR-2, FR-3 and FR-4 sequence derived from an alignment of several different parental chicken heavy chain regions is also provided below. As further shown in FIGURE 4, in FR-3 there is a conserved cysteine (C) residue at the third position N-terminal to CDR-H3 in the parental clones (corresponding to the cysteine at position 31 in SEQ ID NO:26), which is retained in FR-3 in the constructs containing an engrafted bioactive peptide in CDR-H3. As further shown in FIGURE 4, in FR-4 there is a conserved tryptophan (W) residue at the position immediately adjacent to CDR-H3 in the parental clones (corresponding to the tryptophan at position 1 in SEQ ID NO:28), which is retained in FR-4 in the constructs containing an engrafted bioactive peptide in CDR-H3.

### DTLacO parental chicken (clone #1) heavy chain variable region: (DTLacO VH) (SEQ ID NO:23)

AVTLDESGGGLQTPGRALSLVCKASGFTFSSYNMGWVRQAPGKGLEFVAGIDNTGR
YTGYGSAVKGRATISRDNGQSTVRLQLNNLRAEDTGTYYCAKAAGGSGYCGSGAYI
DAWGHGTEVIVSS

The V_{H} CDRs (31-35 (H1); 50-66 (H2); and 99-114 (H3) are underlined, and the Framework regions (1-30 (FR-1); 36-49 (FR-2); 67-98 (FR-3) and 115-125 (FR-4) are italicized.

### DTLacO parental chicken (clone #2) heavy chain variable region (DTLacO VH) (SEQ ID NO:91)

AVTLDESGGGLQTPGGALSLVCKASGFTFSSNAMGWVRQAPGKGLEWVAGIDDDGS
GTRYAPAVKGRATISRDNGQSTLRLQLNNLRAEDTGIYYCTKCAYSSGCDYEGGYID
AWGHGTEVIVSS

Conserved FR-1 region from the DTLacO VH is set forth as SEQ ID NO:24:
AVTLDESGGGLQTPGXALSLVCKASGFTFS
Where X=R or G

Conserved FR-2 region from the DTLacO VH is set forth as SEQ ID NO:25:
WVRQAPGKGLEXVA
Where X=F or W

Conserved FR-3 region from the DTLacO VH is set forth as SEQ ID NO:26:
RATISRDNGQSTX₁RLQLNNLRAEDTGIYYCX₂K
Where:
X₁=V or L, and
X₂=A or T

Conserved FR-3 flanking region adjacent to CDR-H3 is set forth as SEQ ID NO:27:
YYCXK
where X=A or T

Conserved FR-4 region from the DTLacO VH is set forth as SEQ ID NO:28:
WGHGTEVIVSS

Conserved FR-4 flanking region adjacent to CDR-H3 is set forth as SEQ ID NO:29
WGHGT

As shown in FIGURE 4, in some cases, a peptide linker was included at the amino terminus of the bioactive peptide, or at the carboxy terminus of the bioactive peptide, or at both locations. The peptide linker may be any flexible linker sequence, such a sequence shown in TABLE 4. In some cases, the linker sequence was derived from the native CDR-H3 sequence in the parental clone. As further shown in FIGURE 4, in some cases, the bioactive peptide sequence replaced all but one of the sixteen original amino acid residues of the native CDR-H3 (see, e.g. SGMI-1L), wherein the remaining one amino acid sequence is included as a linker. In some cases, eight of the sixteen original amino acid residues of the native CDR-H3 were retained in either the C-terminal linker (see e.g., SGMI-1L5), and up to fourteen of the original sixteen amino acid residues of the native CDR-H3 were retained in the C-terminal and N-terminal linker regions (see SGMI-L7).

### 2. SGMI-1 and SGMI-2 engrafted into the CDR-L1 of a parental chicken light chain variable region.

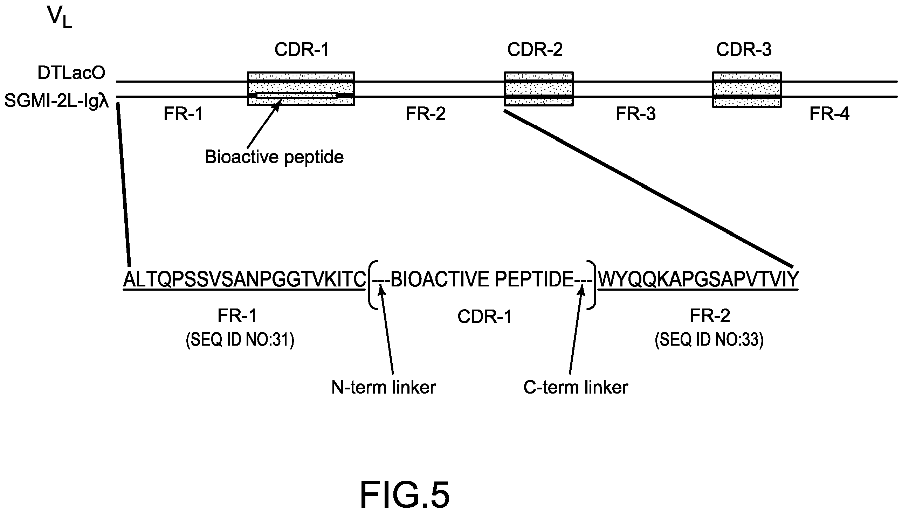

A DT40 chicken light chain variable region was chosen as the starting parental clone for use as a scaffold into which SGMI-1 or SGMI-2 peptide sequences were engrafted into the CDR-L1 region, as shown in FIGURES 5 and 6.

FIGURE 5 illustrates an exemplary parental (DTLacO) variable light chain polypeptide sequence compared to a variable light chain polypeptide sequence comprising a bioactive peptide amino acid sequence engrafted within CDR-L1. As shown in FIGURE 5, the chicken light chain variable region contains three CDRs (CDR-L1, CDR-L2 and CDR-L3), flanked by four framework regions (FR-1, FR-2, FR-3 and FR-4). Similar to the results obtained with CDR-H3 in the variable heavy chain polypeptide, the inventors have discovered that by engrafting a bioactive peptide sequence (e.g. SGMI-1) into CDR-L1 of the variable light chain polypeptide from a parental chicken antibody (which provides the antibody scaffold), the parental antibody comprising the engrafted bioactive peptide sequence is converted into an antibody that comprises biological activity of the bioactive peptide (i.e., inhibition of the lectin pathway was observed with the construct SGMI-IL, data not shown).

Exemplary parental chicken light chain variable regions are provided below. As shown in FIGURE 6, although the native CDR regions vary between parental clones, the Framework regions between the CDRs are conserved in chicken, accordingly, a consensus FR-1, FR-2, FR-3 and FR-4 sequence derived from an alignment of several different parental chicken light chain regions is also provided below. As further shown in FIGURE 6, in FR-1 there is a conserved cysteine (C) residue at the position immediately adjacent to CDR-L1 in the parental clones (corresponding to the cysteine at position 23 in SEQ ID NO:31), which is retained in FR-1 in the constructs containing an engrafted bioactive peptide in CDR-L1. As further shown in FIGURE 6, in FR-2 there is a conserved tryptophan (W) residue at the position immediately adjacent the CDR-L1 in the parental clones (corresponding to the tryptophan at position 1 in SEQ ID NO:33), which is retained in FR-2 in the constructs containing an engrafted bioactive peptide in CDR-L1.

### DTLacO chicken (clone #1) light chain variable region (DTLacO VL) (SEQ ID NO:30)

ALTQPSSVSANPGGTVKITCSGDSSYYGWYQQKAPGSAPVTVIYDNTNRPSNIPSRFS
GSKSGSTATLTITGVRADDNAVYYCASTDSSSTAFGAGTTLTVL

The V_{L} CDRs (21-28 (L1); 45-51 (L2); and 84-92 are underlined and the Framework regions (1-20 (FR-1); 29-44 (FR-2); 52-83 (FR-3) and 93-102 (FR-4) are italicized.

### DTLacO chicken (clone#2) light chain variable region (DTLacO VL) SEQ ID NO:92)

ALTQPASVSANPGETVKITCSGGGSYAGSYYGWYQQKAPGSAPVTLIYYNNKRPSD
IPSRFSGSLSGSTNTLTITGVRADDEAVYFCGSADNSGAAFGAGTTLTVL

Conserved FR-1 region from the DTLacO VL is set forth as SEQ ID NO:31:
ALTQPX₁SVSANX₂GX₃TVKITC

Where:
Xi=A or S
X₂=L or P
X₃=G or E

Conserved FR-1 flanking region adjacent to CDR-L1 is set forth as SEQ ID NO:32
VKITC

Conserved FR-2 region from the DTLacO VL is set forth as SEQ ID NO:33:
WYQQKX₁PGSAPVTX₂IY

Where
Xi=A or S,
X₂=V or L

Conserved FR-2 flankinggion adjacent to CDR-L1 is set forth as SEQ ID NO:34
WXQQK

Wherein X=Y, F or H.

Conserved FR-3 region from the DTLacO VL is set forth as SEQ ID NO:35:
X₁IPSRFSGSX₂SGSTX₃TLTITGVRADDX₄AVYXsC

Where:
Xi=N or D
X₂=K or L
X₃=A or N
X₄=N or E
X₅=Y or F

Conserved FR-4 region from the DTLacO VL is set forth as SEQ ID NO:36
FGAGTTLTVL

As shown in FIGURE 6, in some cases, a peptide linker was included at the amino terminus of the bioactive peptide, or at the carboxy terminus of the bioactive peptide, or at both locations. The peptide linker may be any flexible linker sequence, such as the sequences shown in TABLE 4. In some cases, the linker sequence was derived from the native CDR-L1 sequence in the parental clone. As further shown in FIGURE 6, in some cases, the bioactive peptide replaced five of the thirteen original amino acid residues of the native CDR-L1 (see, e.g. SGMI-2L), retaining a portion of the original CDR-L1 sequence as a peptide linker flanking the bioactive peptide sequence.

**Table 4: Exemplary Peptide Linkers for engrafting bioactive peptides into CDRs:**

| SEQ ID NO: | Sequence |
|---|---|
| 12 | GTGGGSGSSSRS |
| 13 | GTGGGSGSSS |
| 37 | AAGGSG |
| 38 | AAGGSGGSGA |
| 39 | YIDA |
| 40 | AYIDA |
| 41 | GTGGGSGSSS YIDA |
| 42 | GSGAYIDA |
| 43 | AAGGSGGSGAYIDA |
| 44 | SGGGS |
| 45 | YYYG |
| 46 | GSGA |
| SGG | SGG |
| 192 | SGGGGSGS |
| 193 | SGGGSGGGS |
| 194 | GTGGGSGSSSYG |
| 195 | GSGSYG |

In some cases, the chicken variable heavy chain region is fused to a human IgG1 constant region, resulting in a chicken/human chimeric antibody. An exemplary human IgG1 constant region is provided below as SEQ ID NO:47.
human IgG1 Constant Region (CH1-hinge-CH2-CH3): SEQ ID NO:47

In some cases, the chicken variable light chain region is fused to a human lambda light chain constant region, resulting in a chicken/human chimeric antibody. An exemplary human lambda light chain constant region is provided below as SEQ ID NO:48.

### Human lambda light chain constant region (SEQ ID NO:48)

GQPKAAPSVTLFPPSSEELQANKATLVCLISDFYPGAVTVAWKADSSPVKAGVET
TTPSKQSNNKYAASSYLSLTPEQWKSHRSYSCQVTHEGSTVEKTVAPTECS

The resulting polynucleotide constructs were designated pcDNA3-SGMI-1L-IgG1, -1M-IgG1, -1S-IgG1, and -1-L1-IgG1 to -1-L12-IgG1 (SEQ ID NOS: 49, 51, 53, 55, 57, 59, 61, 63, 65, 67, 69, 71, 73, 75 and 77) and pcDNA3-SGMI-2L-Igλ, -2M-Igλ, and -2S-Igλ (SEQ ID NOS:79, 81 and 83), while the polypeptides were termed Ab-SGMI-1L-IgG1,-1M-IgG1, -1S-IgG1, and -1-L1-IgG1 to -1-L12-IgG1 (SEQ ID NOS: 50, 52, 54, 56, 58, 60, 62, 64, 66, 68, 70, 72, 74, 76 and 78), as shown in FIGURE 4, and Ab-SGMI-2L-Igλ-, -2M-Igλ, and -2S-Igλ (SEQ ID NOS: 80, 82 and 84), as show in FIGURE 6. As further shown in FIGURE 6, various linker combinations and a modification of one of the Vernier zone residues (i.e. outside of CDR-L1) were used to generate additional polypeptides: Ab-SGMI-1-F1-Igλ, Ab-SGMI-1-F2-Igλ, Ab-SGMI-1-F3-Igλ, Ab-SGMI-1-F4-Igλ, Ab-SGMI-1-F5-Igλ, Ab-SGMI-1-F3.1-Igλ, Ab-SGMI-1-F3.2-Igλ, and Ab-SGMI-1-F3.3-Igλ.

Freestyle 293-F or Expi293F cells were transiently transfected with combinations of expression plasmids as follows: (a) pcDNA3-SGMI-1-IgG1-1L, (et al.), plus a light chain plasmid encoding the DTLacO VL; (b) pcDNA3-SGMI-2-Igλ-L1 (et. al.), plus a heavy chain plasmid encoding the DTLacO VH; (c) pcDNA3-SGMI-1-IgG1-1L plus pcDNA3-SGMI-2-Igλ-L1, and (d) pcDNA3-SGMI-1-F1-Igλ to pcDNA3-SGMI-1-F3.3-Igλ plus a heavy chain plasmid encoding the DTLacO VH. After four days of incubation at 37°C, the culture media were harvested and the SGMI-bearing chimeric antibodies were purified by Protein A affinity chromatography.

### Results:

### Chimeric chicken/human antibodies comprising SGMI-1 engrafted into CDR-H3

The Wieslab^{®} Complement System Screen, MBL Pathway, as described in Example 2, was used to measure functionality of the chimeric antibodies. Assays were run in duplicate with the SGMI-1Fc (generated as described in Example 2) as the positive (inhibitory) controls. A matching isotype antibody was included as a negative control.

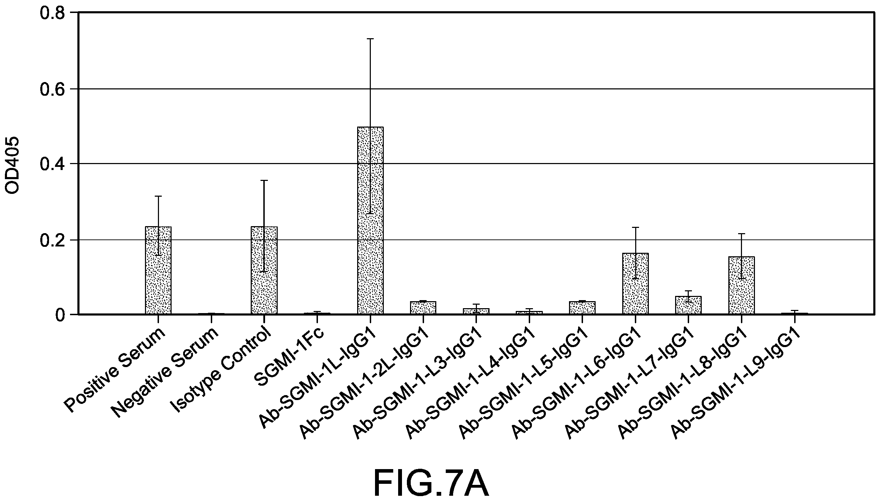

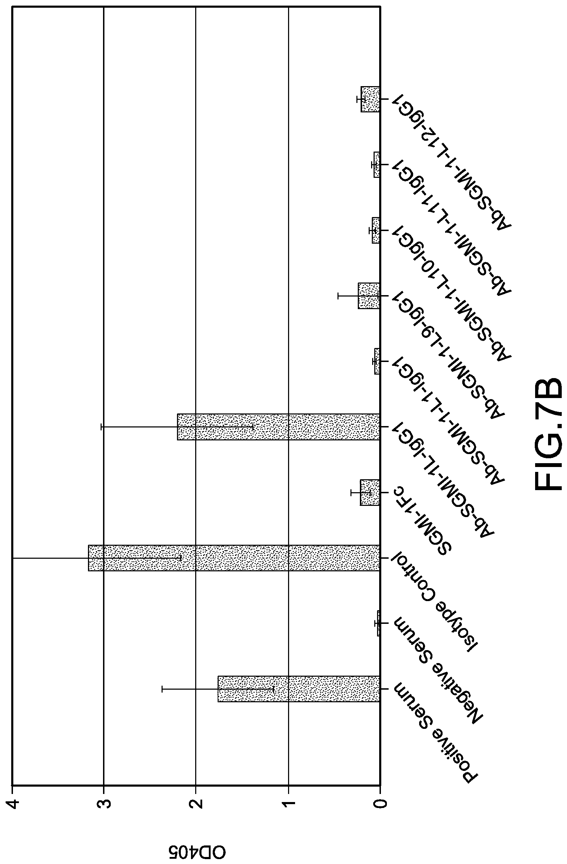

FIGURES 7A and 7B graphically illustrate the inhibitory activity of various representative chimeric chicken/human mAbs containing SGMI-1 engrafted into CDR-H3 on MBL complement activity. The data are distributed across two figures because the assays were conducted at different times. As shown in FIGURE 7A and 7B, several of the chimeric mAbs containing SGMI-1 engrafted within the CDR-H3 inhibit C5b-C9 deposition to a degree similar to the positive SGMI1-Fc fusion protein (e.g., Ab-SGMI-1-L2, -L3, -L4, -L5, -L7, -L9, -L1, -L10, -L11 and -L12). As shown in FIGURE 4, these constructs differ only in the nature of the flexible linkers separating the inhibitory peptide from the antibody framework regions. Interestingly, another chimeric mAb with SGMI-1 engrafted into the CDR-H3, referred to as "Ab-SGMI-1L," has no inhibitory activity. As shown in FIGURE 4, Ab-SGMI-1L has only a one amino acid residue linker between the bioactive peptide and framework segments.

The Ab-SGMI-1 antibodies were also assessed for lectin pathway inhibition in an assay of C3b deposition on mannan-coated beads. This assay, which determines degree of activity by flow cytometry, offers greater resolution than the Wieslab^{®} assay. The Lectin Pathway bead assay was carried out as follows: mannan was adsorbed to 7 µM-diameter polystyrene beads (Bangs Laboratories; Fishers, IN, USA) overnight at 4°C in carbonate-bicarbonate buffer (pH 9.6). The beads were washed in PBS and exposed to 10% serum, or 10% serum pre-incubated with antibodies or inhibitors. The serum-bead mixture was incubated at room temperature for one hour while agitating. Following the serum incubation, the beads were washed, and C3 deposition on the beads was measured by detection with an anti-C3c rabbit polyclonal antibody (Dako North America; Carpinteria, CA, USA) and a PE-Cy5 conjugated goat anti-rabbit secondary antibody (Southern Biotech; Birmingham, AL, USA). Following the staining procedure, the beads were analyzed using a FACS Calibur cytometer. The beads were gated as a uniform population using forward and side scatter, and C3 deposition was apparent as FL3-positive particles (FL-3, or "FL-3 channel" indicates the 3rd or red channel on the cytometer). The Geometric Mean Fluorescence Intensity (MFI) for the population for each experimental condition was plotted relative to the antibody/inhibitor concentration to evaluate lectin pathway inhibition.

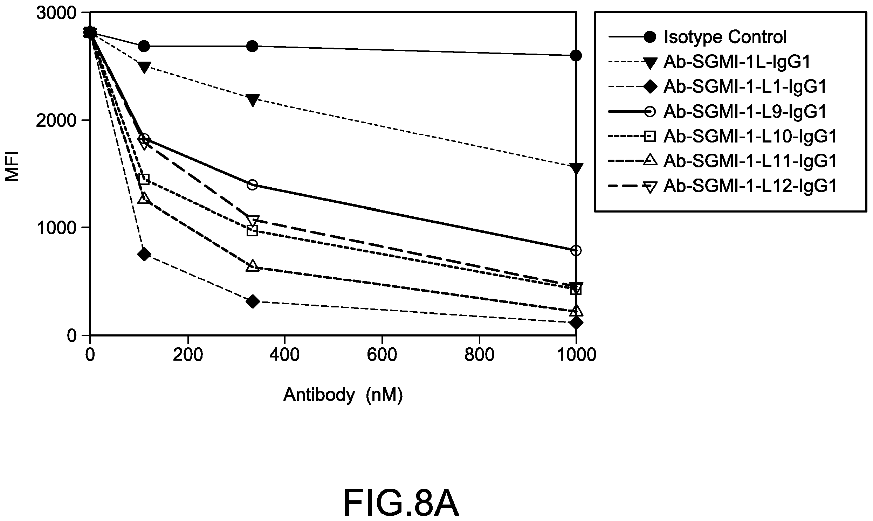

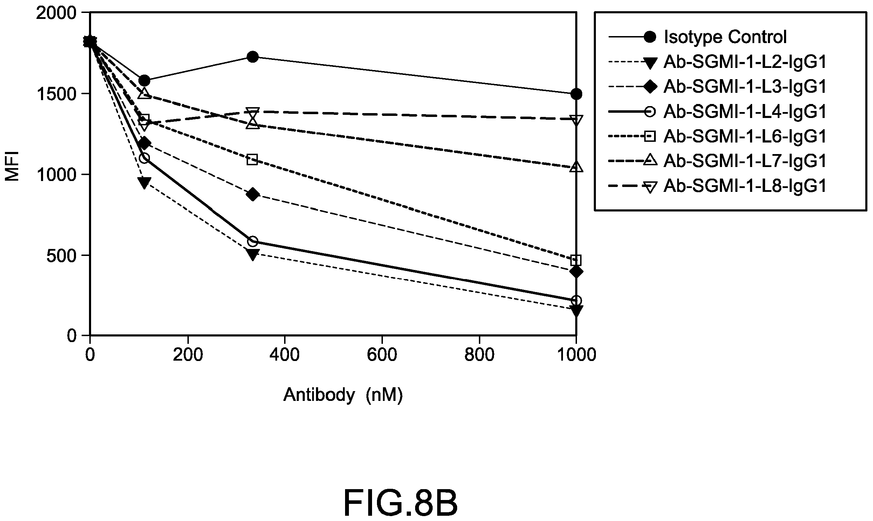

FIGURES 8A and 8B graphically illustrate the inhibitory activity of various representative chimeric chicken/human mAbs containing SGMI-1 engrafted into CDR-H3 on complement C3b deposition activity in a dose-response manner. As shown in FIGURES 8A and 8B, all of the antibodies containing SGMI-1 engrafted into CDR-H3 inhibited lectin pathway activity in the bead assay, but with varying degrees of potency.

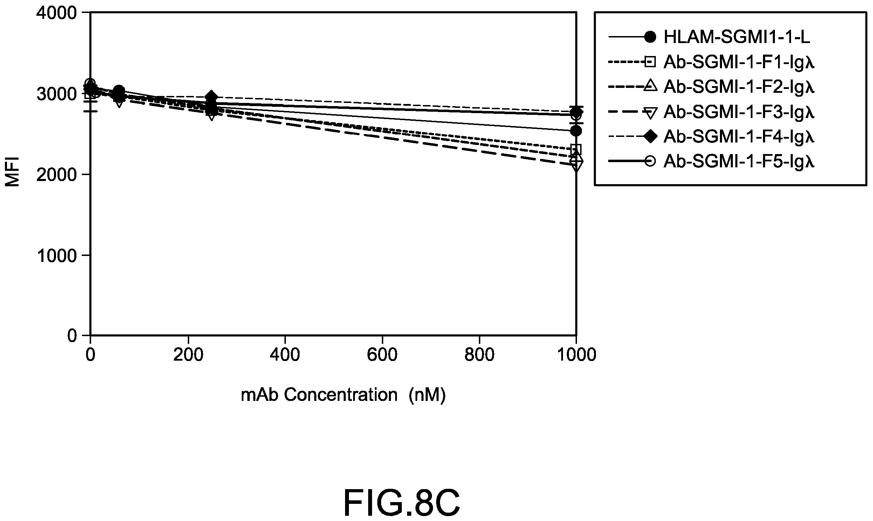

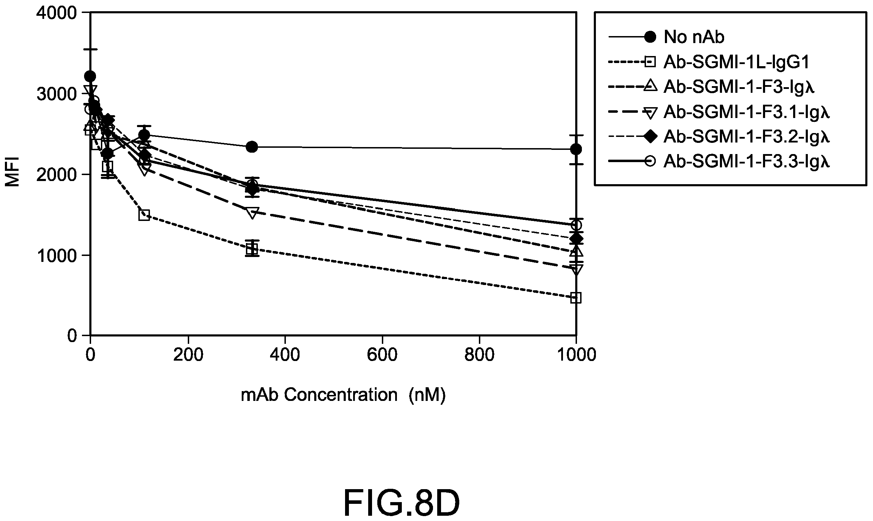

FIGURE 8C and 8D graphically illustrate the inhibitory activity of various representative chimeric chicken/human mAbs containing SGMI-1 engrafted into CDR-L1 on complement C3b deposition activity in a dose-response manner. As shown in FIGURE 8C, all of the antibodies containing SGMI-1 engrafted into CDR-L1 inhibited lectin pathway activity in the bead assay, with "F1", "F2" and "F3", which have various linker combinations and in some instances have a modification in the Vernier zone residue "Y" to "F" or "H" in FR-2 adjacent to CDR-L1 (as shown in FIGURE 6) having more inhibitory activity than "F4" and "F5." As shown in FIGURE 6, these constructs differ in the nature of the flexible linkers separating the inhibitory peptide from the antibody framework regions and in the amino acid at the second position of the FR-2 region. As further shown in FIGURE 6, the most potent antibody, Ab-SGMI-F3-Igλ, which contained a three amino acid flexible linker at the amino terminus of the SGMI peptide was further optimized by creating constructs "F3.1", "F3.2" and "F3.3", which increased the size of the flexible linker at the amino terminus of the SGMI-1 peptide to five amino acids, seven amino acids and nine amino acids, respectively. As shown in FIGURE 8D, a flexible linker of five amino acids (construct "F3.1") at the amino terminus of the SGMI-1 peptide resulted in the most potent inhibition of the lectin pathway.

It is noted that the differences between the antibodies are more readily discerned in this bead assay as compared to the Wieslab^{®} assay.

In summary, these results demonstrate that inhibitory therapeutic polypeptides may be generated by engrafting a bioactive peptide into the CDR-H3 or into the CDR-L1 of a chicken antibody scaffold.

### Chimeric chicken/human antibodies comprising SGMI-2 engrafted into CDR-L1

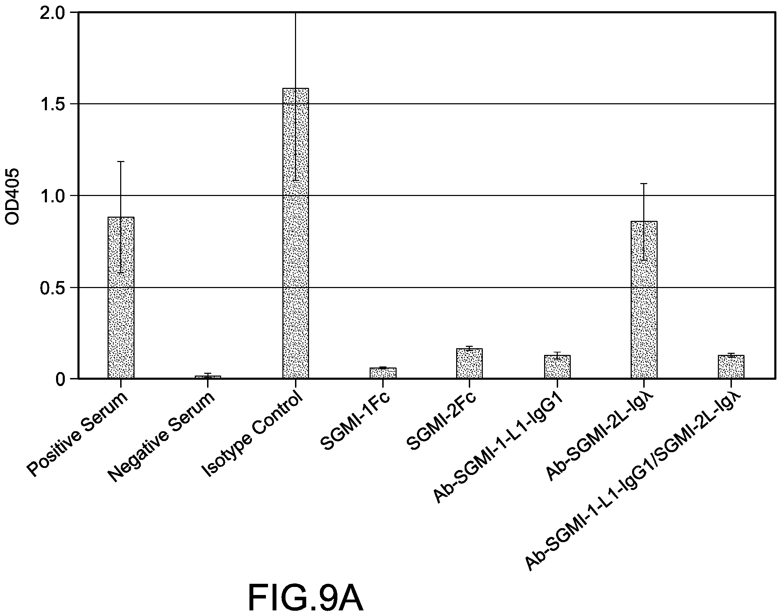

FIGURE 9A graphically illustrates that a chimeric chicken/human mAb comprising SGMI-2 engrafted within CDR-L1 (Ab-SGMI-2L-Igk) exerts little to no inhibitory activity in the Wieslab complement system MBL pathway assay. These results leave room for optimization of the linker elements flanking the bioactive peptide, which significantly impacted the efficacy of the SGMI-1-containing mAbs (as shown in Figures 7A, 7B, and 8A-8D).

### Chimeric chicken/human antibodies comprising SGMI-1 and SGMI-2

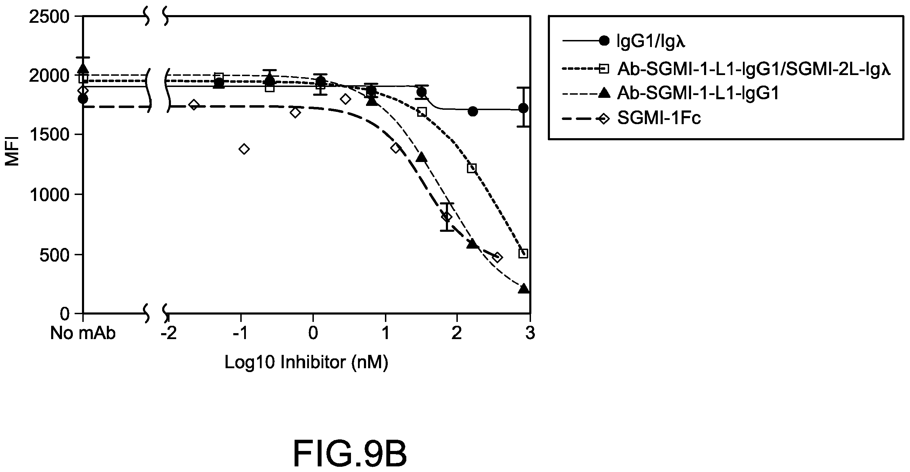

Figure 9A also shows the activity of a chimeric chicken/human antibody comprising SGMI-1 and SGMI-2 engrafted into CDR-H3 and CDR-L1, respectively. Ab-SGMI-1-L1-IgG1/SGMI-2-L-Igλ is nearly as potent as the mAb containing only the SGMI-1 peptide (Ab-SGMI-1-L1-IgG1). This outcome was confirmed using the flow cytometric mannan-coated bead assay, as shown in Figure 9B. Together, these data demonstrate that the SGMI-1 peptide engrafted into CDR-H3 inhibits the lectin pathway whether or not the SGMI-2 peptide is present engrafted into CDR-L1. Based on the results described herein, further optimization of the SGMI-2 flanking linkers is expected to add MASP-2 inhibitory activity to the antibody already carrying SGMI-1-mediated MASP-1 inhibitory activity.

### EXAMPLE 4

This Example describes the generation of chimeric antibodies comprising one or more bioactive peptides (e.g. SGMI-1 or SGMI-2) fused onto the amino or carboxy termini of the heavy and light chains of a chimeric chicken/human antibody.

### Rationale:

As demonstrated in Examples 2 and 3, the inhibitory functions of the SGMI-1 and SGMI-2 peptides (and truncated variants thereof) were preserved in the SGMI-Fc proteins and also, for SGMI-1, when displayed within the CDR regions of a full antibody. In this Example, experiments were carried out to determine whether the SGMI peptides would retain activity when fused to the amino or carboxy termini of antibody heavy or light chains of a chimeric chicken/human antibody.

**TABLE 5: Chimeric chicken/human antibodies with the bioactive peptides SGMI-1 and SGMI-2 fused to the N- or C-termini of the heavy or light chains.**

| **Antibody** | **Peptide Location on Antibody** | | | | **SEQ ID NO:** |
|---|---|---|---|---|---|
| | **HC-N** | **HC-C** | **LC-N** | **LC-C** | |
| Ab-IgG1-S10 | SGMI-1 | - | - | - | 94 |
| Ab-IgG1-S20 | SGMI-2 | | - | - | 96 |
| Ab-IgG1-S01 | - | SGMI-1 | - | - | 98 |
| Ab-IgG1-S02 | - | SGMI-2 | - | - | 100 |
| Ab-Igλ-S10 | - | | SGMI-1 | - | 102 |
| Ab-Igλ-S20 | | | SGMI-2 | | 104 |
| Ab-Igλ-S01 | | | - | SGMI-1 | 106 |
| Ab-Igλ-S02 | | | | SGMI-2 | 108 |

| | | | | | |
|---|---|---|---|---|---|
| Abbreviations in Table 5: "HC-N"= amino terminus of heavy chain "HC-C"=carboxyl terminus of heavy chain "LC-N"=amino terminus of light chain "LC-C"=carboxyl terminus of light chain | | | | | |

For the N-terminal fusions shown in TABLE 5, a peptide linker (SEQ ID NO: 14) was added between the bioactive peptide and the chicken variable region.

For the C-terminal fusions shown in TABLE 5, a peptide linker (SEQ ID NO:37) was added between the constant region and the bioactive peptide, and a second peptide "GSGA" was added at the C-terminal end of the fusion polypeptide to protect C-terminal SGMI peptides from degradation. These fusion constructs are illustrated schematically in FIGURE 10.

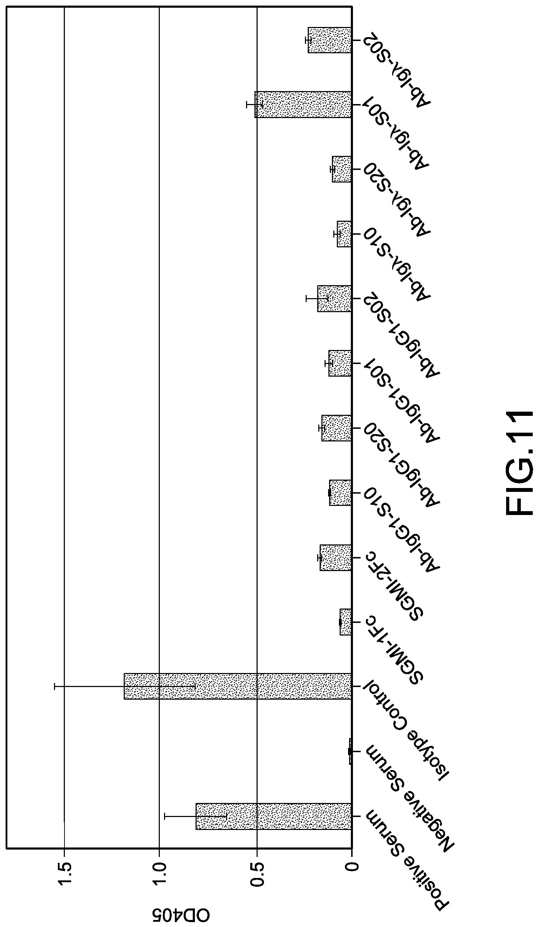

FIGURE 11 illustrates the inhibitory activity of the N- and C-terminal peptides in the Wieslab assay. Compared to the positive and negative controls, all of the fusion mAbs inhibited C5b-9 deposition. All except for one fusion mAb - SGMI-1 fused to the C-terminus of the light chain - exhibited levels of inhibition comparable to those of the control SGMI-1 and SGMI-2 Fc-fusion proteins. Several of these N- and C-terminal peptide-mAb fusions were also tested in the flow cytometric mannan-coated bead assay described in Example 3, with similar results (data not shown). These antibodies may be used for the development of bi-specific antibodies bearing combinations of SGMI-1 and SGMI-2.

### EXAMPLE 5

This Example describes the identification, using phage display, of fully human scFv antibodies that bind to MASP-2 and inhibit lectin-mediated complement activation while leaving the classical (Clq-dependent) pathway component of the immune system intact.

### Overview:

MASP-2 is a complex protein with many separate functional domains, including: binding site(s) for MBL and ficolins, a serine protease catalytic site, a binding site for proteolytic substrate C2, a binding site for proteolytic substrate C4, a MASP-2 cleavage site for autoactivation of MASP-2 zymogen, and two Ca⁺⁺ binding sites. As described in WO 2012/151481, hereby incorporated herein by reference, scFv antibody fragments were identified that bind with high affinity to MASP-2, and the identified scFv fragments were tested in a functional assay to determine if they were able to block MASP-2 functional activity. A functional assay that measures inhibition of lectin pathway C3 convertase formation was used to evaluate the "blocking activity" of the identified anti-MASP-2 scFv antibody fragments. It is known that the primary physiological role of MASP-2 in the lectin pathway is to generate the next functional component of the lectin-mediated complement pathway, namely the lectin pathway C3 convertase. The lectin pathway C3 convertase is a critical enzymatic complex (C4b2a) that proteolytically cleaves C3 into C3a and C3b. MASP-2 is not a structural component of the lectin pathway C3 convertase (C4b2a); however, MASP-2 functional activity is required in order to generate the two protein components (C4b, C2a) that comprise the lectin pathway C3 convertase. Furthermore, all of the separate functional activities of MASP-2 listed above appear to be required in order for MASP-2 to generate the lectin pathway C3 convertase. For these reasons, a preferred assay to use in evaluating the "blocking activity" of anti-MASP-2 Fab2s and scFv antibody fragments is believed to be a functional assay that measures inhibition of lectin pathway C3 convertase formation.

As described herein, MASP-2 antibodies, such as for example the fully human MASP-2 antibodies identified by screening a phage display library, may be used as a scaffold to generate MASP-2 antibody-SGMI fusions with enhanced inhibitory activity.

### Generation of MASP-2 inhibitory scaffold antibodies

As described in WO2012/151481, the variable light and heavy chain fragments of the MASP-2 inhibitory antibodies were isolated in both a scFv format and in a full-length IgG format. The human MASP-2 antibodies are useful for inhibiting cellular injury associated with lectin pathway mediated alternative complement pathway activation while leaving the classical (Clq-dependent) pathway component of the immune system intact. In some cases, the MASP-2 inhibitory scaffold antibodies have the following characteristics: (a) high affinity for human MASP-2 (e.g., a K_{D} of 10 nM or less), and (b) inhibit MASP-2-dependent complement activity in 90% human serum with an IC₅₀ of 30 nM or less.

### Methods:

### Expression of full-length catalytically inactive MASP-2:

The full-length cDNA sequence of human MASP-2 (SEQ ID NO: 3), encoding the human MASP-2 polypeptide with leader sequence (SEQ ID NO:4) was subcloned into the mammalian expression vector pCI-Neo (Promega), which drives eukaryotic expression under the control of the CMV enhancer/promoter region (described in Kaufman R.J. et al., Nucleic Acids Research 19:4485-90, 1991; Kaufman, Methods in Enzymology, 185:537-66 (1991)).

In order to generate catalytically inactive human MASP-2A protein, site-directed mutagenesis was carried out as described in US2007/0172483, hereby incorporated herein by reference. The PCR products were purified after agarose gel electrophoresis and band preparation and single adenosine overlaps were generated using a standard tailing procedure. The adenosine-tailed MASP-2A was then cloned into the pGEM^{®}-T Easy vector (Promega) and transformed into *E. coli.* The human MASP-2A was further subcloned into either of the mammalian expression vectors pED (SinoBio) or pCI-Neo (Promega).

The MASP-2A expression construct described above was transfected into DXB1 cells using the standard calcium phosphate transfection procedure (Maniatis et al., 1989). MASP-2A was produced in serum-free medium to ensure that preparations were not contaminated with other serum proteins. Culture medium was harvested from confluent cells every second day (four times in total). The level of recombinant MASP-2A averaged approximately 1.5 mg/liter of culture medium. The MASP-2A (Ser-Ala mutant described above) was purified by affinity chromatography on MBP-A-agarose columns

### MASP-2A ELISA on ScFv Candidate Clones identified by panning/scFv conversion and filter screening

A phage display library of human immunoglobulin light- and heavy-chain variable region sequences in an scFv format was subjected to antigen panning followed by automated antibody screening and selection to identify high-affinity scFv antibodies to human MASP-2 protein. Three rounds of panning the scFv phage library against HIS-tagged or biotin-tagged MASP-2A were carried out. The third round of panning was eluted first with MBL and then with TEA (alkaline). To monitor the specific enrichment of phages displaying scFv fragments against the target MASP-2A, a polyclonal phage ELISA against immobilized MASP-2A was carried out. The scFv genes from panning round 3 were cloned into a pHOG expression vector and run in a small-scale filter screening to look for specific clones against MASP-2A.

Bacterial colonies containing plasmids encoding scFv fragments from the third round of panning were picked, gridded onto nitrocellulose membranes and grown overnight on non-inducing medium to produce master plates. A total of 18,000 colonies were picked and analyzed from the third panning round, half from the competitive elution and half from the subsequent TEA elution. Panning of the scFv phagemid library against MASP-2A followed by scFv conversion and a filter screen yielded 137 positive clones. 108/137 clones were positive in an ELISA assay for MASP-2 binding, of which 45 clones were further analyzed for the ability to block MASP-2 activity in normal human serum.

### Assay to measure inhibition of formation of Lectin Pathway C3 convertase

A functional assay that measures inhibition of lectin pathway C3 convertase formation was used to evaluate the "blocking activity" of the MASP-2 scFv candidate clones. MASP-2 serine protease activity is required in order to generate the two protein components (C4b, C2a) that comprise the lectin pathway C3 convertase. Therefore, a MASP-2 scFv that inhibits MASP-2 functional activity (i.e., a blocking MASP-2 scFv), will inhibit *de novo* formation of lectin pathway C3 convertase. C3 contains an unusual and highly reactive thioester group as part of its structure. Upon cleavage of C3 by C3 convertase in this assay, the thioester group on C3b can form a covalent bond with hydroxyl or amino groups on macromolecules immobilized on the bottom of the plastic wells via ester or amide linkages, thus facilitating detection of C3b in the ELISA assay.

Yeast mannan is a known activator of the lectin pathway. In the following method to measure formation of C3 convertase, plastic wells coated with mannan were incubated with diluted human serum to activate the lectin pathway. The wells were then washed and assayed for C3b immobilized onto the wells using standard ELISA methods. The amount of C3b generated in this assay is a direct reflection of the *de novo* formation of lectin pathway C3 convertase. MASP-2 scFv clones at selected concentrations were tested in this assay for their ability to inhibit C3 convertase formation and consequent C3b generation.

### Methods:

The 45 candidate clones identified as described above were expressed, purified and diluted to the same stock concentration, which was again diluted in Ca⁺⁺ and Mg⁺⁺ containing GVB buffer (CaMgGVB: 4.0 mM barbital, 141 mM NaCl, 1.0 mM MgCl₂, 2.0 mM CaCl₂, 0.1% gelatin, pH 7.4) to assure that all clones had the same amount of buffer. The scFv clones were each tested in triplicate at the concentration of 2 µg/mL. The positive control was OMS100 Fab2 and was tested at 0.4 µg/mL. C3b formation was monitored in the presence and absence of the scFv/IgG clones.

Mannan was diluted to a concentration of 20 µg/mL (1 µg/well) in 50mM carbonate buffer (15mM Na₂CO₃ + 35mM NaHCO₃ + 1.5 mM NaN₃), pH 9.5 and coated on an ELISA plate overnight at 4°C. The next day, the mannan-coated plates were washed 3 times with 200 µl PBS. 100 µl of 1% HSA blocking solution was then added to the wells and incubated for 1 hour at room temperature. The plates were washed 3 times with 200 µl PBS, and stored on ice with 200 µl PBS until addition of the samples.

Normal human serum was diluted to 0.5% in CaMgGVB buffer, and scFv clones or the OMS100 Fab2 positive control were added in triplicates at 0.01 µg/mL; 1 µg/mL (only OMS100 control) and 10 µg/mL to this buffer and pre-incubated 45 minutes on ice before addition to the blocked ELISA plate. The reaction was initiated by incubation for one hour at 37°C and was stopped by transferring the plates to an ice bath. C3b deposition was detected with a Rabbit α-Mouse C3c antibody followed by Goat α-Rabbit HRP. The negative control was buffer without antibody (no OMS100 = maximum C3b deposition), and the positive control was buffer with EDTA (no C3b deposition). The background was determined by carrying out the same assay except that the wells were mannan-free. The background signal derived from wells without mannan was subtracted from the signals in the mannan-containing wells. A cut-off criterion was set at half of the activity of an irrelevant scFv clone (VZV) and buffer alone.

Results: Based on the cut-off criterion, 13 clones were found to block the activity of MASP-2. All 13 clones producing > 50% pathway suppression were selected and sequenced, yielding 10 unique clones. All ten clones were found to have the same light chain subclass, λ3, but three different heavy chain subclasses: VH2, VH3 and VH6. In the functional assay, five out of the ten candidate scFv clones gave IC₅₀ nM values less than the 25 nM target criterion using 0.5% human serum.

**TABLE 6: Sequence of ScFv Clones shown in FIGURES 14A and B and FIGURES 15A and B**

| **scFv Clone Reference ID** | **Full length amino acid sequence** |
|---|---|
| 17D20 | SEQ ID NO:151 |
| 18L16 | SEQ ID NO:152 |
| 4D9 | SEQ ID NO:153 |
| 17L20 | SEQ ID NO:154 |
| 17N16 | SEQ ID NO:155 |
| 3F22 | SEQ ID NO:156 |
| 9P13 | SEQ ID NO:157 |

FIGURE 14A and 14B show an amino acid sequence alignment of the full length scFv clones 17D20, 18L16, 4D9, 17L20, 17N16, 3F22 and 9P13. The scFv clones comprise a heavy chain variable region (aa1-120), a linker region (aa121-145) and a light chain variable region (aa 146-250). As shown in FIGURE 14A, alignment of the heavy chain region (residues 1-120) of the most active clones revealed two distinct groups belonging to VH2 and VH6 gene family, respectively. As further shown in FIGURE 14A, the VH region with respect to the clones of the VH2 class: (17D20, 18L16 and 4D9) has variability in 20aa positions in the total 120 amino acid region (i.e., 83% identity).

As further shown in FIGURE 14A, the VH region with respect to the clones of the VH6 class: 17L20, 17N16, 3F22 and 9P13, has variability in 18 amino acid positions in the total 120 amino acid region (i.e., 85% identity). FIGURE 15A and B shows a sequence alignment of the scFv clones 17D20, 17N16, 18L16 and 4D9. The scFv clones comprise a heavy chain variable region (aa1-120), a linker region (aa121-145) and a light chain variable region (aa 146-250).

The scFv clones were tested for functional potency in 1% human serum using 1000 nM scFv purified protein for the ability to inhibit C3b deposition. 17N16, 17D20 and 18L16 were chosen for affinity maturation based on functional potency and different VH gene families.

### Chain Shuffling and Affinity Maturation of mother clones 17N16, 17D20 and 18L16

To identify antibodies with improved potency, the three mother scFv clones, 17N16, 17D20 and 18L16, identified as described above, were subjected to light chain shuffling. This process involved the generation of a combinatorial library consisting of the VH of each of the mother clones paired up with a library of naive, human lambda light chains (VL) derived from six healthy donors, which was then screened for scFv clones with improved binding affinity and/or functionality. Daughter clones were identified with higher functional activity and affinity than the mother clones, as shown in TABLE 7.

**TABLE 7: Comparison of functional potency in IC₅₀ (nM) of representative scFv daughter clones**

| **scFv clone** | **1% human serum C3 assay (IC₅₀ nM) (average value)** |
|---|---|
| 17D20mc | 38 |
| 17D20m_d3521N11 | 26 |
| 17N16mc | 68 |
| 17N16m_d17N9 | 48 |

### Heavy Chain Variable Region

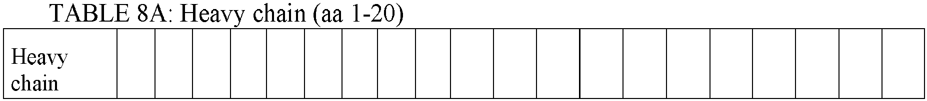

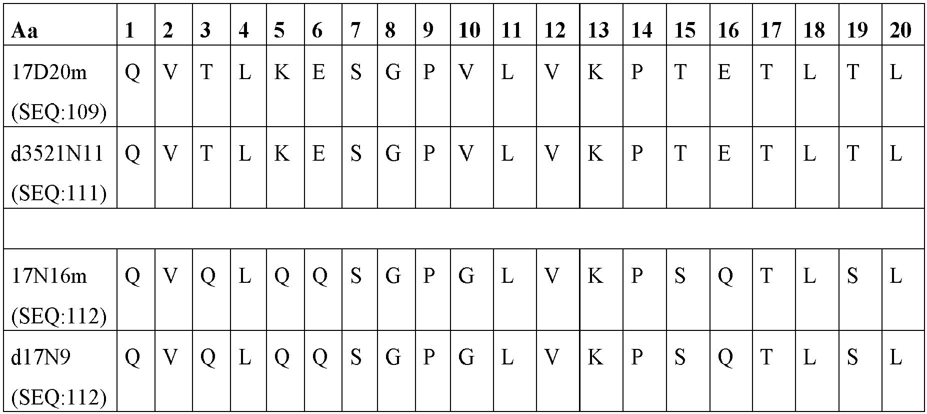

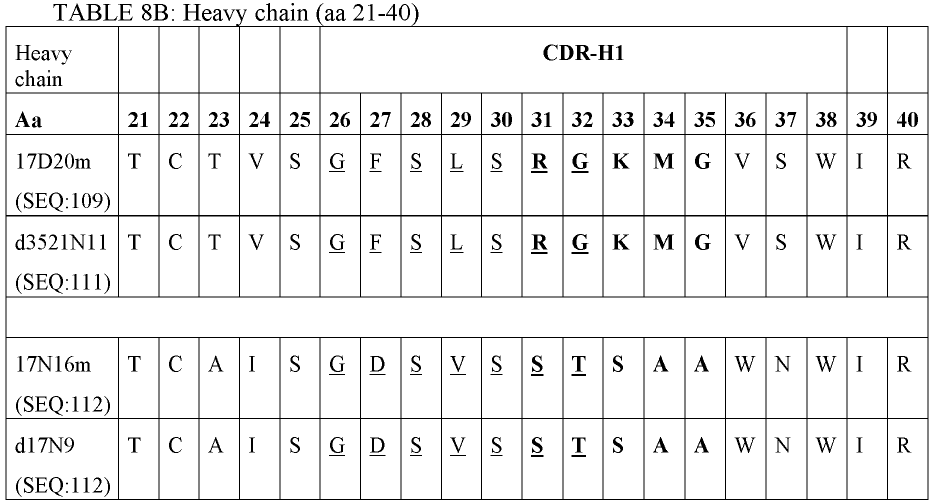

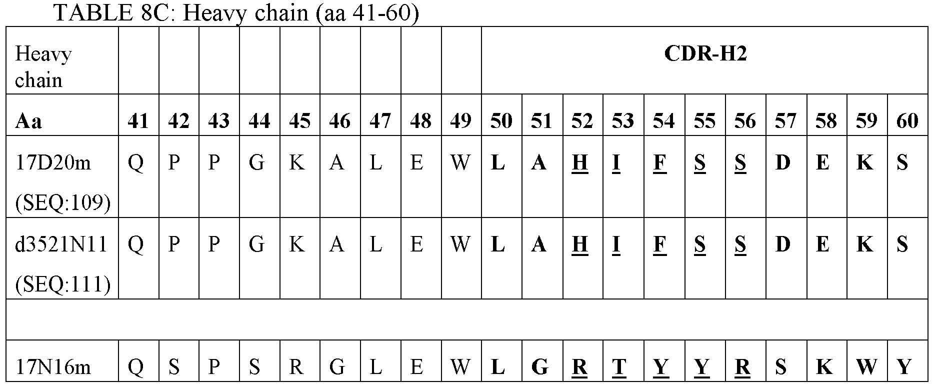

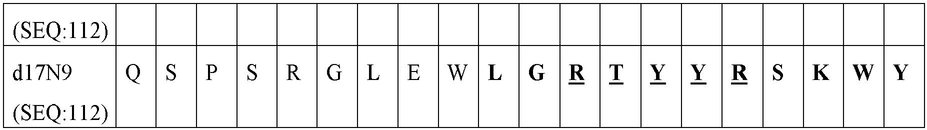

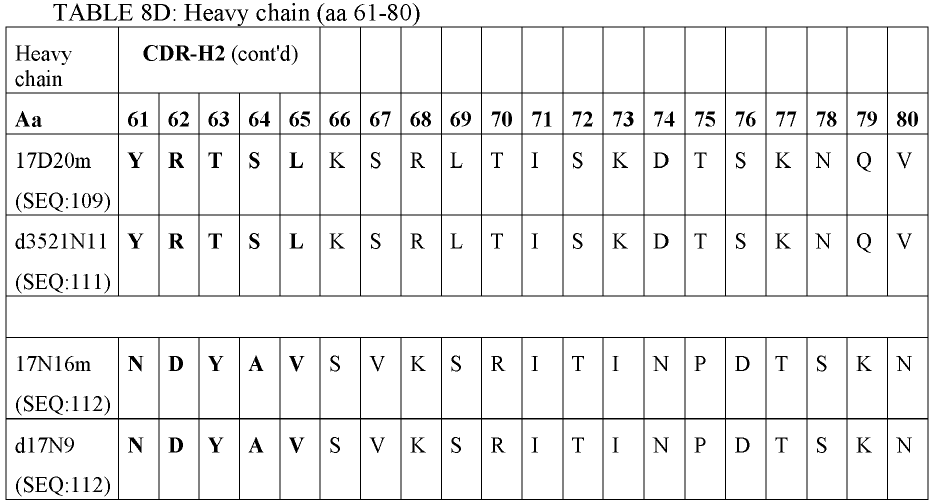

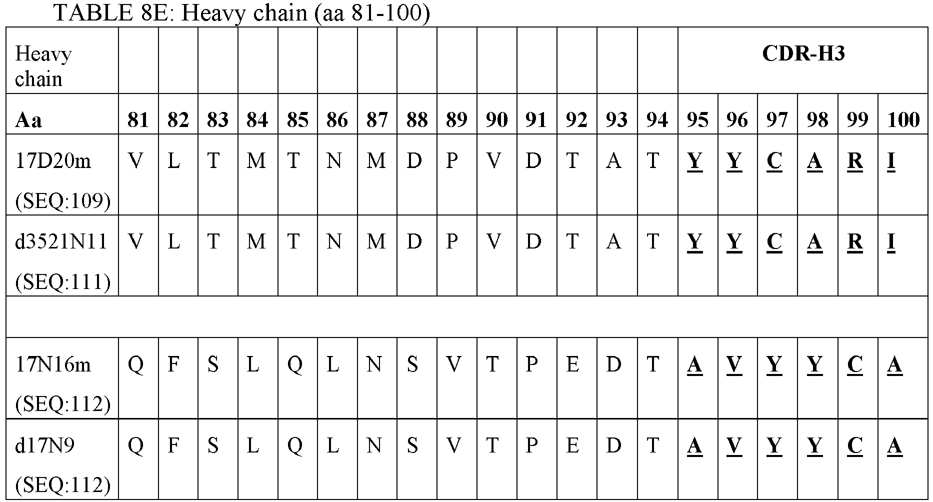

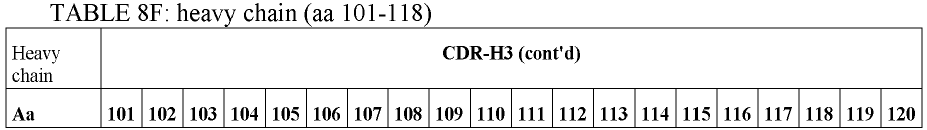

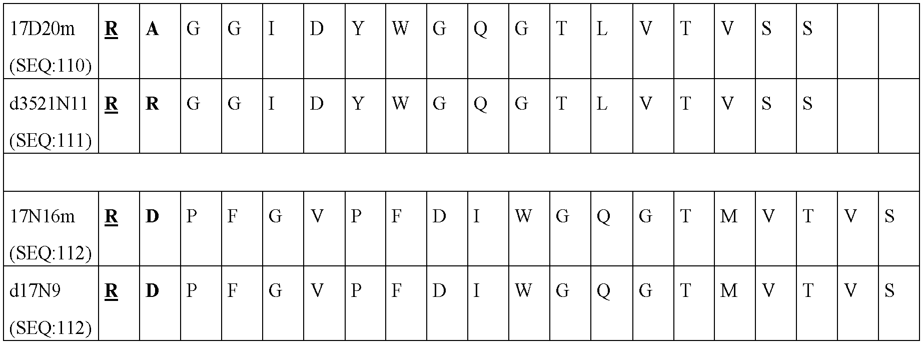

Presented below are the heavy chain variable region (VH) sequences for the mother clones and daughter clones listed above in TABLE 7 and TABLES 8A-F.

The Kabat CDRs (31-35 (H1), 50-65 (H2) and 95-102 (H3)) are bolded; and the Chothia CDRs (26-32 (H1), 52-56 (H2) and 95-101 (H3)) are underlined.
17D20 heavy chain variable region (VH) (SEQ ID NO:109): 
QVTLKESGPVLVKPTETLTLTCTVSGFSLSRGKMGVSWIRQPPGKALEW
LAHIFSSDEKSYRTSLKSRLTISKDTSKNQVVLTMTNMDPVDTATYYCARIRAG
GIDYWGQGTLVTVSS
17D20_35VH-21N11VL heavy chain variable region (VH) (SEQ ID NO:111) 
QVTLKESGPVLVKPTETLTLTCTVSGFSLSRGKMGVSWIRQPPGKALEW
LAHIFSSDEKSYRTSLKSRLTISKDTSKNQVVLTMTNMDPVDTATYYCARIRRG
GIDYWGQGTLVTVSS
17N16 heavy chain variable region (VH) (SEQ ID NO:112)

QVQLQQSGPGLVKPSQTLSLTCAISGDSVSSTSAAWNWIRQSPSRGLEWL
GRTYYRSKWYNDYAVSVKSRITINPDTSKNQFSLQLNSVTPEDTAVYYCARDPF
GVPFDIWGQGTMVTVSS

**TABLE 9: Heavv Chain CDRs**

| **Clone Reference** | **CDR** | **aa Sequence** | **SEQ ID NO:** |
|---|---|---|---|
| 17D20m | CDR-H1 (kabat) | RGKMG | 119 |
| d3521N11 | CDR-H1 (kabat) | RGKMG | 119 |
| 17N16m | CDR-H1 (kabat) | STSAA | 120 |
| d17N9 | CDR-H1 (kabat) | STSAA | 120 |
| 17D20m | CDR-H1 (chothia) | GFSLSRG | 121 |
| d3521N11 | CDR-H1 (chothia) | GFSLSRG | 121 |
| 17N16m | CDR-H1 (chothia) | GDSVSST | 122 |
| d17N9 | CDR-H1 (chothia) | GDSVSST | 122 |
| NimoAb101 (rat) | CDR-H1 | GFTFSNFGM | 166 |
| | | | |
| 17D20m | CDR-H2 (kabat) | LAHIFSSDEKSYRTSL | 123 |
| d3521N11 | CDR-H2 (kabat) | LAHIFSSDEKSYRTSL | 123 |
| 17N16m | CDR-H2 (kabat) | LGRTYYRSKWYNDYAV | 124 |
| d17N9 | CDR-H2 (chothia) | LGRTYYRSKWYNDYAV | 124 |
| 17D20m | CDR-H2 (chothia) | HIFSS | 125 |
| d3521N11 | CDR-H2 (chothia) | HIFSS | 125 |
| 17N16m | CDR-H2 (chothia) | RTYYR | 126 |
| d17N9 | CDR-H2 (chothia) | RTYYR | 126 |
| NimoAb101 (rat) | CDR-H2 | SISSGGTYI | 167 |
| | | | |
| 17D20m | CDR-H3 (kabat) | YYCARIRA | 127 |
| d3521N11 | CDR-H3 (kabat) | YYCARIRR | 128 |
| 17D20m and d3521N11 consensus | CDR-H3 (kabat) | YYCARIRX (wherein X at position 8 is A (Ala) or R (Arg)) | 146 |
| 17N16m | CDR-H3 (kabat) | AVYYCARD | 129 |
| d17N9 | CDR-H3 (kabat) | AVYYCARD | 129 |
| 17D20m | CDR-H3 (chothia) | YYCARIR | 130 |
| d3521N11 | CDR-H3 (chothia) | YYCARIR | 130 |
| 17N16m | CDR-H3 (chothia) | AVYYCAR | 131 |
| d17N9 | CDR-H3 (chothia) | AVYYCAR | 131 |
| NimoAb101 (rat) | CDR-H3 | GPYHSRYIPYLMDA | 168 |

### Light Chain Variable Regions

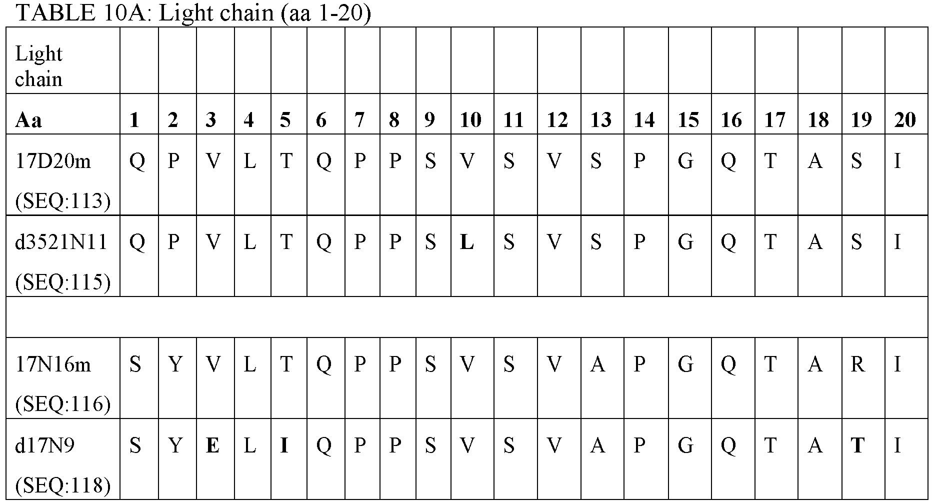

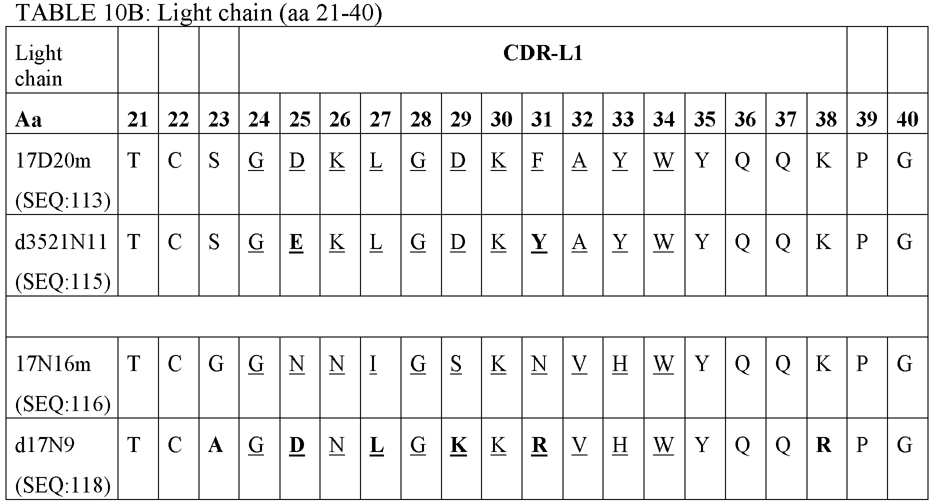

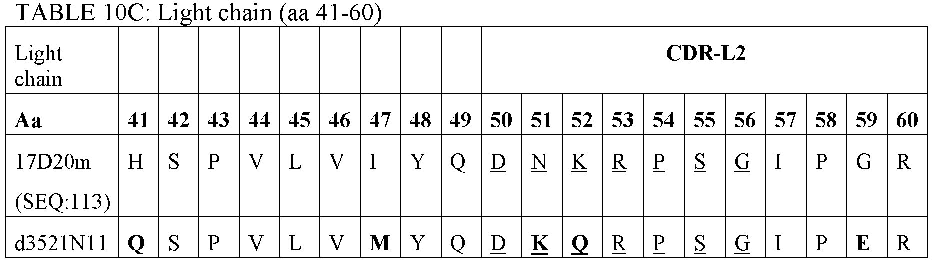

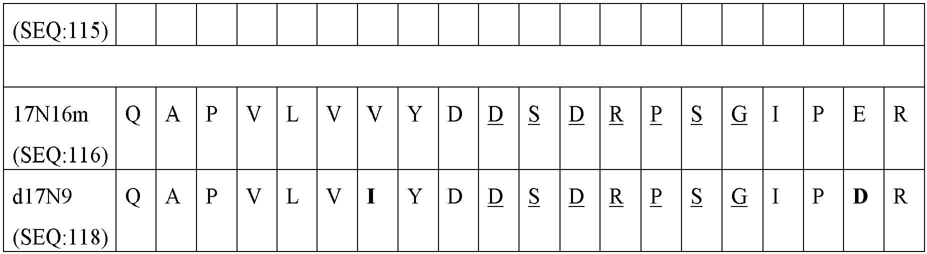

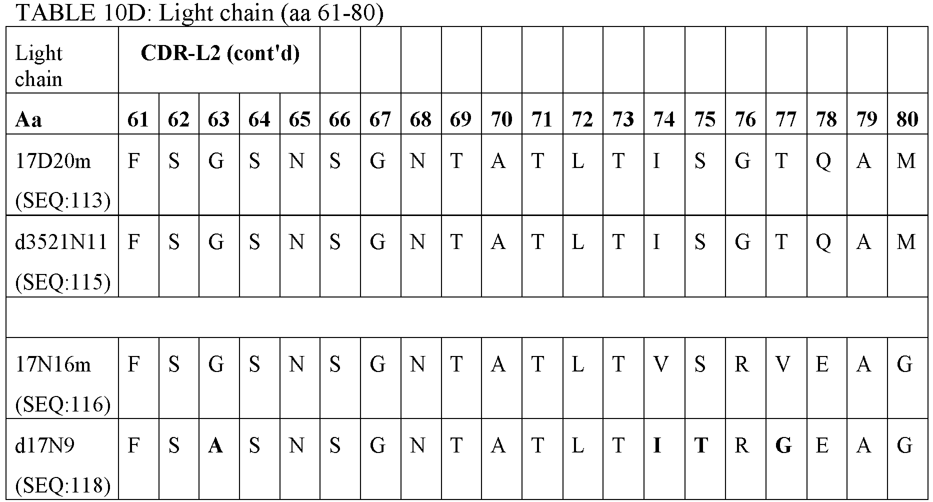

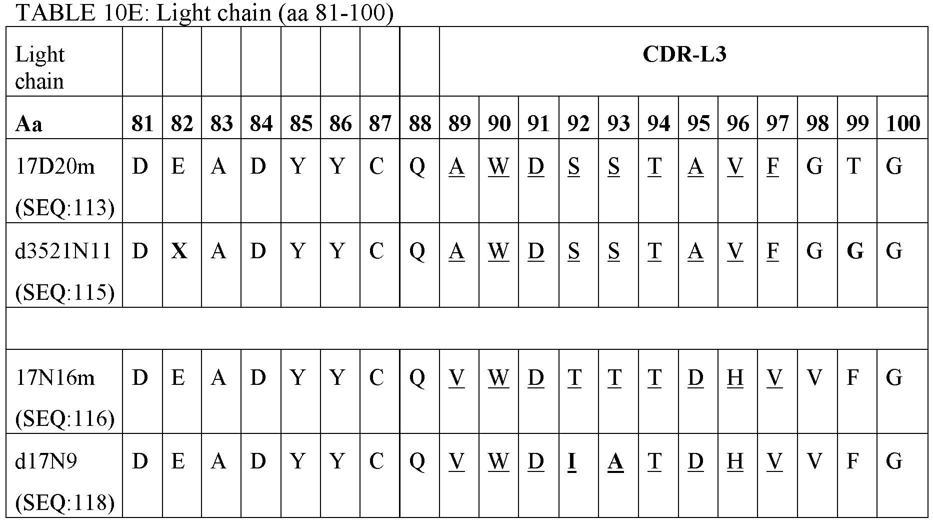

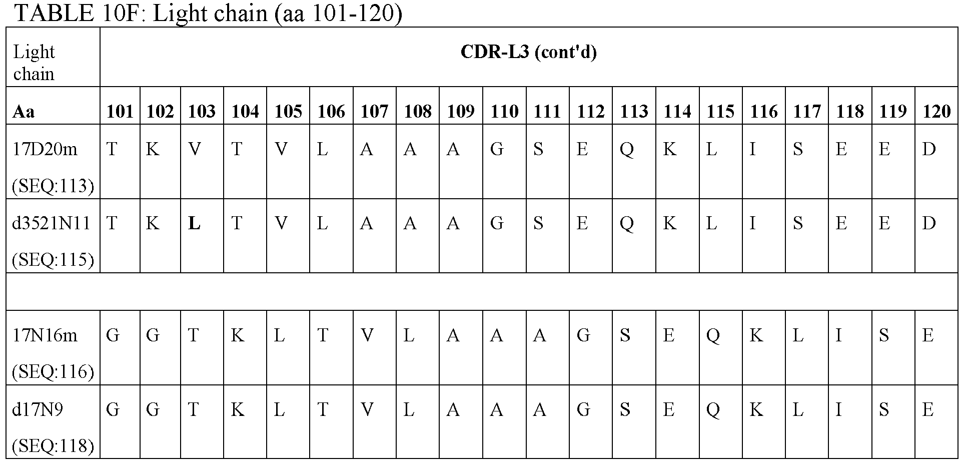

Presented below are the light chain variable region (VL) sequences for the mother clones and daughter clones listed above in TABLE 7 and TABLES 10A-F.

The Kabat CDRs (24-34 (L1); 50-56 (L2); and 89-97 (L3) are bolded; and the Chothia CDRs (24-34 (L1); 50-56 (L2) and 89-97 (L3) are underlined. These regions are the same whether numbered by the Kabat or Chothia system.
17D20m light chain variable region (VL) (SEQ ID NO:113) 
QPVLTQPPSVSVSPGQTASITCSGDKLGDKFAYWYQQKPGHSPVLVIYQ
DNKRPSGIPGRFSGSNSGNTATLTISGTQAMDEADYYCQAWDSSTAVFGTGTKV
TVLA
17D20m_d3521N11 light chain variable region (VL) (SEQ ID NO:115) 
QPVLTQPPSLSVSPGQTASITCSGEKLGDKYAYWYQQKPGQSPVLVMYQ
DKQRPSGIPERFSGSNSGNTATLTISGTQAMDEADYYCQAWDSSTAVFGGGTKL
TVL
17N16m light chain variable region (VL) (SEQ ID NO:116) 
SYVLTQPPSVSVAPGQTARITCGGNNIGSKNVHWYQQKPGQAPVLVVYD
DSDRPSGIPERFSGSNSGNTATLTVSRVEAGDEADYYCQVWDTTTDHVVFGGG
TKLTVLAAAGSEQKLISE
17N16m_d17N9 light chain variable region (VL) (SEQ ID NO:118)

SYELIQPPSVSVAPGQTATITCAGDNLGKKRVHWYQQRPGQAPVLVIYD
DSDRPSGIPDRFSASNSGNTATLTITRGEAGDEADYYCQVWDIATDHVVFGGGT
KLTVLAAAGSEQKLISE

**TABLE 11: Light Chain CDRs (Kabat/chothia)**

| **Reference** | **CDR** | **aa Sequence** | **SEQ ID NO:** |
|---|---|---|---|
| 17D20m | CDR-L1 | GDKLGDKFAYW | 132 |
| d3521N11 | CDR-L1 | GEKLGDKYAYW | 133 |
| 17D20m and d3521N11 consensus | CDR-L1 | GXKLGDKXAYW (wherein X at position 2 is D (Asp) or E (Glu); and wherein X at position 8 is F (Phe) or Y (Tyr) | 147 |
| 17N16m | CDR-L1 | GNNIGSKNVHW | 134 |
| d17N9 | CDR-L1 | GDNLGKKRVHW | 135 |
| 17N16m and d17N9 consensus | CDR-L1 | GXNXGXKXVHW (wherein X at position 2 is N (Asn) or D (Asp); wherein X at position 4 is I (Ile) or L (Leu); wherein X at position 6 is S (Ser) or K (Lys); and wherein X at position 8 is N (Asn) or R (Arg)) | 148 |
| d17N9 | CDR-L1 (aa23-38) | AGDNLGKKRVHWYQQR | 136 |
| NimoAb101 (rat) | CDR-L1 | RASDDIYSNLA | 169 |
| | | | |
| 17D20m | CDR-L2 | DNKRPSG | 137 |
| d3521N11 | CDR-L2 | DKQRPSG | 138 |
| d3521N11 | CDR-L2 (aa50-60) | DKQRPSGIPER | 139 |
| 17N16m | CDR-L2 | DSDRPSG | 140 |
| d17N9 | CDR-L2 | DSDRPSG | 140 |
| 17D20m, | CDR-L2 | DXXRPSG | 149 |
| d3521N11, 17N16m, d17N9 consensus | | (wherein X at position 2 is N (Asn), K (Lys) or S (Ser); and wherein X at position 3 is K (Lys), Q (Gln) or D (Asp)) | |
| d17N9 | CDR-L2 (aa 50-63) | DSDRPSGIPDRFSA | 141 |
| NimoAb101 (rat) | CDR-L2 | DGNRLAD | 170 |
| | | | |
| 17D20m | CDR-L3 | AWDSSTAVF | 142 |
| d3521N11 | CDR-L3 | AWDSSTAVF | 142 |
| d3521N11 | CDR-L3 (aa 89-104) | AWDSSTAVFGGGTKLT | 143 |
| 17N16m | CDR-L3 | VWDTTTDHV | 144 |
| d17N9 | CDR-L3 | VWDIATDHV | 145 |
| 17N16m and d17N9 consensus | CDR-L3 | VWDXXTDHV (wherein X at position 4 is T (Thr) or I (Ile); and wherein X at position 5 is T (Thr) or A (Ala)) | 150 |
| NimoAb101 (rat) | CDR-L3 | QQYNNYPLT | 171 |

### Conversion of candidate clones into IgG4, IgG4/S228P and IgG2 format

The mother and daughter clones were converted into IgG4, IgG4/S228P hinge mutant, and IgG2 format and the functionality of these antibodies were assessed in a C3 assay. The S228P hinge region mutant was included to increase serum stability (see Labrijn A.F. et al., Nature Biotechnology 27:767 (2009)). The sequences of the candidate clones converted to IgG4, IgG4/S228P and IgG2 formats are provided as follows:
SEQ ID NO: 158: cDNA encoding wild-type IgG4
SEQ ID NO: 159: wild-type IgG4 polypeptide
SEQ ID NO: 160: cDNA encoding IgG4 mutant S228P
SEQ ID NO: 161: IgG4 mutant S228P polypeptide
SEQ ID NO: 162: cDNA encoding wild-type IgG2
SEQ ID NO:163: wild-type IgG2 polypeptide

**TABLE 12: Representative MASP-2 antibodies (human)**

| **Antibody Reference #** | **Ig format** | **VH** | **VL** | **Average IC₅₀ value** (C3b deposition assay in 95% human serum) |
|---|---|---|---|---|
| mAb#1 | IgG2 (SEQ ID NO: 163) | SEQ ID NO:112 | SEQ ID NO:118 | 10nM |
| mAb#2 | IgG4 (SEQ ID NO: 159) | SEQ ID NO:112 | SEQ ID NO:118 | 11.9nM |
| mAb#3 | IgG4 (mutant S228P) (SEQ ID NO: 161) | SEQ ID NO:112 | SEQ ID NO:118 | 9.4nM |
| mAb#4 | IgG2 (SEQ ID NO: 163) | SEQ ID NO:111 | SEQ ID NO:115 | 0.9nM |
| mAb#5 | IgG4 (SEQ ID NO: 159) | SEQ ID NO:111 | SEQ ID NO:115 | 2.6nM |
| mAb#6 | IgG4 mutant (S228P) (SEQ ID NO: 161) | SEQ ID NO:111 | SEQ ID NO:115 | 1.5nM |

### Epitope Mapping

The MASP-2 antibodies OMS100 and mAb#6, which have both been demonstrated to bind to human MASP-2 with high affinity and have the ability to block functional complement activity, were analyzed with regard to epitope binding by dot blot analysis as described in WO2012/151481. The results showed that mAb#6 and OMS100 antibodies are highly specific for MASP-2 and do not bind to MASP-1 or MASP-3. Neither antibody bound to MAp19 nor to MASP-2 fragments that did not contain the CCP1 domain of MASP-2, leading to the conclusion that the binding sites encompass CCP 1.

### EXAMPLE 6

This Example describes the identification of rat monoclonal antibodies that bind to MASP-2 and inhibit lectin-mediated complement activation.

### Methods:

As described in WO2004/106384, incorporated herein by reference, monoclonal MASP-2 antibodies were raised by injecting 3µg recombinant human MASP-2 CCP1-CCP2-SP domain into female Wistar rats, fusing spleen cells with mouse myeloma cells, and screening hybridoma supernatants for anti-MASP-2 antibodies. Inhibitory antibodies were identified by screening for inhibition of MASP-2-catalyzed C4 deposition. A representative MASP-2 inhibitory monoclonal antibody, capable of inhibiting C4 deposition in full human serum (IC₅₀=0.0318 ng/µl vs. Eu/Eumax) was identified that was produced by the hybridoma cell line deposited on May 9, 2003 with the European Collection of Cell Cultures (ECACC), Salisbury Wiltshire, United Kingdom, under the accession number 03050904, which produces the antibody NimoAb101 (also referred to as "4A8"). Epitope mapping by Western blot indicated that NimoAb101 recognizes a linear epitope on the B-chain of MASP-2. The sequences of the VH and VL regions of the antibody NimoAb101 are provided below.
NimoAb101: Heavy chain variable region (rat) (SEQ ID NO:164) 
MSFSNTLVFLLFLLKGILCEVQLVESGGGLVQPGRSLKLSCLVSGFTFSNF
GMNWIRQAPGKGLEWVASISSGGTYIYHADTLKGRFTISRENAKNTLYLQMTSL
RSEDTALYYCARGPYHSRYIPYLMDAWGQGASVTVSSAETTAPSVYPLAPGTAL
 
KSNSMVTLGCLVKGYFPEPVTVTWNSGALSSGVHTFPAVLQSGLYTLTSSVTVPS
STWPSQTVTCNVAHPASSTKVDKKIV
NimoAb101: Light chain variable region (rat) (SEQ ID NO:165)

MGVPTQLLGLLLLWITDAICDIQMTQSPGSLCASLGETVTIECRASDDIYS
NLAWYQQKPGNSPQLLIFDGNRLADGVPSRFSGSGSGTQYSLKMKSLQFEDVAS
YFCQQYNNYPLTFGSGTKLEIKRADAAPTVSIFPPSMEQLTSGGATVVCFVNNFY
PRDISVKWKIDGSEQRDGVLDSVTDQDSKDSTYSMSSTLSLTKVEYERHNLYTCE
VVHKTSSSPVVKSFNRNEKGEFQHT

**TABLE 13: CDRs from NimoAb 101**

| **Reference** | **CDR** | **Amino acid** | **SEQ ID NO:** |
|---|---|---|---|
| NimoAb101 | CDR-H1 | GFTFSNFGM | SEQ ID NO:166 |
| NimoAb101 | CDR-H2 | SISSGGTYI | SEQ ID NO:167 |
| NimoAb101 | CDR-H3 | GPYHSRYIPYLMDA | SEQ ID NO:168 |
| NimoAb101 | CDR-L1 | RASDDIYSNLA | SEQ ID NO:169 |
| NimoAb101 | CDR-L2 | DGNRLAD | SEQ ID NO:170 |
| NimoAb101 | CDR-L3 | QQYNNYPLT | SEQ ID NO:171 |

### EXAMPLE 7

This Example describes the generation of MASP-2 antibodies comprising one or more SGMI inhibitory peptides (e.g. SGMI-1 or SGMI-2) fused onto the amino or carboxy termini of the heavy and/or light chains, or the amino or carboxy termini of the heavy chain variable region and/or the light chain variable region of the human MASP-2 antibody.

### Rationale:

As demonstrated in Example 2, it was determined that the inhibitory functions of the SGMI-1 and SGMI-2 peptides are preserved in the SGMI-Fc proteins. As described in this Example, experiments were carried out to determine whether the SGMI peptides would retain activity when fused to the amino or carboxy termini of the heavy or light chains of a MASP-2 antibody scaffold, such as a fully human MASP-2 inhibitory antibody, and to further determine whether the resulting MASP-2 antibody/SGMI peptide fusions would have enhanced inhibitory activity as compared to the naked (non-SGMI containing) MASP-2 scaffold antibody.

FIGURE 16 illustrates an exemplary MASP-2 antibody scaffold comprising one or more SGMI peptides fused to the N-terminus of the heavy chain variable region (A); and/or the N-terminus of the light chain variable region (B); and/or the C-terminus of the heavy chain constant region (C); and/or the C-terminus of the light chain constant region (D).

FIGURE 17A illustrates a MASP-2 scFv antibody scaffold comprising an SGMI peptide fused to the N-terminus of the heavy chain variable region and/or to the C-terminus of the light chain variable region. FIGURE 17B illustrates a MASP-2 scFV antibody scaffold comprising an SGMI peptide fused to the N-terminus of the light chain variable region and/or to the C-terminus of the heavy chain variable region.

### Methods:

To examine the inhibitory activity of the SGMI peptides when fused to a MASP-2 antibody scaffold, eight expression constructs were generated, as illustrated in FIGURE 18, to encode SGMI-1 or SGMI-2 fused either to the N- or C-terminus of the heavy or light chain of a representative MASP-2 inhibitory antibody mAb#6, which was generated as described in Example 5.

**TABLE 14: MASP-2 antibody/SGMI fusions**

| Antibody reference | Peptide Location on Antibody | | | | SEQ ID NO: |
|---|---|---|---|---|---|
| | H-N | H-C | L-N | L-C | |
| HL-M2 (naked MASP-2 mab#6) | - | - | - | - | 111 +115 |
| H-DT40-Ab-SGMI-1-N | SGMI-1 | - | - | - | 190 |
| L-DT40-Ab-SGMI-1-C | - | - | - | SGMI-1 | 191 |
| H-M2-SGMI-1-N | SGMI-1 | - | - | - | 175 |
| H-M2-SGMI-1-C | - | SGMI-1 | - | - | 179 |
| L-M2-SGMI-1-N | - | - | SGMI-1 | - | 183 |
| L-M2-SGMI-1-C | - | - | - | SGMI-1 | 187 |
| H-M2-SGMI-2-N | SGMI-2 | - | - | - | 177 |
| H-M2-SGMI-2-C | - | SGMI-2 | - | - | 181 |
| L-M2-SGMI-2-N | - | - | SGMI-2 | - | 185 |
| L-M2-SGMI-2-C | - | - | - | SGMI-2 | 189 |
| HL-M2-SGMI-1-1-NN | SGMI-1 | - | SGMI-1 | - | 175+183 |
| HL-M2-S GMI-1-1-NC | SGMI-1 | - | - | SGMI-1 | 175+187 |
| HL-M2-SGMI-1-1-CN | - | SGMI-1 | SGMI-1 | - | 179+183 |
| HL-M2-S GMI-1-1-CC | - | SGMI-1 | - | SGMI-1 | 179 + 187 |
| HL-M2-SGMI-2-2-NN | SGMI-2 | - | SGMI-2 | - | 177 + 185 |
| HL-M2-SGMI-2-2-NC | SGMI-2 | - | - | SGMI-2 | 177 + 189 |
| HL-M2-SGMI-2-2-CN | - | SGMI-2 | SGMI-2 | - | 181 + 185 |
| HL-M2-SGMI-2-2-CC | - | SGMI-2 | - | SGMI-2 | 181 + 189 |
| HL-M2-SGMI-1-2-NN | SGMI-1 | - | SGMI-2 | - | 175 + 185 |
| HL-M2-SGMI-1-2-NC | SGMI-1 | - | - | SGMI-2 | 175 + 189 |
| HL-M2-SGMI-1-2-CN | - | SGMI-1 | SGMI-2 | - | 179 + 185 |
| HL-M2-SGMI-1-2-CC | - | SGMI-1 | - | SGMI-2 | 179 + 189 |
| HL-M2-SGMI-2-1-NN | SGMI-2 | - | SGMI-1 | - | 177 + 183 |
| HL-M2-SGMI-2-1-NC | SGMI-2 | - | - | SGMI-1 | 177 + 187 |
| HL-M2-SGMI-2-1-CN | - | SGMI-2 | SGMI-1 | - | 181 + 183 |
| HL-M2-SGMI-2-1-CC | - | SGMI-2 | - | SGMI-1 | 181 + 187 |

| | | | | | |
|---|---|---|---|---|---|
| Abbreviations in Table 14: "H-N"= amino terminus of heavy chain "H-C"=carboxyl terminus of heavy chain "L-N"=amino terminus of light chain "L-C"=carboxyl terminus of light chain "M2"=MASP-2 ab scaffold (representative mAb#6) | | | | | |

For the N-terminal fusions shown in TABLE 14, a peptide linker ('GTGGGSGSSS' SEQ ID NO: 172) was added between the SGMI peptide and the variable region.

For the C-terminal fusions shown in TABLE 14, a peptide linker ('AAGGSG' SEQ ID NO: 173) was added between the constant region and the SGMI peptide, and a second peptide "GSGA" was added at the C-terminal end of the fusion polypeptide to protect C-terminal SGMI peptides from degradation. These fusion constructs are illustrated schematically in FIGURES 16 and 18.

Amino acid sequences are provided below for the following representative MASP-2 antibody/SGMI fusions:
H-M2ab6-SGMI-1-N: (SEQ ID NO; 175, encoded by SEQ ID NO: 174): 
LEVTCEPGTTFKDKCNTCRCGSDGKSAFCTRKLCYQGTGGGSGSSSQVTLKESGP
VLVKPTETLTLTCTVSGFSLSRGKMGVSWIRQPPGKALEWLAHIFSSDEKSYRTS
LKSRLTISKDTSKNQVVLTMTNMDPVDTATYYCARIRRGGIDYWGQGTLVTVSS
ASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCP
APEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISK
 
AKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLG
K
 [491 aa protein, aa 1-36=SGMI-1, aa37-46=linker; aa47-164=heavy chain variable region of MASP-2 ab#6; aa165-491=IgG4 constant region with hinge mutation].
H-M2ab6-SGMI-2-N (SEQ ID NO: 177, encoded by SEQ ID NO: 176): 
LEVTCEPGTTFKDKCNTCRCGSDGKSAVCTKLWCNQGTGGGSGSSSQVTLKESG
PVLVKPTETLTLTCTVSGFSLSRGKMGVSWIRQPPGKALEWLAHIFSSDEKSYRT
SLKSRLTISKDTSKNQVVLTMTNMDPVDTATYYCARIRRGGIDYWGQGTLVTVS
SASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCP
APEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLG
K
 [491 aa protein, aa 1-36=SGMI-2 (underlined), aa37-46=linker (italicized); aa47-164=heavy chain variable region of MASP-2 ab#6 (underlined); aa165-491=IgG4 constant region with hinge mutation.]
H- M2ab6-SGMI-1-C (SEQ ID NO:179, encoded by SEQ ID NO:178): 
QVTLKESGPVLVKPTETLTLTCTVSGFSLSRGKMGVSWIRQPPGKALEWLAHIFS
SDEKSYRTSLKSRLTISKDTSKNQVVLTMTNMDPVDTATYYCARIRRGGIDYWG
QGTLVTVSSASTKGPSVFPLAPCSRSTSESTAALGCLVKDYFPEPVTVSWNSGAL
TSGVHTFPAVLQSSGLYSLSSVVTVPSSSLGTKTYTCNVDHKPSNTKVDKRVESK
YGPPCPPCPAPEFLGGPSVFLFPPKPKDTLMISRTPEVTCVVVDVSQEDPEVQFNW
YVDGVEVHNAKTKPREEQFNSTYRVVSVLTVLHQDWLNGKEYKCKVSNKGLPS
SIEKTISKAKGQPREPQVYTLPPSQEEMTKNQVSLTCLVKGFYPSDIAVEWESNG
QPENNYKTTPPVLDSDGSFFLYSRLTVDKSRWQEGNVFSCSVMHEALHNHYTQK
SLSLSLGKAAGGSGLEVTCEPGTTFKDKCNTCRCGSDGKSAFCTRKLCYQGSGA
 [491aa protein, aa1-118=heavy chain variable region of MASP-2 ab#6 (underlined); aa 119-445=IgG4 constant region with hinge mutation; aa 446-451= 1^{st} linker (italicized); aa 452-487=SGMI-1; aa488-491=2^{nd} linker (italicized).]
H- M2ab6-SGMI-2-C (SEQ ID NO:181, encoded by SEQ ID NO:180): 
QVTLKESGPVLVKPTETLTLTCTVSGFSLSRGKMGVSWIRQPPGKALEWLAHIFS
SDEKSYRTSLKSRLTISKDTSKNQVVLTMTNMDPVDTATYYCARIRRGGIDYWG
QGTLVTVSSASTKGPSVFPLAPCSRSTSESTAALGCLVKDYFPEPVTVSWNSGAL
TSGVHTFPAVLQSSGLYSLSSVVTVPSSSLGTKTYTCNVDHKPSNTKVDKRVESK
YGPPCPPCPAPEFLGGPSVFLFPPKPKDTLMISRTPEVTCVVVDVSQEDPEVQFNW
YVDGVEVHNAKTKPREEQFNSTYRVVSVLTVLHQDWLNGKEYKCKVSNKGLPS
SIEKTISKAKGQPREPQVYTLPPSQEEMTKNQVSLTCLVKGFYPSDIAVEWESNG
QPENNYKTTPPVLDSDGSFFLYSRLTVDKSRWQEGNVFSCSVMHEALHNHYTQK
SLSLSLGKAAGGSGLEVTCEPGTTFKDKCNTCRCGSDGKSAVCTKLWCNQGSGA
 [491aa protein, aa1-118=heavy chain variable region of MASP-2 ab#6 (underlined); aa 119-445=IgG4 constant region with hinge mutation; aa 446-451= 1^{st} linker (italicized); aa 452-487=SGMI-2; aa488-491=2^{nd} linker (italicized).]
L-M2ab6-SGMI-1-N (SEQ ID NO:183, encoded by SEQ ID NO:182): 
LEVTCEPGTTFKDKCNTCRCGSDGKSAFCTRKLCYQGTGGGSGSSSQPVLTQPPS
LSVSPGQTASITCSGEKLGDKYAYWYQQKPGQSPVLVMYQDKQRPSGIPERFSG
SNSGNTATLTISGTQAMDEADYYCQAWDSSTAVFGGGTKLTVLGQPKAAPSVTL
FPPSSEELQANKATLVCLISDFYPGAVTVAWKADSSPVKAGVETTTPSKQSNNKY
AASSYLSLTPEQWKSHRSYSCQVTHEGSTVEKTVAPTECS
 [258aa protein, aa1-36=SGMI-1 (underlined); aa37-46=linker (italicized); aa47-152=light chain variable region of MASP-2 ab#6 (underlined); aa153-258=human Ig lambda constant region]
L-M2ab6-SGMI-2-N (SEQ ID NO:185, encoded by SEQ ID NO:184): 
LEVTCEPGTTFKDKCNTCRCGSDGKSAVCTKLWCNQGTGGGSGSSSQPVLTQPPS
LSVSPGQTASITCSGEKLGDKYAYWYQQKPGQSPVLVMYQDKQRPSGIPERFSG
SNSGNTATLTISGTQAMDEADYYCQAWDSSTAVFGGGTKLTVLGQPKAAPSVTL
FPPSSEELQANKATLVCLISDFYPGAVTVAWKADSSPVKAGVETTTPSKQSNNKY
AASSYLSLTPEQWKSHRSYSCQVTHEGSTVEKTVAPTECS
 [258aa protein, aa1-36=SGMI-2 (underlined); aa37-46=linker (italicized); aa47-152=light chain variable region of MASP-2 ab#6 (underlined); aa153-258=human Ig lambda constant region]
L-M2ab6-SGMI-1-C (SEQ ID NO:187, encoded by SEQ ID NO:186): 
QPVLTQPPSLSVSPGQTASITCSGEKLGDKYAYWYQQKPGQSPVLVMYQDKQRP
SGIPERFSGSNSGNTATLTISGTQAMDEADYYCQAWDSSTAVFGGGTKLTVLGQ
PKAAPSVTLFPPSSEELQANKATLVCLISDFYPGAVTVAWKADSSPVKAGVETTT
PSKQSNNKYAASSYLSLTPEQWKSHRSYSCQVTHEGSTVEKTVAPTECSAAGGSG
LEVTCEPGTTFKDKCNTCRCGSDGKSAFCTRKLCYQGSGA
 [258aa protein, aa1-106=light chain variable region of MASP-2 ab#6 (underlined); aa 107-212=human Ig lambda constant region; aa 213-218=1^{st} linker; aa219-254=SGMI-1; aa255-258=2^{nd} linker]
L-M2ab6-SGMI-2-C (SEQ ID NO:189, encoded by SEQ ID NO:188): 
QPVLTQPPSLSVSPGQTASITCSGEKLGDKYAYWYQQKPGQSPVLVMYQDKQRP
SGIPERFSGSNSGNTATLTISGTQAMDEADYYCQAWDSSTAVFGGGTKLTVLGQ
PKAAPSVTLFPPSSEELQANKATLVCLISDFYPGAVTVAWKADSSPVKAGVETTT
PSKQSNNKYAASSYLSLTPEQWKSHRSYSCQVTHEGSTVEKTVAPTECSAAGGSG
LEVTCEPGTTFKDKCNTCRCGSDGKSAVCTKLWCNQGSGA
 [258aa protein, aa1-106=light chain variable region of MASP-2 ab#6 (underlined); aa 107-212=human Ig lambda constant region; aa 213-218=1^{st} linker; aa219-254=SGMI-2; aa255-258=2^{nd} linker]

### Functional Assays:

The eight MASP-2-SGMI fusion antibody constructs were transiently expressed in Expi293F cells (Invitrogen), purified by Protein A affinity chromatography, and tested in 10% normal human serum for inhibition of C3b deposition in a mannan-coated bead assay as described below. (Note: the H-M2-SGMI-1-C construct did not express well, so it could not be tested in this assay).

### Experiment #1: Testing the MASP-2-SGMI fusions in the mannan-coated bead assay for C3b deposition

The MASP-2-SGMI fusion antibodies assessed for lectin pathway inhibition in an assay of C3b deposition on mannan-coated beads. This assay, which determines degree of activity by flow cytometry, offers greater resolution than the Wieslab^{®} assay. The lectin pathway bead assay was carried out as follows: mannan was adsorbed to 7 µM-diameter polystyrene beads (Bangs Laboratories; Fishers, IN, USA) overnight at 4°C in carbonate-bicarbonate buffer (pH 9.6). The beads were washed in PBS and exposed to 10% human serum, or 10% serum pre-incubated with antibodies or inhibitors. The serum-bead mixture was incubated at room temperature for one hour while agitating. Following the serum incubation, the beads were washed, and C3b deposition on the beads was measured by detection with an anti-C3c rabbit polyclonal antibody (Dako North America; Carpinteria, CA, USA) and a PE-Cy5 conjugated goat anti-rabbit secondary antibody (Southern Biotech; Birmingham, AL, USA). Following the staining procedure, the beads were analyzed using a FACSCalibur flow cytometer. The beads were gated as a uniform population using forward and side scatter, and C3b deposition was apparent as FL3-positive particles (FL-3, or "FL-3 channel" indicates the 3rd or red channel on the cytometer). The Geometric Mean Fluorescence Intensity (MFI) for the population for each experimental condition was plotted relative to the antibody/inhibitor concentration to evaluate lectin pathway inhibition.

The **IC₅₀** values were calculated using the GraphPad PRISM software. Specifically, **IC₅₀** values were obtained by applying a variable slope (four parameter), nonlinear fit to log (antibody) versus mean fluorescence intensity curves obtained from the cytometric assay.

The results are shown in TABLE 15.

**TABLE 15: C3b deposition (mannan-coated bead assay) in 10% human serum Experiment #1)**

| **Construct** | **IC₅₀ (nM)** |
|---|---|
| Naked N2 ab (mAb#6) | ≥ 3.63 nM |
| H-M2-SGMI-2-N | 2.11 nM |
| L-M2-SGMI-2-C | 1.99 nM |
| H-M2-SGMI-1-N | 1.09 nM |
| H-M2-SGMI-2-N | 2.24 nM |
| L-M2-SGMI-1-C | 1.09 nM |
| L-M2-SGMI-1-N | 4.00 nM |
| L-M2-SGMI-2-N | 3.71 nM |

### Results:

The control, non-SGMI-containing MASP-2 "naked" scaffold antibody (mAb#6), was inhibitory in this assay, with an IC50 value of ≥ 3.63 nM, which is consistent with the inhibitory results observed in Example 5. Remarkably, as shown in TABLE 15, all seven of the SGMI-MASP-2 antibody fusions that were tested improved the potency of the MASP-2 scaffold antibody in this assay. The two most potent fusions, L-M2-SGMI-1C and H-M2-SGMI-1-N, both contain the MASP-1 specific SGMI-1 peptide (SEQ ID NO:6), suggesting that the dual MASP-1/MASP-2 specificity may be advantageous in the inhibition of C3b deposition. It is also noted that two of the MASP-2-SGMI fusion antibodies bearing the MASP-2 specific SGMI-2 peptide (SEQ ID NO:9), H-M2-SGMI-2-N and H-M2-SGMI-2-C, are nearly as active, suggesting that increased valency may also be beneficial in the inhibition of C3b deposition.

### Experiment #2: Mannan-coated bead assay for C3b deposition

A mannan-coated bead assay for C3b deposition was carried out with 10% human serum to assess the relative contributions of the two components of the MASP-2-SGMI fusions, the MASP-2 antibody scaffold and the SGMI peptides, to the improved inhibitory activity. The two most potent fusions were used in this analysis. For each, the MASP-2 antibody scaffold (mAb#6) was compared to the MASP-2-SGMI fusion versus the corresponding chimeric DT40 antibody-SGMI fusion, generated from a non-specific DT40 chicken antibody (does not bind to MASP-2), and the non-specific chicken antibody with an SGMI-1 peptide fused to the N-terminal region of the heavy chain (H-DT40-Ab-SGMI-1-N, set forth as SEQ ID NO: 190), and the non-specific chicken antibody with an SGMI-1 peptide fused to the C-terminal region of the light chain (L-DT40-Ab-SGMI-1-C, set forth as SEQ ID NO:100). The assay was carried out as described above, and the results are shown below in TABLE 16.

**TABLE 16: C3b deposition carried out with 10% human serum (Experiment #2**

| **Construct** | **IC₅₀ (nM)** |
|---|---|
| HL-M2 Naked MASP-2 ab (mAb#6) | 0.45 nM |
| H-M2-SGMI-1-N | 0.38 nM |
| H-DT40-Ab-SGMI-1-N | 32.03 nM |
| L-M2-S GMI-1-C | 0.33 nM |
| L-DT40-Ab-SGMI-1-C | ND |

As shown in TABLE 16, in both cases, the SGMI-1 peptide improved the activity of the MASP-2 scaffold antibody. Of the two fusions, the most striking improvement in activity was observed with SGMI-1 fused to the C-terminus of the MASP-2 antibody light chain (L-M2-SGMI-1-C, with an **IC₅₀** of 0.33 nM). In contrast, when the SGMI-1 peptide is fused to the same position of the non-specific chicken chimeric mAb (L-DT40-AB-SGMI-1-C), its inhibitory activity was barely detectable. When SGMI-1 is fused to the N-terminus of the non-specific chicken mAb heavy chain (H-DT40-Ab-SGMI-1-N), it was considerably more active than L-DT40-AB-SGMI-1-C, but moving it to the same position on the MASP-2 antibody scaffold (H-M2-SGMI-1-N) still increases its inhibitory activity by nearly two orders of magnitude (IC₅₀ of 32 nM versus 0.38 nM). Together, these data indicate that, when fused into a single antibody molecule, the MASP-2 antibody scaffold and SGMI synergize to block activation of the lectin pathway.

### Experiment #3: C3b deposition assay with 10% mouse serum

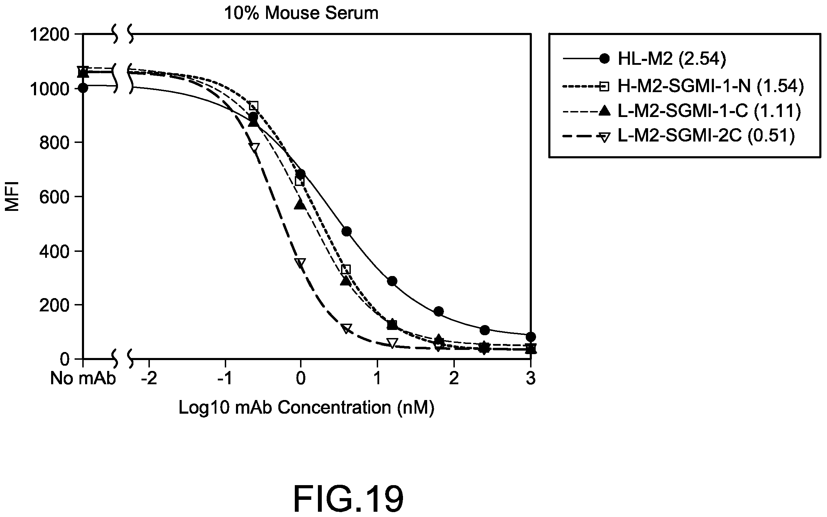

A mannan-coated bead assay for C3b deposition was carried out as described above with 10% mouse serum. FIGURE 19 graphically illustrates the results of a C3b deposition assay carried out in 10% mouse serum with a representative naked MASP-2 (HL-M2) scaffold antibody (mAb#6) in comparison to MASP-2 antibody (M2)-SGMI fusions comprising SGMI-1 or SGMI-2 over a range of antibody concentrations. As shown in FIGURE 19, fusion of either SGMI-1 or SGMI-2 to the MASP-2 scaffold antibody increased the MASP-2 antibody inhibitory potency under these conditions.

### Experiment #4: C4b deposition assay with 10% human serum

A C4b deposition assay was carried out with 10% human serum using the same assay conditions as described above for the C3b deposition assay with the following modifications. C4b detection and flow cytometric analysis was carried out by staining the deposition reaction with an anti-C4b mouse monoclonal antibody (1:500, Quidel) and staining with a secondary goat anti-mouse F(ab')2 conjugated to PE Cy5 (1:200, Southern Biotech) prior to flow cytometric analysis.

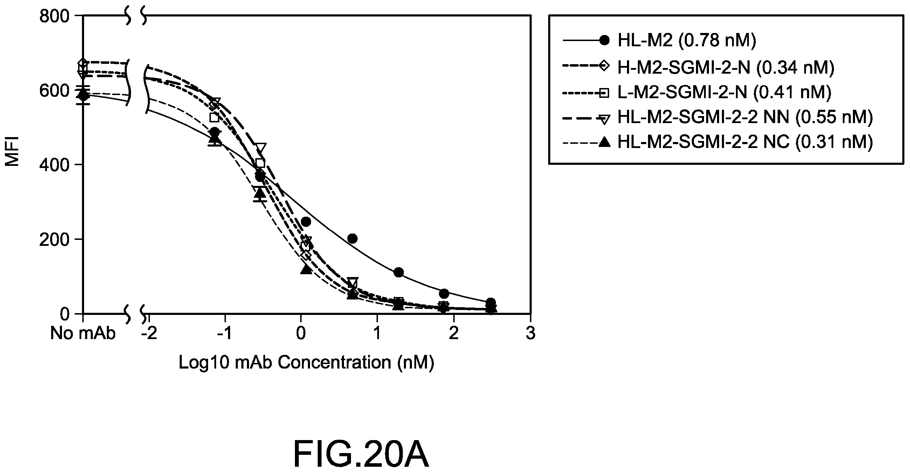

As shown in FIGURE 20A, the single SGMI-2-bearing MASP-2 antibody fusions (H-M2-SGMI-2-N, L-M2-SGMI-2-N), and the double SGMI-2-bearing MASP-2 antibody fusions (HL-M2-SGMI-2-2-NN and HL-M2-SGMI-2-2-NC) all had increased potency as compared to the MASP-2 scaffold antibody (HL-M2).

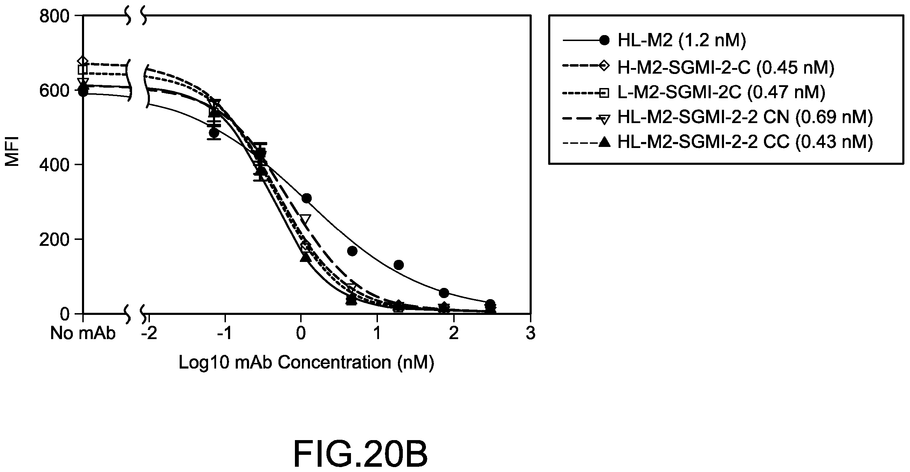

Similarly, as shown in FIGURE 20B, the single SGMI-2 bearing MASP-2 antibody fusions (H-M2-SGMI-2-C and L-M2-SGMI-2C) and the double SGMI-2 bearing MASP-2 antibody fusions (HL-M2-SGMI-2-2-CN and HL-M2-SGMI-2-2-CC) all had increased potency as compared to the MASP-2 scaffold antibody (HL-M2).

### Experiment #5: C3b deposition assay with 10% human serum

A C3b deposition assay was done in 10% human serum to assess the relative contributions of the two components of the fusion antibodies, the MASP-2 antibody scaffold (mAb#6) or the SGMI peptides, to the improved inhibitory activity. Two representative fusions that were previously determined to have high potency, namely H-M2-SGMI-1-N and L-M2-SGMI-1-C were used for this analysis. Each fusion construct was compared to the corresponding chimeric DT40 antibody-SGMI-fusion (generated as described above in Example 4).

FIGURE 21 graphically illustrates the results of a C3b deposition assay carried out in 10% human serum with a non-specific chimeric chicken/human antibody comprising the SGMI-1 peptide fused to the C-terminus of the light chain constant region (L-SGMI-1-C); or a non-specific chimeric/human antibody comprising the SGMI-1 peptide fused to the N-terminus of the heavy chain variable region (H-SGMI-1-N) in comparison to the counterpart MASP-2 antibody (M2)-SGMI-1 fusions. As shown in FIGURE 21, the most striking improvement in activity was achieved with SGMI-1 fused to the C-terminus of the light chain of the MASP-2 scaffold antibody (L-M2-SGMI-1-C, with an IC50 of 0.33 nM), whereas when the SGMI-1 peptide is fused to the C-terminus of the non-specific chimeric DT40 antibody (L-SGMI-1-C), its inhibitory activity is barely detectable. As further shown in FIGURE 21, when SGMI-1 is fused to the N-terminus of the non-specific chimeric DT40 antibody (H-SGMI-1-N), it had some inhibitory activity (IC50 of 30 nM), however, a striking improvement of nearly two orders of magnitude in inhibitory activity was achieved when SGMI-1 was fused to the same N-terminal position on the MASP-2 scaffold antibody (H-M2-SGMI-1-N, IC50 of 0.38 nM). In conclusion, in both cases, SGMI-1 dramatically improved the inhibitory activity of the MASP-2 scaffold antibody. Together, these data indicate that, when fused into a single antibody molecule, a MASP-2 scaffold antibody and the bioactive peptide SGMI-1 synergize to block activation of the lectin pathway.

### Alternative Cases:

In view of the disclosure herein, it will be understood by one of skill in the art that the use of the MASP-2 antibody mAb#6 described in this example is a representative MASP-2 scaffold antibody, and an SGMI peptide can be fused to any MASP-2 antibody, such as MASP-2 antibodies described in TABLE 17, using routine methods known in the art to generate a MASP-2 antibody/SGMI fusion in accordance with the invention. In some cases, the MASP-2 scaffold antibody without the SGMI peptide may have weak or no inhibitory activity, and the SGMI bearing MASP-2 antibody fusion provides, or increases, the inhibitory activity of the MASP-2 scaffold antibody. In some cases, the MASP-2 scaffold antibody has an initial level of inhibitory activity, and the SGMI bearing MASP-2 antibody fusion has enhanced inhibitory activity. Non-limiting examples of suitable MASP-2 inhibitory antibody heavy chain variable regions and light chain variable regions are provided in TABLES 18 and 19, respectively.

**TABLE 17: MASP-2 SPECIFIC ANTIBODIES FROM THE LITERATURE**

| ANTIGEN | ANTIBODY TYPE | REFERENCE |
|---|---|---|
| Recombinant human CCP1/2-SP fragment (MoAb 8B5) | Rat MoAb (subclass IgG1) | Moller-Kristensen, M., et al., J. of Immunol. Methods 282:159-167, 2003 |
| Recombinant human MAp 19 (MoAb 6G12) (cross reacts with MASP-2) | Rat MoAb (subclass IgG1) | Moller-Kristensen, M., et al., J. of Immunol. Methods 282:159-167, 2003 |
| hMASP-2 | Mouse MoAb (S/P) | Peterson, S.V., et al., Mol. Immunol. 35:409, April 1998 |
| | Mouse MoAb (N-term) | |
| hMASP-2 (CCP1-CCP2-SP domain | rat MoAb: Nimoab 101, produced by hybridoma cell line 03050904 (ECACC) | WO 2004/106384 |
| hMASP-2 (full length-his tagged) | murine MoAbs: | WO 2004/106384 |
| | NimoAb104, produced by hybridoma cell line M0545YM035 (DSMZ) | |
| | NimoAb108, produced by hybridoma cell line M0545YM029 (DSMZ) | |
| | NimoAb 109 produced by hybridoma cell line M0545YM046 (DSMZ) | |
| | NimoAb 110 produced by hybridoma cell line M0545YM048 (DSMZ) | |

**TABLE 18: MASP-2 inhibitory antibody heavy chain variable regions**

| **Antibody Reference** | **Heavy chain variable region** |
|---|---|
| 17D20 | SEQ ID NO:109 |
| d3521N11 | SEQ ID NO:111 |
| 18L16 | aa 1-120 of SEQ ID NO: 152 |
| 4D9 | aa 1-120 of SEQ ID NO:153 |
| 17L20 | aa 1-120 of SEQ ID NO:154 |
| 17N16 | SEQ ID NO:112 |
| 3F22 | aa 1-120 of SEQ ID NO:156 |
| 9P13 | aa 1-120 of SEQ ID NO:157 |
| NimoAb 101 | SEQ ID NO:164 |

**TABLE 19: MASP-2 inhibitory antibody light chain variable regions**

| **Antibody Reference** | **Light chain variable region** |
|---|---|
| 17D20 | SEQ ID NO:113 |
| d3521N11 | SEQ ID NO:115 |
| 18L16 | aa 146-250 of SEQ ID NO:152 |
| 4D9 | aa 146-250 of SEQ ID NO:153 |
| 17L20 | aa 146-250 of SEQ ID NO:153 |
| 17N16 | SEQ ID NO:116 |
| d 17N9 | SEQ ID NO:118 |
| 3F22 | aa 146-250 of SEQ ID NO:156 |
| 9P13 | aa 146-250 of SEQ ID NO:157 |
| NimoAb101 (4A8) | SEQ ID NO:165 |

### EXAMPLE 8

This Example describes a mouse pharmacodynamics study that was carried out to evaluate the inhibitory activity of SGMI-1 and SGMI-2 bearing MASP-2 antibody fusions *in vivo.*

### B ackground/Rati onal e:

As described in Example 7, it has been determined that bioactive peptide inhibitors of MASP-1 and MASP-2, SGMI-1 and SGMI-2, respectively, significantly enhance the capacity of a MASP-2 scaffold antibody to block activation of the lectin pathway in human and mouse sera. The following experiment was carried out to evaluate the inhibitory activity of SGMI-1 and SGMI-2 bearing MASP-2 antibody fusions in a seven day mouse pharmacodynamics study.

### Methods:

Mice (n=3/group) were injected intraperitoneally with 5 mg/kg of either a MASP-2 scaffold antibody (mAb#6), SGMI-1 peptide-bearing MASP-2 antibodies (L-M2-SGMI-1-C; L-M2-SGMI-1-N or H-M2-SGMI-1-N), SGMI-2 peptide-bearing MASP-2 antibodies (L-M2-SGMI-2-N, H-M2-SGMI-2-C, H-M2-SGMI-2-N or L-M2-SGMI-2-C), or an isotype IgG4 control antibody (ET904).

Serum samples were obtained from the mice at 24, 72 and 168 hours after antibody injection, and lectin pathway activity was measured in a C3b deposition assay using 90% serum. It was observed that two of the fusion antibodies, namely H-M2-SGMI-2-C and H-M2-SGMI-1-N had the highest potency and inhibited lectin pathway activity to nearly undetectable levels by 24 hours after injection and maintained a high level of inhibition for a week.

In order to assay for pharmacokinetics, ELISAs were also run to measure the level of antibody in the serum samples at the same time points used for the pharmacodynamics assay. The concentrations of the antibodies, as determined by ELISA (the pharmacokinetic data set) were then plotted against the lectin pathway activity, as determined by the C3b deposition assay (the pharmacodynamics data set). Pharmacodynamic data from the untreated mice were used to provide common "0 nM antibody" data points for all conditions. Data from all time points were combined for each treatment group.

### Results:

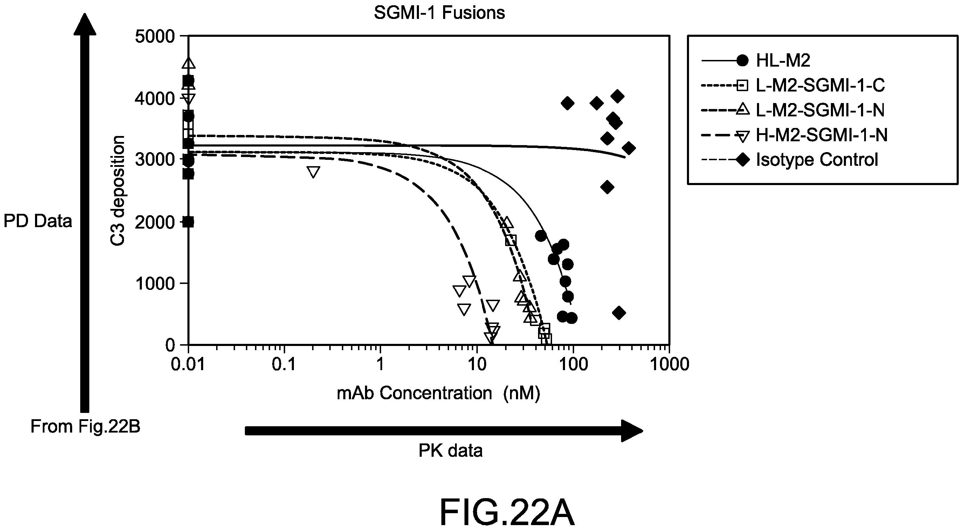

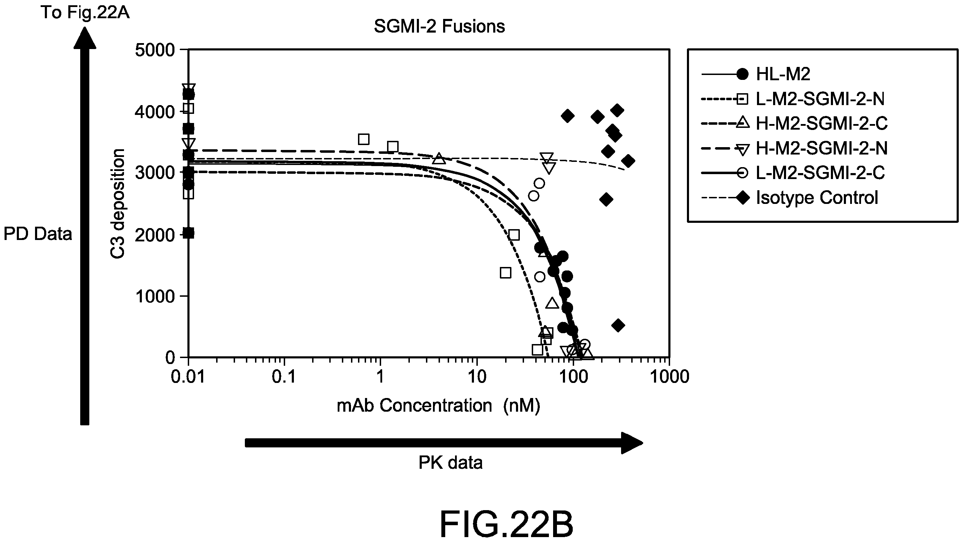

FIGURE 22A shows a plot of the concentration of a representative naked MASP-2 (HL-M2) scaffold antibody (mAb#6) or MASP-2 antibody (M2)-SGMI-1 fusions (pharmacokinetic (PK) data set) versus lectin pathway activity (C3b deposition; pharmacodynamics (PD) data set). FIGURE 22B shows a plot of the concentration of a representative naked MASP-2 (HL-M2) scaffold antibody (mAb#6) or MASP-2 antibody (M2)-SGMI-2 fusions (pharmacokinetic (PK) data set) versus lectin pathway activity (C3b deposition; pharmacodynamics (PD) data set).

As shown in FIGURE 22A, the SGMI-1 peptide significantly enhances the capacity of a MASP-2 scaffold antibody to block activation of the lectin pathway in a mouse *in vivo.* As further shown in FIGURE 22B, the SGMI-2 peptide also enhances the capacity of a MASP-2 scaffold antibody to block activation of the lectin pathway in a mouse *in vivo,* but to a lesser extent that the SGMI-1 peptide. These results suggest that the dual MASP-1/MASP-2 specificity afforded by the antibody-peptide fusion may be advantageous in the inhibition of the lectin pathway *in vivo.* It is noted that improved results with the MASP-2-SGMI fusion antibodies bearing the MASP-2 specific SGMI-2 peptide suggest that increased valency may also be beneficial in the inhibition of the lectin pathway *in vivo.*

While the preferred cases of the disclosure have been illustrated and described, it will be appreciated that various changes can be made therein.

## Claims

1. An isolated bioactive peptide-bearing antibody, or antigen-binding fragment thereof, comprising:
(i) a heavy chain variable region and a light chain variable region each comprising three CDRs that specifically bind to human MASP-2, set forth as SEQ ID NO:4, wherein said heavy chain variable region comprises SEQ ID NO: 111 and said light chain variable region comprises SEQ ID NO: 115; and
(ii) an SGMI peptide sequence set forth as SEQ ID NO:6 or SEQ ID NO 9,
(a) wherein the SGMI peptide sequence comprising SGMI-1 set forth as SEQ ID NO:6 is fused to the amino terminal region of the heavy chain variable region of the antibody;
(b) wherein the SGMI peptide sequence comprising SGMI-1 set forth as SEQ ID NO:6 is fused to the amino terminal region of the light chain variable region of the antibody;
(c) wherein the SGMI peptide sequence comprising SGMI-1 set forth as SEQ ID NO:6 is fused to the carboxy terminal region of the light chain constant region of the antibody;
(d) wherein the SGMI peptide sequence comprising SGMI-2 set forth as SEQ ID NO:9 is fused to the carboxy terminal region of the heavy chain constant region of the antibody;
(e) wherein the SGMI peptide sequence comprising SGMI-2 set forth as SEQ ID NO:9 is fused to the amino terminal region of the light chain variable region of the antibody; and
(f) wherein the SGMI peptide sequence comprising SGMI-2 set forth as SEQ ID NO:9 is fused to the carboxy terminal region of the light chain constant region of the antibody.

2. The antibody or fragment thereof of claim 1, further comprising a linker region of from 1 amino acid residue to 20 amino acid residues between the SGMI peptide amino acid sequence and the amino terminus of the light or heavy chain variable region of the antibody.

3. The antibody or fragment thereof of claim 1 or 2, further comprising a linker region of from 1 amino acid residue to 20 amino acid residues between the SGMI peptide amino acid sequence and the carboxy terminus of the light or heavy chain variable region of the antibody.

4. The antibody or fragment thereof of claim 1, wherein the antibody is selected from the group consisting of a whole antibody, a single chain antibody, and a univalent antibody lacking a hinge region.

5. The antibody fragment of claim 1, wherein the fragment is selected from the group consisting of a Fab, Fab', F(ab')2, or scFv fragment.

6. A nucleic acid molecule encoding the amino acid sequence of an isolated bioactive peptide-bearing antibody as set forth in any of claims 1 to 5.

7. A cell comprising at least one of the nucleic acid molecules according to claim 6.

8. A pharmaceutical composition comprising the antibody, or antigen-binding fragment thereof, of any one of claims 1 to 5, and a pharmaceutically acceptable excipient.

9. A pharmaceutical composition according to claim 8, formulated for systemic delivery.

10. A pharmaceutical composition according to claim 8, formulated for intra-arterial administration.

11. A pharmaceutical composition according to claim 8, formulated for intravenous administration.

12. A pharmaceutical composition according to claim 8, formulated for intracranial administration.

13. A pharmaceutical composition according to claim 8, formulated for intramuscular administration.

14. A pharmaceutical composition according to claim 8, formulated for inhalational administration.

15. A pharmaceutical composition according to claim 8, formulated for nasal or subcutaneous administration.

## Patentansprüche

1. Isolierter bioaktiver peptidtragender Antikörper oder antigenbindendes Fragment davon, umfassend:
(i) eine variable Region der schweren Kette und eine variable Region der leichten Kette, die jeweils drei CDRs umfassen, die spezifisch an menschliches MASP-2 binden, dargelegt als SEQ ID NO:4, wobei die variable Region der schweren Kette die SEQ ID NO: 111 umfasst und die variable Region der leichten Kette die SEQ ID NO: 115 umfasst; und
(ii) eine SGMI-Peptidsequenz, die als SEQ ID NO:6 oder SEQ ID NO 9 dargelegt ist,
(a) , wobei die SGMI-Peptidsequenz, die SGMI-1 wie als SEQ ID NO:6 dargelegt umfasst, an die aminoterminale Region der variablen Region der schweren Kette des Antikörpers fusioniert ist;
(b) , wobei die SGMI-Peptidsequenz, die SGMI-1 wie als SEQ ID NO:6 dargelegt umfasst, an die aminoterminale Region der variablen Region der leichten Kette des Antikörpers fusioniert ist;
(c) , wobei die SGMI-Peptidsequenz, die SGMI-1 wie als SEQ ID NO:6 dargelegt umfasst, an die carboxyterminale Region der konstanten Region der leichten Kette des Antikörpers fusioniert ist;
(d) , wobei die SGMI-Peptidsequenz, die SGMI-2 wie als SEQ ID NO:9 dargelegt umfasst, an die carboxyterminale Region der konstanten Region der schweren Kette des Antikörpers fusioniert ist;
(e) , wobei die SGMI-Peptidsequenz, die SGMI-2 wie als SEQ ID NO:9 dargelegt umfasst, an die aminoterminale Region der variablen Region der leichten Kette des Antikörpers fusioniert ist; und
(f) , wobei die SGMI-Peptidsequenz, die SGMI-2 wie als SEQ ID NO:9 dargelegt umfasst, an die carboxyterminale Region der konstanten Region der leichten Kette des Antikörpers fusioniert ist.

2. Antikörper oder Fragment davon nach Anspruch 1, ferner umfassend eine Linker-Region von 1 Aminosäurerest bis 20 Aminosäureresten zwischen der SGMI-Peptid-Aminosäuresequenz und dem Aminoterminus der variablen Region der leichten oder schweren Kette des Antikörpers.

3. Antikörper oder Fragment davon nach Anspruch 1 oder 2, ferner umfassend eine Linker-Region von 1 Aminosäurerest bis 20 Aminosäureresten zwischen der SGMI-Peptid-Aminosäuresequenz und dem Carboxyterminus der variablen Region der leichten oder schweren Kette des Antikörpers.

4. Antikörper oder Fragment davon nach Anspruch 1, wobei der Antikörper ausgewählt ist aus der Gruppe bestehend aus einem ganzen Antikörper, einem einzelkettigen Antikörper und einem univalenten Antikörper ohne Gelenksregion.

5. Antikörperfragment nach Anspruch 1, wobei das Fragment ausgewählt ist aus der Gruppe bestehend aus einem Fab-, Fab'-, F(ab')2- oder scFv-Fragment.

6. Nukleinsäuremolekül, das für die Aminosäuresequenz eines isolierten bioaktiven peptidtragenden Antikörpers nach einem der Ansprüche 1 bis 5 kodiert.

7. Zelle, die zumindest eines der Nukleinsäuremoleküle nach Anspruch 6 umfasst.

8. Pharmazeutische Zusammensetzung, umfassend den Antikörper oder das antigenbindende Fragment davon nach einem der Ansprüche 1 bis 5 und einen pharmazeutisch annehmbaren Hilfsstoff.

9. Pharmazeutische Zusammensetzung nach Anspruch 8, die für die systemische Verabreichung formuliert ist.

10. Pharmazeutische Zusammensetzung nach Anspruch 8, die für die intraarterielle Verabreichung formuliert ist.

11. Pharmazeutische Zusammensetzung nach Anspruch 8, die für die intravenöse Verabreichung formuliert ist.

12. Pharmazeutische Zusammensetzung nach Anspruch 8, die für die intrakraniale Verabreichung formuliert ist.

13. Pharmazeutische Zusammensetzung nach Anspruch 8, die für die intramuskuläre Verabreichung formuliert ist.

14. Pharmazeutische Zusammensetzung nach Anspruch 8, die für die inhalatorische Verabreichung formuliert ist.

15. Pharmazeutische Zusammensetzung nach Anspruch 8, die für die nasale oder subkutane Verabreichung formuliert ist.

## Revendications

1. Anticorps porteur de peptide bioactif isolé, ou fragment de liaison à l'antigène de celui-ci, comprenant :
(i) une région variable de chaîne lourde et une région variable de chaîne légère comprenant chacune trois CDR qui se lient spécifiquement à la MASP-2 humaine, représentées comme SEQ ID NO : 4, où ladite région variable de chaîne lourde comprend SEQ ID NO : 111 et ladite région variable de chaîne légère comprend SEQ ID NO : 115 ; et
(ii) une séquence de peptide SGMI représentée comme SEQ ID NO : 6 ou SEQ ID NO 9,
(a) où la séquence de peptide SGMI comprenant SGMI-1 représentée comme SEQ ID NO : 6 est fusionnée à la région amino-terminale de la région variable de chaîne lourde de l'anticorps ;
(b) où la séquence de peptide SGMI comprenant SGMI-1 représentée comme SEQ ID NO : 6 est fusionnée à la région amino-terminale de la région variable de chaîne légère de l'anticorps ;
(c) où la séquence de peptide SGMI comprenant SGMI-1 représentée comme SEQ ID NO : 6 est fusionnée à la région carboxy-terminale de la région constante de chaîne légère de l'anticorps ;
(d) où la séquence de peptide SGMI comprenant SGMI-2 représentée comme SEQ ID NO : 9 est fusionnée à la région carboxy-terminale de la région constante de chaîne lourde de l'anticorps ;
(e) où la séquence de peptide SGMI comprenant SGMI-2 représentée comme SEQ ID NO : 9 est fusionnée à la région amino-terminale de la région variable de chaîne légère de l'anticorps ; et
(f) où la séquence de peptide SGMI comprenant SGMI-2 représentée comme SEQ ID NO : 9 est fusionnée à la région carboxy-terminale de la région constante de chaîne légère de l'anticorps.

2. Anticorps ou fragment de celui-ci de la revendication 1, comprenant en outre une région de liaison de 1 résidu d'acide aminé à 20 résidus d'acide aminé entre la séquence d'acides aminés de peptide SGMI et l'extrémité amino de la région variable de chaîne légère ou lourde de l'anticorps.

3. Anticorps ou fragment de celui-ci de la revendication 1 ou 2, comprenant en outre une région de liaison de 1 résidu d'acide aminé à 20 résidus d'acide aminé entre la séquence d'acides aminés de peptide SGMI et l'extrémité carboxy de la région variable de chaîne légère ou lourde de l'anticorps.

4. Anticorps ou fragment de celui-ci de la revendication 1, dans lequel l'anticorps est choisi dans le groupe consistant en un anticorps entier, un anticorps à chaîne unique et un anticorps univalent dépourvu de région charnière.

5. Fragment d'anticorps de la revendication 1, dans lequel le fragment est choisi dans le groupe consistant en un fragment Fab, Fab', F(ab')2 ou scFv.

6. Molécule d'acide nucléique codant pour la séquence d'acides aminés d'un anticorps porteur de peptide bioactif isolé selon l'une des revendications 1 à 5.

7. Cellule comprenant au moins l'une des molécules d'acide nucléique selon la revendication 6.

8. Composition pharmaceutique comprenant l'anticorps, ou un fragment de liaison à l'antigène de celui-ci, de l'une quelconque des revendications 1 à 5, et un excipient pharmaceutiquement acceptable.

9. Composition pharmaceutique selon la revendication 8, formulée pour une administration systémique.

10. Composition pharmaceutique selon la revendication 8, formulée pour une administration intra-artérielle.

11. Composition pharmaceutique selon la revendication 8, formulée pour une administration intraveineuse.

12. Composition pharmaceutique selon la revendication 8, formulée pour une administration intracrânienne.

13. Composition pharmaceutique selon la revendication 8, formulée pour une administration intramusculaire.

14. Composition pharmaceutique selon la revendication 8, formulée pour une administration par inhalation.

15. Composition pharmaceutique selon la revendication 8, formulée pour une administration nasale ou sous-cutanée.
